# EUROPEAN PATENT SPECIFICATION

(11) **EP 1 638 953 B1**
(45) Date of publication and mention of the grant of the patent: **27.08.2008**
(21) Application number: 04749012.3
(22) Date of filing: 16.06.2004
(51) Int. Cl.: C07D 401/12

(54) **3-SUBSTITUTED 5,6-DIARYL-PYRAZINE-2-CARBOXAMIDE AND -2-SULFONAMIDE DERIVATIVES AS CB1 MODULATORS**
3-SUBSTITUIERTE 5,6-DIARYL-PYRAZIN-2-CARBONSÄUREAMID- UND -2-SULFONSÄUREAMIDDERIVATIVE ALS CB1 MODULATOREN
DERIVES DE 2-CARBOXAMIDE ET 2-SULFONAMIDE-5,6-DIARYL-PYRAZINE SUBSTITUES EN 3, UTILISES COMME MODULATEURS DE CB1

(30) Priority: 18.06.2003 GB 0314057
(43) Date of publication of application: 29.03.2006
(73) Proprietor: AstraZeneca AB, 151 85 Södertälje (SE)
(72) Inventor: CHENG, Leifeng, S-431 83 Mölndal (SE)
(74) Representative: Wahlström, Stig Christer Gunnar
(86) International application number: PCT/SE2004/000970
(87) International publication number: WO 2004/111034

(56) References cited:
- EP-A1- 0 656 354
- WO-A-20/04111039
- WO-A1-01/70700
- WO-A1-92/02513
- WO-A1-03/051850
- WO-A1-03/051851
- OHTA A. ET AL.: 'Anti-platelet aggregation activity of some pyrazines' BIOL. PHARM. BULL. vol. 20, no. 10, 1997, pages 1076 - 1081, XP001118289

## Description

### Field of invention

The present invention relates to certain 3-substituted-4,5-diarylpyrazine-2-carboxamides compounds of formula I to processes for preparing such compounds, to their use in the treatment of obesity, psychiatric and neurological disorders, to the compounds of formula I to be used in methods for their therapeutic use and to pharmaceutical compositions containing them.

### Background of the invention

It is known that certain CB₁ modulators (known as antagonists or inverse agonists) are useful in the treatment of obesity, psychiatric and neurological disorders (WO01/70700 and EP 656354). However, there is a need for CB₁ modulators with improved physicochemical properties and/or DMPK properties and/or pharmacodynamic properties.

Pyrazinecarboxamides are reported to possess antithrombotic properties (WO 92/ 02513). The compounds disclosed in this document are disclaimed from the compound claims of the present invention. 5,6-Diphenyl-2-pyrazinecarboxylic acid is disclosed in CH 458 361.

Co-pending applications PCT/GB02/05742 (WO03051851) and WO03051850 disclose compounds of the general formula (A) 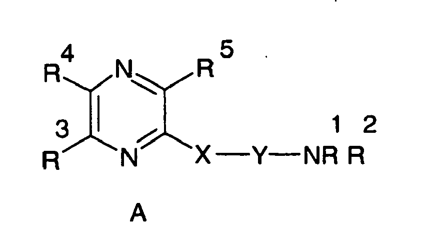 and pharmaceutically acceptable salts, prodrugs, solvates and crystalline forms thereof, in which
R¹ and R² independently represent:
a C₁₋₆alkyl group;
an (amino)C₁₋₄alkyl- group in which the amino is optionally substituted by one or more C₁₋₃alkyl groups;
an optionally substituted non-aromatic C₃₋₁₅carbocyclic group;
a (C₃₋₁₂cycloalkyl)C₁₋₃alkyl- group;
a group -(CH₂)ᵣ(phenyl )ₛ in which r is 0,1, 2, 3 or 4, s is 1 when r is 0 otherwise s is 1 or 2 and the phenyl groups are optionally independently substituted by one, two or three groups represented by Z;
naphthyl;
anthracenyl;
a saturated 5 to 8 membered heterocyclic group containing one nitrogen and optionally one of the following: oxygen, sulphur or an additional nitrogen wherein the heterocyclic group is optionally substituted by one or more C₁₋₃alkyl groups, hydroxy or benzyl ;
1-adamantylmethyl;
a group - (CH₂)ₜ Het in which t is 0,1, 2, 3 or 4, and the alkylene chain is optionally substituted by one or more C₁₋₃alkyl groups and Het represents an aromatic heterocycle optionally substituted by one, two or three groups selected from a C₁₋₅alkyl group, a C₁₋₅alkoxy group or halo;
or R¹ represents H and R² is as defined above;
or R¹ and R² together with the nitrogen atom to which they are attached represent a saturated 5 to 8 membered heterocyclic group containing one nitrogen and optionally one of the following: oxygen, sulphur or an additional nitrogen; wherein the heterocyclic group is optionally substituted by one or more C₁₋₃alkyl groups, hydroxy or benzyl;
X is CO or SO₂;
Y is absent or represents NH optionally substitututed by a C₁₋₃alkyl group;
R³ and R⁴ independently represent phenyl, thienyl or pyridyl each of which is optionally substituted by one, two or three groups represented by Z;
Z represents a C₁₋₃alkyl group, a C₁₋₃alkoxy group, hydroxy, halo, trifluoromethyl, trifluoromethylthio, trifluoromethoxy, trifluoromethylsulphonyl, nitro, amino, mono or di C₁₋₃alkylamino, mono or di C₁₋₃alkylamido, C₁₋₃alkylsulphonyl, C₁₋₃alkoxycarbonyl, carboxy, cyano, carbamoyl, mono or di C₁₋₃alkyl carbamoyl, sulphamoyl and acetyl; and
R⁵ is H, a C₁₋₃alkyl group, a C₁₋₃alkoxymethyl group, trifluoromethyl, a hydroxyC₁₋₃alkyl group, C₁₋₃alkoxycarbonyl, carboxy, cyano, carbamoyl, mono or di C₁₋₃alkylcarbamoyl, acetyl, or hydrazinocarbonyl of formula -CONHNR^{a}R^{b} wherein R^{a} and R^{b} are as previously defined for R¹ and R² respectively;
with the proviso that when R¹ and R² together with the nitrogen atom to which they are attached represent 4-methylpiperazin-1-yl or R¹ represents H and R² represents methyl or 1-benzylpiperidin-4-yl; X is CO; Y is absent and R⁵ is H; then R³ and R⁴ do not both represent 4-methoxyphenyl; and their use in the treatment of obesity, psychiatric and neurological disorders.

### Description of the invention

The invention relates to a compound of the general formula (I) 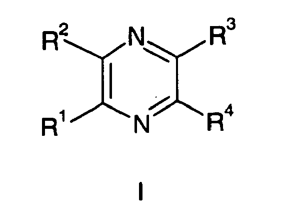 and pharmaceutically acceptable salts thereof, in which
R¹ and R² independently represent phenyl, thienyl or pyridyl each of which is independently optionally substituted by one or more groups represented by Z;
Z represents a C₁₋₈alkyl group, a C₁₋₆alkoxy group, hydroxy, halo, trifluoromethyl, trifluoromethylthio, trifluoromethoxy, trifluoromethylsulphonyl, nitro, mono or di C₁₋₃alkylamido, C₁₋₃alkylsulphonyl, C₁₋₃alkylsulphonyloxy, C₁₋₃alkoxycarbonyl, carboxy, cyano, carbamoyl, mono or di C₁₋₃alkyl carbamoyl, sulphamoyl, acetyl, an aromatic heterocyclic group which is optionally substituted by one or more halo, C₁₋₄alkyl, trifluoromethyl or trifluoromethoxy and a saturated or partially unsaturated 5 to 8 membered heterocyclic group containing one or more heteroatoms selected from nitrogen, oxygen or sulphur wherein the heterocyclic group is optionally substituted by one or more C₁₋₃alkyl groups, hydroxy, fluoro, benzyl or an amino group -NR^{x}R^{y} in which R^{x} and R^{y} independently represent H or C₁₋₄alkyl;
R³ represents a group of formula X-Y-NR⁵R⁶
in which X is CO or SO₂
and Y is absent or represents NH optionally substituted by a C₁₋₃alkyl group
and R⁵ and R⁶ independently represent:
a C₁₋₆alkyl group;
an (amino)C₁₋₄alkyl- group in which the amino is optionally substituted by one or more C₁₋₃alkyl groups;
a group (C₃₋₁₂ycloalkyl)(CH₂)_{g}- wherein g is 0, 1, 2 or 3 wherein the cycloalkyl is optionally substituted by one or more fluoro, hydroxy, C₁₋₃alkyl C₁₋₃alkoxy, trifluoromethyl or trifluoromethoxy;
a group -(CH₂)ᵣ(phenyl )ₛ in which r is 0, 1, 2, 3 or 4, s is 1 when r is 0 otherwise s is 1 or 2 and the phenyl groups are optionally independently substituted by one or more groups represented by Z;
naphthyl;
anthracenyl;
a saturated or partially unsaturated 5 to 8 membered heterocyclic group containing one or more heteroatoms selected from nitrogen, oxygen or sulphur wherein the heterocyclic group is optionally substituted by one or more C₁₋₃alkyl groups, hydroxy, fluoro, trifluoromethyl, benzyl or an amino group -NR^{x}R^{y} in which R^{x} and R^{y} independently represent H or C₁₋₄alkyl;
1-adamantylmethyl;
a group - (CH₂)ₜ Het in which t is 0,1, 2, 3 or 4, and the alkylene chain is optionally substituted by one or more C₁₋₃alkyl groups and Het represents an aromatic heterocyclic group optionally substituted by one, two or three groups selected from a C₁₋₅alkyl group, a C₁₋₅alkoxy group or halo;
or R⁵ represents H and R⁶ is as defined above;
or R⁵ and R⁶ together with the nitrogen atom to which they are attached represent a saturated or partially unsaturated 5 to 8 membered heterocyclic group containing one nitrogen and optionally one of the following: oxygen, sulphur or an additional nitrogen; wherein the heterocyclic group is optionally substituted by one or more more C₁₋₃alkyl groups, hydroxy, fluoro, trifluoromethyl, trifluoromethoxy, benzyl, C₁₋₆alkanoyl or an amino group -NR^{x}R^{y} in which R^{x} and R^{y} independently represent H or C₁₋₄alkyl;
R⁴ represents a group of formula (CH₂)ₙCOOR⁷ in which n is 0, 1, 2, 3 or 4; and R⁷ represents a C₄₋₁₂alkyl group, a C₃₋₁₂cycloalkyl group or a (C₃₋₁₂cycloalkyl)C₁₋₃alkyl-group each of which is optionally substituted by one or more of the following: a C₁₋₆alkyl group; fluoro, amino or hydroxy, or
R⁷ represents a group -(CH₂)ₐphenyl in which a is 0, 1, 2, 3 or 4 and the phenyl group is optionally substituted by one or more groups represented by Z which may be the same or different or
R⁷ represents a saturated or partially unsaturated 5 to 8 membered heterocyclic group containing one or more of the of the following: oxygen, sulphur or nitrogen; wherein the heterocyclic group is optionally substituted by one or more C₁₋₃alkyl groups, C₁₋₃acyl groups, hydroxy, amino or benzyl; or
R⁴ represents a group of formula -(CH₂)ₒ-O-(CH₂)ₚ- R⁸ in which o and p independently represent an integer 0, 1, 2, 3 or 4 and R⁸ represents a C₁₋₁₂alkyl group with the proviso that R⁴ is not a C₁₋₃alkoxymethyl group, or R⁸ represents phenyl optionally independently substituted by one or more Z groups or R⁸ represents an aromatic heterocyclic group or a saturated or partially unsaturated 5 to 8 membered heterocyclic group containing one or more of one following: oxygen, sulphur or nitrogen wherein each of these rings is optionally substituted by one or more groups represented by Z which may be the same or different;
R⁴ represents a C₄₋₁₂alkyl group optionally substituted by one or more fluoro, hydroxy, or amino; or
R⁴ represents a group of formula -(CH₂)_{q}R⁹ in which q is 0, 1, 2, 3 or 4 and R⁹ represents a C₃₋₁₂cycloalkyl group, phenyl, an aromatic heterocyclic group or a saturated or partially unsaturated 5 to 8 membered heterocyclic group containing one or more of one following: oxygen, sulphur or nitrogen wherein each of these rings is optionally substituted by one or more groups represented by Z which may be the same or different; or
R⁴ represents a group of formula -(CH₂)ₘ-O-(CO)- R¹⁰ in which m represents an integer 0, 1, 2, 3 or 4, in which R¹⁰ represents a C₁₋₁₂alkyl group optionally substituted by one or more fluoro, hydroxy, or amino or R¹⁰ represents a group of formula -(CH₂)_{q}R⁹ in which q and R⁹ is as previously described,
wherein the term aromatic heterocyclic group means an aromatic 5-, 6-, or 7-membered monocyclic ring or a 9- or 10-membered bicyclic ring, with up to five ring heteroatoms selected from oxygen, nitrogen and sulfur.
It will be understood that where a substituent Z is present in more than one group that these substituents are independently selected and may be the same or different.

Suitable aromatic heterocyclic groups include, for example furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl, preferably furyl, pyrrolyl, thienyl, oxazolyl, isoxazolyl, imidazolyl, pyrazolyl, oxazolyl thiazolyl, isothiazolyl, oxadiazolyl, thiadiazolyl, triazolyl, tetrazolyl, pyridyl, pyridazinyl, pyrimidinyl, pyrazinyl or 1,3,5-triazenyl and more preferably pyrrolyl, thienyl, imidazolyl, oxazolyl or pyridyl.

Suitable saturated or partially unsaturated 5 to 8 membered heterocyclic group containing one or more heteroatoms selected from nitrogen, oxygen or sulphur include, for example oxiranyl, oxetanyl, tetrahydrofuranyl, tetrahydropyranyl, 2,3-dihydro-1,3-thiazolyl, 1,3-thiazolidinyl, pyrrolinyl, pyrrolidinyl, morpholinyl, tetrahydro-1,4-thiazinyl, 1-oxotetra-hydrothienyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl or tetra-hydropyrimidinyl, preferably tetrahydrofuranyl, tetrahydropyranyl, pyrrolidinyl, morpholinyl, piperidinyl or piperazinyl, more preferably tetrahydrofuran-3-yl, tetrahydropyran-4-yl, pyrrolidin-3-yl, morpholino, piperidino, piperidin-4-yl or piperazin-1-yl.

Further values of R¹, R², R³, R⁴, R⁵ and R⁶ in compounds of formula I now follow. It will be understood that such values may be used where appropriate with any of the definitions, claims or embodiments defined hereinbefore or hereinafter.

In one particular group of compounds of formula I, R¹ and R² represent pheriyl independently optionally substituted by one or more chloro or methyl.

In another particular group of compounds of formula I, R³ represents CONHR^{k} in which R^{k} represents piperidino, 4,4-difluorocyclohexyl or C₃₋₆alkyl optionally substituted by hydroxy.

One particular group of compounds of formula I is represented by formula IIa 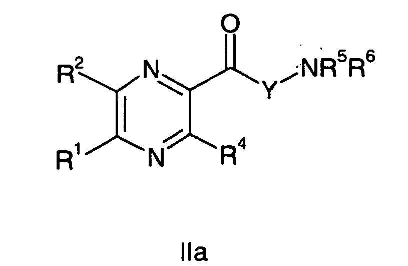 wherein R¹ and R² independently represent phenyl optionally independently substituted by halo or pyridyl,
R⁴ represents a C₄₋₈alkyl group, a group CH₂OR⁸ in which R⁸ is a C₄₋₈alkyl group, a group CO₂R⁷ in which R⁷ represents a C₄₋₈alkyl group,
and Y is represents NH and
R⁵ represents H and
R⁶ represents perfluorophenyl or phenyl optionally substituted by trifluoromethyl or R⁵ and R⁶ together with the nitrogen to which they are attached represent piperidino, morpholino or piperazino each of which is optionally substituted by one or more C₁₋₃alkyl groups, hydroxy, fluoro, trifluoromethyl, trifluoromethoxy, benzyl, C₁₋₆alkanoyl or an amino group -NR^{x}R^{y} in which R^{x} and R^{y} independently represent H or C₁₋₄alkyl;
or Y is absent and
R⁵ represents H or a C₁₋₆alkyl group optionally substituted by amino;
R⁶ represents tetrahydropyranyl or 4- piperidinyl optionally substituted by one or more C₁-₃alkyl groups, hydroxy, fluoro, trifluoromethyl, trifluoromethoxy, benzyl, C₁₋₆alkanoyl or an amino group -NR^{x}R^{y} in which R^{x} and R^{y} independently represent H or C₁₋₄alkyl or a C₁₋₆alkyl group optionally substituted by amino;
or
R⁵ and R⁶ together with the nitrogen to which they are attached represent piperidino, morpholino or piperazino each of which is optionally substituted by C₁₋₃alkyl or fluoro.

A second particular group of compounds of formula I is represented by formula IIb 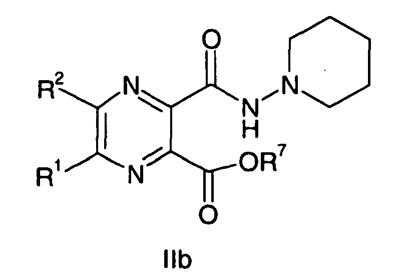 wherein R¹ and R² represent phenyl independently optionally substituted by one or more chloro; and
R⁷ represents butyl, tert-butyl, cyclohexyl or benzyl.

"Pharmaceutically acceptable salt", where such salts are possible, includes both pharmaceutically acceptable acid and base addition salts. All tautomers, where possible, are included within the scope of the invention.

Throughout the specification and the appended claims, a given chemical formula or name shall encompass all stereo and optical isomers and racemates thereof as well as mixtures in different proportions of the separate enantiomers, where such isomers and enantiomers exist, as well as pharmaceutically acceptable salts thereof. Isomers may be separated using conventional techniques, e.g. chromatography or fractional crystallisation. The enantiomers may be isolated by separation of racemate for example by fractional crystallisation, resolution or HPLC. The diastereomers may be isolated by separation of isomer mixtures for instance by fractional crystallisation, HPLC or flash chromatography. Alternatively the stereoisomers may be made by chiral synthesis from chiral starting materials under conditions which will not cause racemisation or epimerisation, or by derivatisation, with a chiral reagent. All stereoisomers are included within the scope of the invention.

The following definitions shall apply throughout the specification and the appended claims.

Unless otherwise stated or indicated, the term "alkyl" denotes either a straight or branched alkyl group. Examples of said alkyl include methyl, ethyl, n-propyl, isopropyl, n-butyl, isobutyl, sec-butyl and t-butyl. Preferred alkyl groups are methyl, ethyl, propyl, isopropyl and tertiary butyl.

Unless otherwise stated or indicated, the term "alkoxy" denotes a group O-alkyl, wherein alkyl is as defined above.

Unless otherwise stated or indicated, the term "halogen" shall mean fluorine, chlorine, bromine or iodine.

Specific compounds of the invention are one or more of the following:
*tert*-butyl 5,6-bis(4-chlorophenyl)-3-[(piperidin-1-ylamino)carbonyl]pyrazine-2-carboxylate;
butyl 5,6-bis(4-chlorophenyl)-3-[(piperidin-1-ylamino)carbonyl]pyrazine-2-carboxylate; cyclohexyl 5,6-bis(4-chlorophenyl)-3-[(piperidin-1-ylamino)carbonyl]pyrazine-2-carboxylate;
benzyl 5,6-bis(4-chlorophenyl)-3-[(piperidin-1-ylamino)carbonyl]pyrazine-2-carboxylate; tert-butyl 5,6-bis(4-chlorophenyl)-3-({[cis-2-hydroxycyclohexyl]amino}carbonyl)-pyrazine-2-carboxylate;
tert-butyl 5,6-bis(4-chlorophenyl)-3-({[trans-2-hydroxycyclohexyl]amino}carbonyl)-pyrazine-2-carboxylate;
tert-butyl 5,6-bis(4-chlorophenyl)-3-({2-[4-(trifluoromethyl)phenyl]hydrazino}carbonyl)-pyrazine-2-carboxylate;
tert-butyl 5,6-bis(4-chlorophenyl)-3-[(morpholin-4-ylamino)carbonyl]pyrazine-2-carboxylate;
tert-butyl 5,6-bis(4-chlorophenyl)-3-({2-[4-(tert-butylhydrazino}carbonyl)pyrazine-2-carboxylate;
tert-butyl 5,6-bis(4-chlorophenyl)-3-{[(4,4-difluorocyclohexyl)amino]carbonyl}pyrazine-2-carboxylate;
tert-butyl 5,6-bis(4-chlorophenyl)-3-[(pentylamino)carbonyl]pyrazine-2-carboxylate;
tert-butyl 5,6-bis(4-chlorophenyl)-3-{[(1-ethylpropyl)amino]carbonyl}pyrazine-2-carboxylate;
tert-butyl 5,6-bis(4-chlorophenyl)-3-{[(4,4-difluoropiperidin-1-yl)amino]carbonyl}-pyrazine-2-carboxylate;
5,6-bis(4-chlorophenyl)-N-piperidin-1-yl-3-[(4-propyl-1H-1,2,3-triazol-1-yl)methyl]pyrazine-2-carboxamide;
5,6-bis(4-chlorophenyl)-3-(phenoxymethyl)-*N*-piperidin-1-ylpyrazine-2-carboxamide;
5,6-bis(4-chlorophenyl)-3-(morpholin-4-ylmethyl)-*N*-piperidin-1-ylpyrazine-2-carboxamide;
5,6-bis(4-chlorophenyl)-3-(piperidin-1-ylmethyl)-*N*-piperidin-1-ylpyrazine-2-carboxamide;
*Tert*-butyl 5,6-bis(4-methylphenyl)-3-[(piperidin-1-ylamino)carbonyl]pyrazine-2-carboxylate;
5,6-bis(4-methylphenyl)-*N*-piperidin-1-yl-3-(1*H*-tetrazol-1-ylmethyl)pyrazine-2-carboxamide;
5,6-bis(4-methylphenyl)-*N*-piperidin-1-yl-3-(2*H*-tetrazol-2-ylmethyl)pyrazine-2-carboxamide;
5,6-bis(4-chlorophenyl)-*N*-piperidin-1-yl-3-(2*H*-tetrazol-2-ylmethyl)pyrazine-2-carboxamide;
5,6-bis(4-chlorophenyl)-*N*-piperidin-1-yl-3-(1*H*-tetrazol-1-ylmethyl)pyrazine-2-carboxamide;
5,6-bis(4-chlorophenyl)-*N*-(4,4-difluorocyclohexyl)-3-(2*H*-tetrazol-2-ylmethyl)pyrazine-2-carboxamide;
5,6-bis(4-chlorophenyl)-*N*-(4,4-difluoropiperidin-1-yl)-3-(2*H*-tetrazol-2-ylmethyl)pyrazine-2-carboxamide;
5,6-bis(4-chlorophenyl)-3-[(5-methyl-2*H*-tetrazol-2-yl)methyl]-*N*-piperidin-1-ylpyrazine-2-carboxamide;
5,6-bis(4-chlorophenyl)-3-[(5-methyl-1*H*-tetrazol-1-yl)methyl]-*N*-piperidin-1-ylpyrazine-2-carboxamide;
*tert*-butyl 6-(4-chlorophenyl)-5-(4-methylphenyl)-3-[(piperidin-1-ylamino)carbonyl]pyrazine-2-carboxylate;
*tert*-butyl 5-(4-chlorophenyl)-6-(4-methylphenyl)-3-[(piperidin-1-ylamino)carbonyl]pyrazine-2-carboxylate;
6-(4-chlorophenyl)-5-(4-methylphenyl)-*N*-piperidin-1-yl-3-(2*H*-tetrazol-2-ylmethyl)pyrazine-2-carboxamide;
5-(4-chlorophenyl)-6-(4-methylphenyl)-*N*-piperidin-1-yl-3-(2*H*-tetrazol-2-ylmethyl)pyrazine-2-carboxamide;
5,6-bis-(4-chloro-phenyl)-3-propoxy-pyrazine-2-carboxylic acid piperidin-1-ylamide;
5,6-bis(4-chlorophenyl)-*N*-piperidin-1-yl-3-(2*H*-tetrazol-5-ylmethyl)pyrazine-2-carboxamide
5,6-bis(4-chlorophenyl)-3-[(5-morpholin-4-yl-2*H*-tetrazol-2-yl)methyl]-*N*-piperidin-1-ylpyrazine-2-carboxamide;
5,6-bis(4-chlorophenyl)-3-[(5-morpholin-4-yl-1*H*-tetrazol-1-yl)methyl]-*N*-piperidin-1-ylpyrazine-2-carboxamide;
5,6-bis(4-chlorophenyl)-*N*-piperidin-1-yl-3-[(5-pyrrolidin-1-yl-2*H*-tetrazol-2-yl)methyl]pyrazine-2-carboxamide;
5,6-bis(4-chlorophenyl)-*N*-piperidin-1-yl-3-[(5-pyrrolidin-1-yl-1*H*-tetrazol-1-yl)methyl]pyrazine-2-carboxamide;
5,6-bis(4-chlorophenyl)-*N*-(4,4-difluorocyclohexyl)-3-{[(4-fluorobenzyl)oxy]methyl}pyrazine-2-carboxamide;
5,6-bis(4-chlorophenyl)-3-[(4,4-difluoropiperidin-1-yl)methyl]-*N*-piperidine-1-ylpyrazine-2-carboxamide; or
5,6-bis(4-chlorophenyl)-*N*-(4,4-difluorocyclohexyl)-3-[(4,4-difluoropiperidin-1-yl)methyl]pyrazine-2-carboxamide; and pharmaceutically acceptable salts thereof.

### Methods of preparation

The compounds of the invention may be prepared as outlined below according to any of the following methods. However, the invention is not limited to these methods, the compounds may also be prepared as described for structurally related compounds in the prior art.

Compounds of formula I in which R¹, R² and R⁴ are as previously defined and R³ is COYNR⁵R⁶ as previously defined may be prepared by reacting a compound of formula III 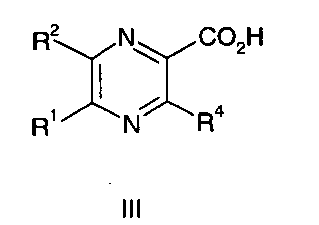 in which R¹, R² and R⁴ are as previously defined with an amine of formula IV

R⁵ R⁶ YNH₂ IV

in which Y, R⁵ and R⁶ are as previously defined in a solvent, for example dichloromethane, in the presence of a coupling agent, for example benzotriazol-1-yl-oxytripyrrolidinophosphonium hexafluorophosphate or for example a carbodimide, e.g., 1-(3-dimethylaminopropyl)-3-ethylcarbodiimide, and optionally in the presence of a base, for example 4-dimethylaminopyridine or triethylamine, at a temperature in the range of - 25°C to 150°C.

Compounds of formula III may be prepared by reacting a compound of formula V 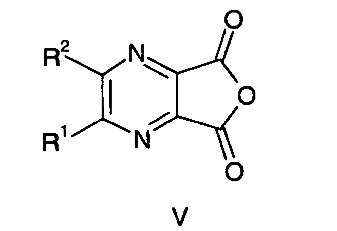 in which R¹ and R² are as previously defined with a compound of formula VI

R⁴OH VI

in which R⁴ is as previously defined in an inert solvent, for example acetonitrile, and optionally in the presence of a catalyst, for example a basic catalyst, e.g., 4-dimethylaminopyridine, at a temperature in the range of -25°C to 150°C.

Compounds of formula I may also be prepared by reacting a compound of formula V with a compound of formula VI and then reacting the product directly with a compound of formula IV.

Compounds of formula V may be prepared by reacting a compound of formula VIII 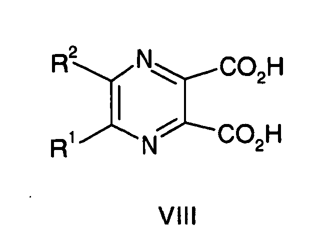 in which R¹ and R² are as previously defined with a dehydrating agent for example acetyl chloride at a temperature in the range of 0°C to 150°C.

Compounds of formulae IV and VI are commercially available or may be prepared by methods known to those skilled in the art. Certain compounds of formulae III, V and VIII are novel and are claimed as a further aspect of the present invention as useful intermediates.

Compounds of formula I in which X is SO₂ may be prepared by reacting a compound of formula IX 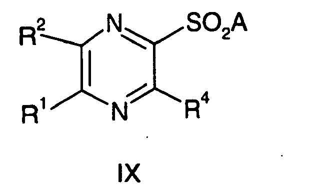 in which R¹, R² and R⁴ are as previously defined and A represents halo with an amine of formula X

R⁵ R⁶ YNH₂ X

in which Y, R⁵ and R⁶ are as previously defined in an inert solvent, for example dichloromethane, and optionally in the presence of a catalyst, for example a basic catalyst, eg 4-dimethylamino-pyridine, at a temperature in the range of -25°C to 150°C.

Compounds of formula I in which R¹, R² and R³ are as previously defined and R⁴ represents a group CH₂(1H-1,2,3-triazol-1-yl) in which the triazole is optionally substituted on carbon by Z may be prepared by reacting an azide of formula XI 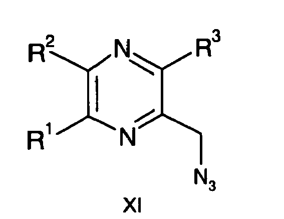 with an acetylene of formula XII

H-C≡C-Z XII

in which Z is as previously defined in an inert solvent, for example toluene, *tert*-butanol or water or mixtures thereof, and optionally in the presence of a catalyst, for example a copper I salt, e.g., formed in situ from a copper II salt, for instance sulphate, in the presence of sodium ascorbate, at a temperature in the range of -25°C to 150°C.

Compounds of formula XI are believed to be novel and are claimed as a further aspect of the present invention as useful intermediates in the preparation of compounds of formula I. Compounds of formula XI in which R¹ and R² are as previously defined and R³ is COYNR⁵R⁶ as previously defined may be prepared by reacting a compound of formula XVI 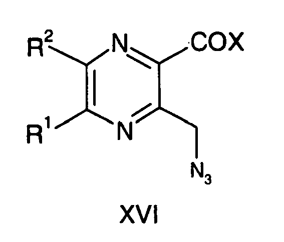 in which R¹ and R² are as previously defined and X is a leaving group for example C₁₋₆alkoxy, halo or hydroxy with an amine of formula IV

R⁵R⁶ YNH₂ IV

in which Y, R⁵ and R⁶ are as previously defined in a solvent, for example dichloromethane, optionally in the presence of a coupling agent, for example benzotriazol-1-yl-oxytripyrrolidinophosphonium hexafluorophosphate or for example a carbodimide, e.g., 1-(3-dimethylaminopropyl)-3-ethylcarbodiimide, and optionally in the presence of a base, for example 4-dimethylaminopyridine or triethylamine, at a temperature in the range of -25°C to 150°C.

Compounds of formula I in which R¹, R² and R⁴ are as previously defined and R³ is COYNR⁵R⁶ may also be prepared by reacting a compound of formula XIV 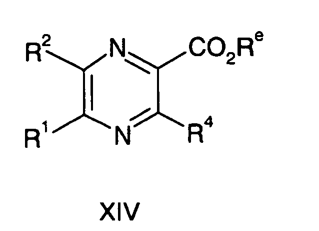 in which R¹, R² and R⁴ are as previously defined and R^{e} represents an alkyl group, for example a C₁₋₆alkyl group, with an amine of formula IV

R⁵R⁶ YNH₂ IV

or a salt thereof in which Y, R⁵ and R⁶ are as previously defined in a solvent, for example dichloromethane and or toluene, in the presence of a coupling agent, for example a trialkylaluminium compound e.g., trimethylaluminium, and optionally in an inert atmosphere for example under nitrogen or argon, at a temperature in the range of -25°C to 150°C.

Compounds of formula I in which R¹, R² and R⁴ are as previously defined and R³ is COYNR⁵R⁶ may be prepared by reacting a compound of formula XV 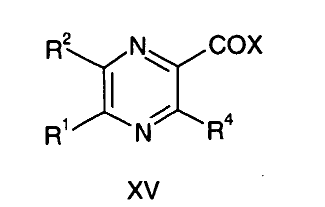 in which R¹, R² and R⁴ are as previously defined and X represents a leaving group for example chloro or bromo, with an amine of formula IV

R⁵R⁶ YNH₂ IV

or a salt thereof in which Y, R⁵ and R⁶ are as previously defined in a solvent, for example dichloromethane and or toluene, optionally in the presence of a base, for example triethylamine, at a temperature in the range of -25°C to 150C.

Compounds of formulae IX, XIV, XV and XVI may be prepared as described in the examples or by analogous methods.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

Compounds of formula I may be prepared by de-protecting compounds of formula I, in which one or more groups is protected, by methods known to those skilled in the art for example by hydrolysis of a tert-butoxy carbonyl protected amine group to give the amine group.

The expression "inert solvent" refers to a solvent which does not react with the starting materials, reagents, intermediates or products in a manner which adversely affects the yield of the desired product.

### Pharmaceutical preparations

The compounds of the invention will normally be administered via the oral, parenteral, intravenous, intramuscular, subcutaneous or in other injectable ways, buccal, rectal, vaginal, transdermal and/or nasal route and/or via inhalation, in the form of pharmaceutical preparations comprising the active ingredient or a pharmaceutically acceptable addition salt, in a pharmaceutically acceptable dosage form. Depending upon the disorder and patient to be treated and the route of administration, the compositions may be administered at varying doses.

Suitable daily doses of the compounds of the invention in the therapeutic treatment of humans are about 0.001-10 mg/kg body weight, preferably 0.01-1 mg/kg body weight.

Oral formulations are preferred particularly tablets or capsules which may be formulated by methods known to those skilled in the art to provide doses of the active compound in the range of 0.5mg to 500mg for example 1 mg, 3 mg, 5 mg, 10 mg, 25mg, 50mg, 100mg and 250mg.

According to a further aspect of the invention there is also provided a pharmaceutical formulation including any of the compounds of the invention, or pharmaceutically acceptable derivatives thereof, in admixture with pharmaceutically acceptable adjuvants, diluents and/or carriers.

### Pharmacological properties

The compounds of formula (I) are useful for the treatment of obesity, psychiatric disorders such as psychotic disorders, schizophrenia, bipolar disorders, anxiety, anxio-depressive disorders, depression, cognitive disorders, memory disorders, obsessive-compulsive disorders, anorexia, bulimia, attention disorders like ADHD, epilepsy, and related conditions, and neurological disorders such as dementia, neurological disorders(e.g. Multiple Sclerosis), Raynaud's syndrome, Parkinson's disease, Huntington's chorea and Alzheimer's disease. The compounds are also potentially useful for the treatment of immune, cardiovascular, reproductive and endocrine disorders, septic shock and diseases related to the respiratory and gastrointestinal systems (e.g. diarrhea). The compounds are also potentially useful as agents in treatment of extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms. The compounds may also eliminate the increase in weight which normally accompanies the cessation of smoking.

In another aspect the present invention provides a compound of formula I as previously defined for use as a medicament.

In a further aspect the present invention provides the use of a compound of formula I in the preparation of a medicament for the treatment or prophylaxis of obesity, psychiatric disorders such as psychotic disorders, schizophrenia, bipolar disorders, anxiety, anxio-depressive disorders, depression, cognitive disorders, memory disorders, obsessive-compulsive disorders, anorexia, bulimia, attention disorders like ADHD, epilepsy, and related conditions, neurological disorders such as dementia, neurological disorders (e.g. Multiple Sclerosis), Parkinson's Disease, Huntington's Chorea and Alzheimer's Disease, immune, cardiovascular, reproductive and endocrine disorders, septic shock, diseases related to the respiratory and gastrointestinal systems (e.g. diarrhea), and extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms.

In a still further aspect the present invention provides a compound of formula I to be used in a method of treating obesity, psychiatric disorders such as psychotic disorders such as schizophrenia and bipolar disorders, anxiety, anxio-depressive disorders, depression, cognitive disorders, memory disorders, obsessive-compulsive disorders, anorexia, bulimia, attention disorders like ADHD, epilepsy, and related conditions, neurological disorders such as dementia, neurological disorders (e.g. Multiple Sclerosis), Parkinson's Disease, Huntington's Chorea and Alzheimer's Disease, immune, cardiovascular, reproductive and endocrine disorders, septic shock, diseases related to the respiratory and gastrointestinal systems (e.g. diarrhea), and extended abuse, addiction and/or relapse indications, e.g. treating drug (nicotine, ethanol, cocaine, opiates, etc) dependence and/or treating drug (nicotine, ethanol, cocaine, opiates, etc) withdrawal symptoms comprising administering a pharmacologically effective amount of a compound of formula I to a patient in need thereof.

The compounds of the present invention are particulary suitable for the treatment of obesity, e.g. by reduction of appetite and body weight, maintenance of weight reduction and prevention of rebound.

### Combination Therapy

The compounds of the invention may be combined with another therapeutic agent that is useful in the treatment of disorders associated with the development and progress of obesity such as hypertension, hyperlipidaemias, dyslipidaemias, diabetes and atherosclerosis. For example, a compound of the present invention may be used in combination with a compound that affects thermogenesis, lipolysis, fat absorption, satiety, or gut motility. The compounds of the invention may be combined with another therapeutic agent that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

The compounds of the invention may be used alongside other therapies for the treatment of obesity and its associated complications the metabolic syndrome and type 2 diabetes, these include biguanide drugs, insulin (synthetic insulin analogues) and oral antihyperglycemics (these are divided into prandial glucose regulators and alpha-glucosidase inhibitors).

In another aspect of the invention, the compound of formula I, or a pharmaceutically acceptable salt thereof may be administered in association with a PPAR modulating agent. PPAR modulating agents include but are not limited to a PPAR alpha and/or gamma agonist, or pharmaceutically acceptable salts, solvates, solvates of such salts or prodrugs thereof. Suitable PPAR alpha and/or gamma agonists, pharmaceutically acceptable salts, solvates, solvates of such salts or prodrugs thereof are well known in the art.

In addition the combination of the invention may be used in conjunction with a sulfonylurea. The present invention also includes a compound of the present invention in combination with a cholesterol-lowering agent. The cholesterol-lowering agents referred to in this application include but are not limited to inhibitors of HMG-CoA reductase (3-hydroxy-3-methylglutaryl coenzyme A reductase). Suitably the HMG-CoA reductase inhibitor is a statin

In the present application, the term "cholesterol-lowering agent" also includes chemical modifications of the HMG-CoA reductase inhibitors, such as esters, prodrugs and metabolites, whether active or inactive.

The present invention also includes a compound of the present invention in combination with an inhibitor of the ileal bile acid transport system (IBAT inhibitor). The present invention also includes a compound of the present invention in combination with a bile acid binding resin.
The present invention also includes a compound of the present invention in combination with a bile acid sequestering agent, for example colestipol or cholestyramine or cholestagel According to an additional further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration one or more of the following agents selected from:
a CETP (cholesteryl ester transfer protein) inhibitor;
a cholesterol absorption antagonist;
a MTP (microsomal transfer protein) inhibitor;
a nicotinic acid derivative, including slow release and combination products;
a phytosterol compound;
probucol;
an anti-coagulant;
an omega-3 fatty acid;
another anti-obesity compound;
an antihypertensive compound for example an angiotensin converting enzyme (ACE) inhibitor, an angiotensin II receptor antagonist, an andrenergic blocker, an alpha andrenergic blocker, a beta andrenergic blocker, a mixed alpha/beta andrenergic blocker, an andrenergic stimulant, calcium channel blocker, an AT-1 blocker, a saluretic, a diuretic or a vasodilator;
a Melanin concentrating hormone (MCH) antagonist;
a PDK inhibitor; or
modulators of nuclear receptors for example LXR, FXR, RXR, and RORalpha;
an SSRI;
a serotonin antagonist;
or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

Therefore in an additional feature of the invention, there is provided a compound of formula I to be used in a method for for the treatment of obesity and its associated complications in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section, or a pharmaceutically acceptable salt, solvate, solvate of such a salt or a prodrug thereof.

Therefore in an additional feature of the invention, there is provided a compond of formula I to be used in a method of treating hyperlipidemic conditions in a warm-blooded animal, such as man, in need of such treatment which comprises administering to said animal an effective amount of a compound of formula I, or a pharmaceutically acceptable salt thereof in simultaneous, sequential or separate administration with an effective amount of a compound from one of the other classes of compounds described in this combination section or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt thereof, and a compound from one of the other classes of compounds described in this combination section or a pharmaceutically acceptable salt, solvate, solvate of such a salt, in association with a pharmaceutically acceptable diluent or carrier.

According to a further aspect of the present invention there is provided a kit comprising a compound of formula I, or a pharmaceutically acceptable salt thereof, and a compound from one of the other classes of compounds described in this combination section or a pharmaceutically acceptable salt, solvate or solvate of such a salt

According to a further aspect of the present invention there is provided a kit comprising:
a) a compound of formula I, or a pharmaceutically acceptable salt thereof, in a first unit dosage form;
b) a compound from one of the other classes of compounds described in this combination section or a pharmaceutically acceptable salt, solvate, solvate of such a salt; in a second unit dosage form; and
c) container means for containing said first and second dosage forms.

According to a further aspect of the present invention there is provided a kit comprising:
a) a compound of formula I, or a pharmaceutically acceptable salt thereof, together with a pharmaceutically acceptable diluent or carrier, in a first unit dosage form;
b) a compound from one of the other classes of compounds described in this combination section or a pharmaceutically acceptable salt, solvate or solvate of such a salt in a second unit dosage form; and
c) container means for containing said first and second dosage forms.

According to another feature of the invention there is provided the use of a compound of the formula I, or a pharmaceutically acceptable salt thereof, and one of the other compounds described in this combination section, or a pharmaceutically acceptable salt, solvate, solvate of such a salt, in the manufacture of a medicament for use in the the treatment of obesity and its associated complications in a warm-blooded animal, such as man.

According to another feature of the invention there is provided the use of a compound of the formula I, or a pharmaceutically acceptable salt thereof, and one of the other compounds described in this combination section, or a pharmaceutically acceptable salt, solvate or solvate of such a salt in the manufacture of a medicament for use in the treatment of hyperlipidaemic conditions in a warm-blooded animal, such as man.

According to a further aspect of the present invention there is provided a combination treatment comprising the administration of an effective amount of a compound of the formula I, or a pharmaceutically acceptable salt thereof, optionally together with a pharmaceutically acceptable diluent or carrier, with the simultaneous, sequential or separate administration of an effective amount of one of the other compounds described in this combination section, or a pharmaceutically acceptable salt, solvate or solvate of such a salt optionally together with a pharmaceutically acceptable diluent or carrier to a warm-blooded animal, such as man in need of such therapeutic treatment.

Furthermore, a compound of the invention may also be combined with therapeutic agents that are useful in the treatment of disorders or conditions associated with obesity (such as type II diabetes, metabolic syndrome, dyslipidemia, impaired glucose tolerance, hypertension, coronary heart disease, non-alcoholic steatorheic hepatitis, osteoarthritis and some cancers) and psychiatric and neurological conditions.

### Examples

### Abbreviations

Ar-argon
HRMS-high resolution mass spectrometer
THF-tetrahydrofuran
Dess-Martin periodinane-1,1,1-tris(acetyloxy)-1,1-duhydro-1,2-benziodoxol-3-(1H)-one
DCM - dichloromethane
DMF - dimethylformamide
DMAP - 4-dimethylaminopyridine
EDC - 1-(3-dimethylaminopropyl)-3-ethylcarbodiimide
TEA - triethylamine
TFA - trifluoroacetic acid
DMSO-dimethyl sulfoxide
DEA - Diethylamine
PCC - Pyridinium chlorochromate
DCM - Dichloromethane
PyBOP - benzotriazol-1-yl-oxytri-pyrrolidinophosphonium hexafluorophosphate
HBTU - *O*-Benzotriazol-1-yl-*N,N,N',N'*-tetramethyluronium Hexafluorophosphate
DAST-(diethyl amino)sulphur trifluoride
DIEA - *N,N*-diisopropylethylamine

- t: triplet
- s: singlet
- d: doublet
- q: quartet
- qvint: quintet
- m: multiplet
- br: broad
- bs: broad singlet
- dm: doublet of multiplet
- bt: broad triplet
- dd: doublet of doublet

### General Experimental Procedures

Mass spectra were recorded on either a Micromass ZQ single quadrupole or a Micromass LCZ single quadrupole mass spectrometer both equipped with a pneumatically assisted electrospray interface (LC-MS). ¹H NMR measurements were performed on either a Varian Mercury 300 or a Varian Inova 500, operating at ¹H frequencies of 300 and 500 MHz respectively. Chemical shifts are given in ppm with CDCl₃ as internal standard. CDCl₃ is used as the solvent for NMR unless otherwise stated. Purification was performed on a semipreparative HPLC with a mass triggered fraction collector, Shimadzu QP 8000 single quadrupole mass spectrometer equipped with 19 x 100 mm C8 column. The mobile phase used was, if nothing else is stated, acetonitrile and buffer (0.1 M NH₄Ac:acetonitrile 95:5).

For isolation of isomers, a Kromasil CN E9344 (250 x 20 mm i.d.) column was used. Heptane:ethyl acetate:DEA 95:5:0.1 was used as mobile phase (1 ml/min). Fraction collection was guided using a UV-detector (330 nm).

### Examples of the Invention

### Example 1

### tert-butyl 5,6-bis(4-chlorophenyl)-3-[(piperidin-1-ylamino)carbonyl]pyrazine-2-carboxylate

### a) 1,2-bis(4-chlorophenyl)-2-hydroxyethanone

To 4-chlorobenzaldehyde (140.6 g, 1 mol) in ethanol (130 ml) was added a solution of sodium cyanide (10.6 g, 0.216 mol) in water (105 ml). The mixture was heated at reflux for 2.5 h and then extracted with DCM. The organic phase was washed with sodium bisulfite solution and the solvent was evaporated in vacuo. The compound was isolated by crystallization from diethyl ether/heptane. 48 g, 34%.
¹H NMR (400 MHz) δ 7.82 (d, 2H), 7.38 (d, 2H), 7.30 (d, 2H), 7.24 (d, 2H), 5.87 (s, 1H), 4.47 (s, 1H).
MS *m*/*z* 279, 281 (M-H)⁻.

### b) 1,2-bis(4-chlorophenyl)ethane-1,2-dione

1,2-bis(4-chlorophenyl)-2-hydroxyethanone, (90 g, 0.320 mol) and nitric acid (170 ml) were heated at 100°C until the evolution of nitrogen oxides ceased after 4 hours. The reaction mixture was cooled, and water (250 ml) was carefully added. The crude product was filtered, washed several times with water and dried under reduced pressure to give a yellow solid (40.4 g, 45%).
¹H NMR (500 MHz) δ 7.94 (d, 4H), 7.53 (d, 4H).

### c) 5,6-bis(4-chlorophenyl)pyrazine-2,3-dicarbonitrile

1,2-bis(4-chlorophenyl)ethane-1,2-dione, (20 g, 71.65 mmol), diaminomaleonitrile (8.5 g, 78.82 mmol) and acetic acid (6 ml) in ethanol (140 ml) and water (93 ml) were heated at 75 °C overnight. The reaction mixture was cooled, and water was added. The precipitate was filtered and washed with ethanol and then ether. The crude product was dissolved in DCM and treated with activated charcoal, then filtered through celite. After evaporation, a solid was formed and recrystallized from DCM/ethanol to give a pale yellow solid (17.3 g, 69%).
¹H NMR (400 MHz) δ 7.49 (d, 4H), 7.38 (d, 4H).

### d) 5,6-bis(4-chlorophenyl)pyrazine-2,3-dicarboxylic acid

To 5,6-bis(4-chlorophenyl)pyrazine-2,3-dicarbonitrile, (16.3 g, 46.28 mmol) and KOH (26 g, 463 mmol) in water (84 ml) was added hydrogen peroxide (35%, 19 ml) followed by a few drops of nonanol to reduce foaming. The reaction mixture was heated at reflux for 2h, cooled and washed once with diehtyl ether and acidified to pH 4 with 2M HCl. The precipitate was collected through a filter, washed with water and dried under reduced pressure to give the crude product. The crude product was convertd to dimethyl ester by refluxing with hydrogen chloride/methanol (100 ml) and purified by HPLC, giving 12.85 g of the methyl ester. The resulting methyl ester was treated with lithium hydroxide (2.95 g, 0.123 mmol) in acetonitrile (140 ml) and water (90 ml) at ambient temperature for 1.5 h. The acetonitrile was removed under reduced pressure and the aqueous solution was washed once with diethyl ether. Acidification with hydrochloric acid (2M) and filtration gave the title compound (11.8 g, 66% mmol) as a pale yellow solid.
¹H NMR (400 MHz) δ 7.51 (d, 4H), 7.41 (d, 4H). MS *m*/*z* 389, 391 (M+H)⁺.

### e) 2,3-bis(4-chlorophenyl)furo[3,4-b]pyrazine-5,7-dione

5,6-bis(4-chlorophenyl)pyrazine-2,3-dicarboxylic acid (6.7 g, 17.30 mmol) and acetyl chloride (20 ml) were heated at reflux overnight. The acetyl chloride was removed under reduced pressure to give the title compound (6.2 g, 97%) as a pale yellow solid.
¹H NMR (400 MHz) δ 7.51 (d, 4H), 7.41 (d, 4H).

### f) 3-(tert-butoxycarbonyl)-5,6-bis(4-chlorophenyl)pyrazine-2-carboxylic acid

To a solution of 2,3-bis(4-chlorophenyl)furo[3,4-*b*]pyrazine-5,7-dione, (877 mg, 2.36 mmol) in acetonitrile (15ml) were added *tert*-butanol (876 mg, 11.8 mmol) and DMAP (346 mg, 2.8 mmol). After 30 minutes the solvent was removed in vacuo and the residue was dissolved in DCM. Washed with 2 M potassium hydrogen sulfate and water followed by drying (magnesium sulfate), filtration and evaporation of the solvent gave a residue which was purified by HPLC to give the title compound (431 mg, 41%).
¹H NMR (400 MHz) δ 7.35-7.17 (m, 8H), 1.57 (s, 9H)
MS *mlz* 445 (M+H)⁺, 443 (M-H)⁻.

### g) tert-butyl 5,6-bis(4-chlorophenyl)-3-[(piperidin-1-ylamino)carbonyl]pyrazine-2-carboxylate

3-(Tert-butoxycarbonyl)-5,6-bis(4-chlorophenyl)pyrazine-2-carboxylic acid, (111 mg, 0.25 mmol) and carbonyldiimidazol (202 mg, 1.26 mmol) were stirred in THF for 3 h and then piperidin-1-amine (30 mg, 0.3 mmol) was added. After 20 h more piperidin-1-amine (56 mg, 0.55 mmol) was introduced. Stirring for 1 h was followed by purification by HPLC to give the target compound (50 mg, 38%). ¹H NMR (400 MHz) δ 8.25 (s, 1H), 7.50-7.30 (m, 8H), 2.93-2.86 (m, 4H), 1.85-1.40 (m, 15H).
MS *mlz* 527, 529 (M+H)⁺, 525, 527 (M-H)⁻.

### Example 2

### Butyl 5,6-bis(4-chlorophenyl)-3-[(piperidin-1-ylamino)carbonyl]pyrazine-2-carboxylate

To a suspension of 2,3-bis(4-chlorophenyl)furo[3,4-*b*]pyrazine-5,7-dione (0.226 g, 0.61 mmol) was dissolved in DCM (1 ml) was added butanol (47 mg, 0.64 mmol). After 2 h a solution of DMAP (10 mg, 0.082 mmol) in DCM (0.1 ml) was added. After an additional 2 h a suspension of HBTU (0.242 g, 0.64 mmol) in DMF (1 ml) was added and after a further 10 minutes piperidin-1-amine (73 mg, 0.73 mmol) and DIEA (76 mg, 0.58 mmol). The mixture was stirred at room temperature for 60 h. The DCM was removed in vacuo and the residue was purified by preparative HPLC to give the title compound 221 mg (69%).
¹H NMR (400 MHz) δ 8.31 (s, 1H), 7.39 (d, 2H), 7.37 (d, 2H), 7.31 (d, 2H), 7.26 (d, 2H), 4.43 (t, 2H), 2.89-2.83 (m, 4H), 1.80-1.68 (m, 6H), 1.48-1.36 (m, 4H), 0.92 (t, 3H)
HRMS *m*/*z* calcd for [C₂₇H₂₈Cl₂N₄O₃]⁺ 527.1617, found 527.1622.

### Example 3

### Cyclohexyl 5,6-bis(4-chlorophenyl)-3-[(piperidin-1-ylamino)carbonyl]pyrazine-2-carboxylate

The title compound was prepared as described in Example 2 using cyclohexanol (64 mg, 0.64 mmol). 129 mg (38%) was obtained.
¹H NMR (400 MHz) δ 8.28 (s, 1H), 7.44-7.25 (m, 8H), 5.19-5.11 (m, 1H), 2.91-2.86 (m, 4H), 2.19-2.10 (m, 2H), 1.88-1.67 (m, 6H), 1.66-1.51 (m, 3H), 1.51-1.33 (m, 4H), 1.30-1.18 (m, 1H).
HRMS *m*/*z* calcd for [C₂₉H₃₁Cl₂N₄O₃]⁺553.1773, found 553.1760.

### Example 4

### Benzyl 5,6-bis(4-chlorophenyl)-3-[(piperidin-1-ylamino)carbonyl]pyrazine-2-carboxylate

The title compound was prepared as described in Example 2 using benzyl alcohol (69 mg, 0.64 mmol). 142 mg (42%) was obtained.
¹H NMR (400 MHz) δ 8.31 (s, 1H), 7.49-7.25 (m, 13H), 5.48 (s, 1H), 2.91-2.84 (m, 4H), 1.81-1.73 (m, 4H), 1.49-1.41 (m, 2H)
HRMS *m*/*z* calcd for [C₃₀H₂₇Cl₂N₄O₃]⁺ 561.1460, found 561.1450.

### Example 5

### tert-butyl 5,6-bis(4-chlorophenyl)-3-({[cis-2-hydroxycyclohexyl]amino}-carbonyl)pyrazine-2-carboxylate

Cis-2-aminocyclohexanol hydrochloride (31 mg, 0.21 mmol) was added to a solution of 3-(tert-butoxycarbonyl)-5,6-bis(4-chlorophenyl)pyrazine-2-carboxylic acid (66 mg, 0.15 mmol) in methylene chloride (3 ml) followed by triethylamine (30 µL, 0.22 mmol) and PyBOP (154 mg, 0.30 mmol). After 3 hr the solvent was removed in vacuo. The residue was purified by preparative HPLC and flash chromatography using 20% ethyl acetate in toluene as solvent to give 27 mg (34%) of the product.
¹H NMR (400 MHz, CDCl₃) δ 7.98 (d, 1H), 7.46-7.36 (m, 4H), 7.36-7.27 (m, 4H), 4.19-4.09 (m, 1H), 4.08-4.02 (m, 1H), 2.01 (broad s, 1H), 1.84-1.37 (m, 17H).
MS m/z calcd for [C28 H29 Cl2 N3 O4]H⁺ 542, found 542

### Example 6

### tert-butyl 5,6-bis(4-chlorophenyl)-3-({[trans-2-hydroxycyclohexyl]amino}-carbonyl)pyrazine-2-carboxylate

Trans-2-aminocyclohexanol hydrochloride was added to a solution of 3-(tert-butoxycarbonyl)-5,6-bis(4-chlorophenyl)pyrazine-2-carboxylic acid in methylene chloride (3 ml). Triethylamine (30 µL, 0.22 mmol) and PyBOP (154 mg, 0.30 mmol) were added. After 24 hr the solvent was removed in vacuo and the residue was purified by flash chromatography using 20% ethyl acetate in toluene as eluent to give 76 mg (95%) of the target compound.
¹H NMR (500 MHz, CDCl₃) δ 7.62 (d, 1H), 7.44 (d, 2H), 7.39 (d, 2H), 7.35 (d, 2H), 7.30 (d, 2H), 3.89-3.80 (m, 1H), 3.52-3.44 (m, 1H), 3.25 (br s, 1H), 2.13-2.04 (m, 2H), 1.90-1.70 (m, 2H), 1.66 (s, 9H), 1.45-1.21 (m, 4H).
HRMS m/z calcd for [C28 H29 Cl2 N3 O4]H⁺ 542.1613, found 542.1624

### Example 7

### tert-butyl 5,6-bis(4-chlorophenyl)-3-({2-[4-(trifluoromethyl)phenyl]hydrazino}-carbonyl)pyrazine-2-carboxylate

5,6-bis(4-chlorophenyl)pyrazine-2,3-dicarboxylic acid (351 mg, 0.902 mmol) was refluxed in acetyl chloride (5 ml) for 2 hours. The excess acetyl chloride was removed in vacuo with the aid of toluene. The residue was dissolved in methylene chloride (5 ml) and a solution of tert-butanol (70 mg, 0.95 mmol) in methylene chloride (5 ml) was added. The mixture was cooled to -78 C and then triethylamine (0.5 ml, 3.6 mmol) and DMAP (22 mg, 0.18 mmol) was added. The mixture was allowed to stir at room temperature for 1 hr and then [4-(trifluoromethyl)phenyl]hydrazine (286 mg, 1.62 mmol) and PyBOP (563 mg, 1.08 mmol) was added. After 16 hrs at room temperature the mixture was worked up by washing with KHSO4 and bicarbonate, evaporation of the solvent in vacuo and purification by preparative HPLC (acetontrile/ammonium acetate) to give 52 mg (9.6%) of the target compound.
¹H NMR (400 MHz, CDCl₃) δ 9.33 (d, 1H), 7.46-7.36 (m, 6H), 7.35-7.27 (m, 4H), 6.94 (d, 1H), 6.85 (d, 2H), 1.58 (s, 9H).
HRMS m/z calcd for [C29 H23 C12 F3 N4 O3]H⁺ 603.1177, found 603.1174

### Example 8

### tert-butyl 5,6-bis(4-chlorophenyl)-3-[(morpholin-4-ylamino)carbonyl]pyrazine-2-carboxylate

(1H-1,2,3-benzotriazol-1-yloxy)(tripyrrolidin-1-yl)phosphonium hexafluorophosphate (421 mg, 0.808 mmol) was added to a solution of 3-(tert-butoxycarbonyl)-5,6-bis(4-chlorophenyl)pyrazine-2-carboxylic acid (300 mg, 0.674 mmol) and triethylamine (0.19 ml, 1.35 mmol) in methylene chloride (5 ml). After 5 minutes morpholin-4-amine (76 mg, 0.74 mmol) was added and the mixture was stirred for 18 hrs. The reaction mixture was diluted with methylene chloride and washed successively with hydrochloric acid, water, bicarbonate solution, and water. After evaporation of the solvent, the residue was purified by preparative HPLC. The resulting material was applied as a solution in methylene chloride to the top of a flash column and then eluted with 20% ethyl acetate in toluene as the eluent to give the product, 178 mg, 50%.
¹H NMR (400 MHz, CDCl₃) δ 8.34 (s, 1H), 7.42 (d, 2H), 7.40-7.31 (m, 4H), 7.28 (d, 2H), 3.88-3.83 (m, 4H), 3.01-2.94 (m, 4H), 1.66 (s, 9H)
HRMS *m*/*z* calcd for [C26 H26 Cl2 N4 O4]H⁺ 529.1409, found 529.1431

### Example 9

### tert-butyl 5,6-bis(4-chlorophenyl)-3-({2-[4-(tert-butylhydrazino}carbonyl)pyrazine-2-carboxylate

(tert-butoxycarbonyl)-5,6-bis(4-chlorophenyl)pyrazine-2-carboxylic acid (316 mg, 0.710 mmol), triethylamine (0.3 ml, 2.13 mmol) and PyBOP (430 mg) were mixed in methylene chloride (5 ml) and after 5 minutes tert-butylhydrazine hydrochloride (96 mg, 0.78 mmol) was added. After 18 h at room temperature the mixture was diluted with more methylene chloride and washed with hydrochloric acid, water, bicarbonate solution and water. After evaporation of the solvent, the residue was purified by HPLC (acetonitrile/ammoniumacetate buffer) to give the title compound.
¹H NMR (400 MHz, CDCl₃) δ 8.71 (s, 1), 7.47-7.42 (m, 2H), 7.41-7.28 (m, 6H), , 4.84 (s, 1H), 1.65 (s, 9H), 1.18 (s, 9H).
HRMS m/z calcd for [C26 H28 C12 N4 O3]H⁺ 515.1617, found 515.1665

### Reference Example 10

### 3-(tert-butoxymethyl)-5,6-bis(4-chlorophenyl)-N-piperidin-1-ylpyrazine-2-carboxamide

### Step A: 5,6-bis(4-chlorophenyl)-3-(ethoxycarbonyl)pyrazine-2-carboxylic acid

2,3-bis(4-chlorophenyl)furo[3,4-*b*]pyrazine-5,7-dione (4.826 g, 13.00 mmol) was suspended in EtOH (160 mL). The suspended material gradually went into solution. After stirring overnight the solvents were evaporated. In order to remove any remaining EtOH the residue was suspended in toluene, whereupon the toluene was evaporated. This was repeated two more times and finally yielded pure 5,6-bis(4-chlorophenyl)-3-(ethoxycarbonyl)pyrazine-2-carboxylic acid as a yellowish solid (5.425 g, 13.00 mmol, quant.).
¹H NMR (500 MHz, CDCl₃) δ 7.50-7.34 (m, 8 H), 4.57 (q, 7.0 Hz, 2H), 1.46 (t, 7.0 Hz, 3H).

### Step B:Ethyl 5,6-bis(4-chlorophenyl)-3-(hydroxymethyl)pyrazine-2-carboxylate

5,6-bis(4-chlorophenyl)-3-(ethoxycarbonyl)pyrazine-2-carboxylic acid (0.562 g, 1.35 mmol) was dissolved in dry THF (13 mL) under Ar. Upon cooling to -20°C triethylamine (0.191 g, 1.89 mmol) was added. Finally, *iso*-butylchloroformiate (0.221 g, 1.62 mmol) was added dropwise during ca 5 min. After stirring at -20°C for 1 h, the temperature was adjusted to 0°C. After stirring at 0°C for another hour the reaction mixture was recooled to -20°C. At this point a small volume (1.7 mL, corresponding to ca 3 eq.) of a pre-prepared solution of sodiumborohydride (0.940 g, 24.8 mmol) in pre-chilled H₂O (10 mL) was added dropwise during ca 5 min to the reaction mixture. Gas evolution could be observed and at the same time the solution became orange-coloured. After stirring at -20°C for ca 1 h the reaction was quenched by the dropwise addition of HCl (aq., 2 M, 5 mL). The reaction mixture was allowed to stir for 1 h (during that time the temperature increased to - 10°C) before it was transferred to a separation funnel with the aid of dichloromethane (100 mL). H₂O (100 mL) was added. The organic phase was sepsarated and the aqueous phase was extracted further with dichloromethane (3 x 30 mL). The collected organic phases were washed with brine (100 mL) before drying with MgSO₄. Upon evaporation of the solvents the obtained residue was dissolved in a minimum volume of dichloromethane-heptane (1:1) and applied on the top of a pre-packed column (silica gel; dichloromethane-heptane 1:1). The products were eluted with two successive gradients: firstly, dichloromethane-heptane 50-100 % and, secondly, EtOAc-dichloromethane 0-6 %. Ethyl 5,6-bis(4-chlorophenyl)-3-(hydroxymethyl)pyrazine-2-carboxylate was obtained as a yellowish viscous oil (0.434 g, 1.08 mmol, 80 %).
¹H NMR (500 MHz, CDCl₃) δ 7.50-7.44 (m, 4H), 7.38-7.32 (m, 4H), 5.17 (d, 5.7 Hz, 2H), 4.52 (q, 7.3 Hz, 2H), 4.25 (t, 5.7 Hz, 1H). 1.48 (t, 7.0 Hz, 3H).

### Step C ethyl 3-(tert-butoxymethyl)-5,6-bis(4-chlorophenyl)pyrazine-2-carboxylate

tert-butyl 2,2,2-trichloroethanimidoate (516 mg, 2.36 mmol) and boron trifluoride diethyl etherate (56 mg, 0.39 mmol) was added at room temperature to a solution of ethyl 5,6-bis(4-chlorophenyl)-3-(hydroxymethyl)pyrazine-2-carboxylate (866 mg, 2.15 mmol) in methylene chloride (0.2 ml) and cyclohexane (5 ml). Workup after 16 hrs by dilution with ethyl acetate and wash with bicarbonate solution. Drying (magnesium sulfate) and removal of the solvent in vacuo, purification by preparative HPLC gave 382 mg (39%) of the target compound.
¹H NMR (400 MHz, CDCl₃) δ 7.42 (d, 2H), 7.40 (d, 2H), 7.32-7.27 (m, 4H), 4.88 (s, 2H), 4.48 (q, 2H), 1.44 (t, 3H), 1.27 (s, 9H)

232 mg of the starting material, ethyl 5,6-bis(4-chlorophenyl)-3-(hydroxymethyl)pyrazine-2-carboxylate was recovered.

### Step D 3-(tert-butoxymethyl)-5,6-bis(4-chlorophenyl)pyrazine-2-carboxylic acid

A mixture of lithium hydroxide (77 mg, 3.33 mmol) in water (6 ml) and ethyl 3-(tert-butoxymethyl)-5,6-bis(4-chlorophenyl)pyrazine-2-carboxylate (382 mg, 0.831 mmol) in acetonitrile (20 ml) was stirred at room temperature for 72 hrs. The solvent was removed in vacuo and the residue was treated with 20 ml 0.3 M KHSO4 and methylene chloride. The organic phase was dried (magnesium sulfate) and removal of the solvent in vacuo gave 327 mg (91%) of the target compound.
¹H NMR (400 MHz, CDCl₃) δ 7.48 (d, 2H), 7.39 (d, 2H), 7.33 (d, 2H), 7.31 (d, 2H), 5.11 (s, 2H), 1.34 (s, 9H)
MS m/z calcd for [C22 H20 C12 N2 O3]H⁺ 431, found 431

### Step E 3-(tert-butoxymethyl)-5,6-bis(4-chlorophenyl)-N-piperidin-1-ylpyrazine-2-carboxamide

3-(tert-butoxymethyl)-5,6-bis(4-chlorophenyl)pyrazine-2-carboxylic acid (324 mg, 0.751 mmol) and triethylamine (84 mg, 0.83 mmol) were dissolved in methylene chloride (20 ml) and the mixture was cooled to -20 degrees. Isobutyl chloridocarbonate (103 mg, 0.751 mmol) was added and after 5 minutes at -20 degrees 1-aminopiperidine hydrochloride (187 mg, 1.37 mmol) and 10 ml 10% aqueous potassium carbonate were introduced. The mixture was stirred for 5 minutes, and then the phases were separated. The organic phase was isolated and the solvent was removed in vacuo to give a residue which was purified by preparative HPLC to give 281 mg, 73%, of the target compound.
¹H NMR (500 MHz, CDCl₃) δ 8.43 (s, 1H), 7.50 (d, 2H), 7.42 (d, 2H), 7.38-7.30 (m, 4H), 5.18 (s, 2H), 2.97-2.91 (m, 4H), 1.84-1.77 (m, 4H), 1.53-1.46 (m, 2H), 1.35 (s, 9H).
HRMS m/z calcd for [C27 H30 C12 N4 O2]H⁺ 513.1824; found 513.1805

### Reference Example 11

### 5,6-bis(4-chlorophenyl)-3-(cyclohexylidenemethyl)-N-piperidin-1-ylpyrazine-2-carboxamide

### Step A: Ethyl 5,6-bis(4-chlorophenyl)-3-formylpyrazine-2-carboxylate

Ethyl 5,6-bis(4-chlorophenyl)-3-(hydroxymethyl)pyrazine-2-carboxylate (0.660 g, 1.64 mmol) was dissolved in dichloromethane (16 mL) and Dess-Martin periodinane (0.729 g, 1.72 mmol) was added in one portion. After stirring for 2 h at ambient temperature a TLC analysis indicated complete consumption of the starting material. Consequently, the reaction was quenched by the sequential addition of Na₂S₂O₃ (aq., 0.2 M, 4 mL) and NaHCO₃ (aq., sat., 4 mL). The resulting mixture was stirred vigorously for 30 min and then allowed to stand overnight. Next morning the mixture was transferred to a separation funnel with the aid of dichloromethane (100 mL). Na₂S₂O₃ (aq., 0.2 M, 15 mL) and NaHCO₃ (aq., sat., 15 mL) were added. The organic phase was separated and the aqueous phase was extracted further with dichloromethane (2 x 30 mL). The collected organic phases were washed with H₂O (25 mL) and brine (75 mL) before drying with MgSO₄. Upon evaporation of the solvents the obtained residue was purified by column chromatography (silica gel, EtOAc-heptane 0-30 %) to yield ethyl 5,6-bis(4-chlorophenyl)-3-formylpyrazine-2-carboxylate as a yellowish, viscous oil (0.519 g, 1.29 mmol, 79 %).
¹H NMR (500 MHz, CDCl₃) δ 10.33 (s, 1H), 7.54-7.50 (m, 4H), 7.40-7.33 (m, 4H), 4.57 (q, 7.3 Hz, 2H), 1.47 (t, 7.0 Hz, 3H).

### Step B ethyl 5,6-bis(4-chlorophenyl)-3-(cyclohexylidenemethyl)pyrazine-2-carboxylate

Under an atmosphere of argon 2 M butyl lithium in pentane (0.3 ml, 0.6 mmol) was added at -78 degrees to a suspension of cyclohexyl(triphenyl)-phosphonium bromide (164 mg, 0.386 mmol) in THF (3 ml). The cooling bath was replaced by an ice bath and the mixture was stirred for 10 minutes at 0 degrees. A dark cherry red clear solution was formed. Cooling again to -78 degrees and addition of ethyl 5,6-bis(4-chlorophenyl)-3-formylpyrazine-2-carboxylate (112 mg, 0.28 mmol) in THF (2 ml). The reaction was kept at -78 for 1 hr and then allowed to attain room temperature. The mixture was diluted with toluene and washed with water, dilute hydrochloric acid, bicarbonate solution, and water. Evaporation of the solvent and purification by preparative HPLC gave 56 mg (43%) of the target compound.
¹H NMR (400 MHz, CDCl₃) δ 7.48 (dd, 4H), 7.30 (d, 4H), 6.69 (s, 1H), 4.48 (q, 2H), 2.76-2.70 (m, 2H), 2.40-2.34 (m, 2H), 1.76-1.69 (m, 2H), 1.68-1.61 (m, 4H), 1.44 (t, 3H).
MS m/z calcd for [C26 H24 C12 N2 O2]H⁺ 467, found 467

### Step C 5,6-bis(4-chlorophenyl)-3-(cyclohexylidenemethyl)pyrazine-2-carboxylic acid

Lithium hydroxide (300 mg, 12.5 mmol) dissolved in water (20 ml) was stirred with a solution of ethyl 5,6-bis(4-chlorophenyl)-3-(cyclohexylidenemethyl)pyrazine-2-carboxylate (131 mg, 0.28 mmol) in THF (50 ml). After 3 hrs the two phase reaction mixture was acidified with potassium hydrogen sulfate and the solvent was removed in vacuo. The remaining aqueous mixture was extracted with ethyl acetate. Drying and evaporation of the solvent, followed by preparative HPLC gave 59 mg of the target compound.
¹H NMR (400 MHz, CD₃OD) δ 7.45-7.35 (m, 4H), 7.30-7.25 (m, 4H), 6.60 (s, 1H), 2.90-2.85 (m, 2H), 2.40-2.35 (m, 2H), 1.75-1.6 (m, 6H)
MS m/z calcd for [C24 H20 Cl2 N2 O2]H⁺ 439, found 439

### Step D 5,6-bis(4-chlorophenyl)-3-(cyclohexylidenemethyl)-N-piperidin-1-ylpyrazine-2-carboxamide

5,6-bis(4-chlorophenyl)-3-(cyclohexylidenemethyl)pyrazine-2-carboxylic acid (34 mg, 0.077 mmol) was dissolved in methylene chloride (1 ml) and oxalyl chloride (400 mg) and DMF (1 drop) was added. After 30 minutes the solvent was removed in vacuo and the last traces of oxalyl chloride were removed by evaporation with the aid of toluene. The residue was dissolved in methylene chloride (1 ml) and added to the amine hydrochloride dissolved in 10% aqueous potassium carbonate (2 ml). After 10 minutes more methylene chloride was added and the aqueous phase was removed. Drying (magnesium sulfate), evaporation of the solvent in vacuo and purification of the residue by preparative HPLC gave 29 mg, 73% of the compound. ¹H NMR (400 MHz, CDCl₃) δ 8.59 (s, 1H), 7.46-7.40 (m, 4H), 7.34-7.25 (m, 4H), 7.22 (s, 1H), 2.97-2.91 (m, 4H), 2.79-2.72 (m, 2H), 2.40-2.35 (m, 2H), 1.80-1.59 (m, 10H), 1.50-1.41 (m, 2H)
HRMS m/z calcd for [C29 H30 C12 N4 O]H⁺ 521.1875, found 521.1871

### Reference Example 12

### 5,6-bis(4-chlorophenyl)-3-(cyanomethyl)-N-piperidin-1-ylpyrazine-2-carboxamide

### Step A Ethyl 5,6-bis(4-chlorophenyl)-3-{[(methylsulfonyl)oxy]methyl}pyrazine-2-carboxylate

Ethyl 5,6-bis(4-chlorophenyl)-3-(hydroxymethyl)pyrazine-2-carboxylate (1184 mg, 2.94 mmol) was dissolved in DCM (15 ml) and TEA (0.614 ml, 4.41 mmol) was added at rt. The reaction mixture was cooled to 0 °C and the mesyl chloride (0.251 ml, 3.23 mmol) was added dropwise. After 40 min at 0 °C the organic phase was washed with ice-cold water, cold 10 % HCl, sat. NaHCO₃ and brine. It was dried (MgSO₄), filtered and evaporated. The residue was used without further purification (1371 mg, 97 %).
¹H NMR (400 MHz, CDCl₃) δ 7.48 (4H, m), 7.33 (4H, m), 5.80 (2H, s), 4.52 (2H, q), 3.14 (3H, s), 1.47 (3H, t).
MS *mlz* 481, 483, 485 (M+H)⁺.

### Step B ethyl 5,6-bis(4-chlorophenyl)-3-(cyanomethyl)pyrazine-2-carboxylate

Ethyl 5,6-bis(4-chlorophenyl)-3-{[(methylsulfonyl)oxy]methyl}pyrazine-2-carboxylate (308 mg, 0.64 mmol), tetrabutylammonium bromide (644 mg, 2 mmol), and sodium cyanide (127 mg, 2.59 mmol) were stirred in methylene chloride (20 ml) and water (0.4 ml) for 3 hrs. The mixture was filtered through silica gel and the solvent was removed in vacuo. Purification by HPLC gave the target compound, 51 mg, 19.3%.
¹H NMR (400 MHz, CDCl₃) δ 7.55-7.46 (m, 4H), 7.36-7.31 (m, 4H), 4.52 (q, 2H), 4.48 (s, 2H), 1.47 (t, 3H)
MS m/z calcd for [C21 H15 Cl2 N3 O2]H⁺ 481, found 481

### Step C 5,6-bis(4-chlorophenyl)-3-(cyanomethyl)pyrazine-2-carboxylic acid

ethyl 5,6-bis(4-chlorophenyl)-3-(cyanomethyl)pyrazine-2-carboxylate (51 mg, 0.12 mmol) and lithium hydroxide (26 mg, 1.1 mmol) were stirred in acetonitrile (10 ml) and water (3 ml) for 3 hrs. Dilution with water, filtration through celite, acidification using 0.3 M KHSO4, extraction with methylene chloride, drying (magnesium sulfate) and evaporation of the solvent gave a residue which was used without further purification.
MS m/z calcd for [C19 H11 C12 N3 O2]-H⁺ 382, found 382

### Step D 5,6-bis(4-chlorophenyl)-3-(cyanomethyl)-N-piperidin-1-ylpyrazine-2-carboxamide

5,6-bis(4-chlorophenyl)-3-(cyanomethyl)pyrazine-2-carboxylic acid (35 mg, 0.091 mmol), piperidin-1-amine hydrochloride (19 mg, 0.14 mmol), and PyBOP (73 mg, 0.14 mmol) were stirred in pyridine (2 ml) over night. The mixture was purified by preparative HPLC to give 21 mg (49%) of the desired product.
¹H NMR (400 MHz, CDCl₃) δ 8.59 (s, 1H), 7.51 (d, 2H), 7.41 (d, 2H), 7.37 (d, 2H), 7.32 (d, 2H), 4.71 (s, 2H), 2.92-2.86 (m, 4H), 1.82-1.74 (m, 4H), 1.52-1.43 (m, 2H)
MS m/z calcd for [C24 H21 C12 N5 O]H⁺ 466, found 466

### Reference Example 13

### 5,6-bis(4-chlorophenyl)-3-(1-methoxyethyl)-N-peridin-1-ylpyrazine-2-carboxamide

### Step A 5,6-bis(4-chlorophenyl)-3-(1-methoxyethyl)pyrazine-2-carboxylic acid

Ethyl 5,6-bis(4-chlorophenyl)-3-(hydroxymethyl)pyrazine-2-carboxylate (1.44 g, 3.58 mmol) was dissolved in dichloromethane (18 mL). Iodomethane (5.086 g, 35.8 mmol) and tetrabutylammonium hydrogensulphate (0.122 g, 0.358 mmol) were added and then, NaOH (aq., 50 % w/w, 18 mL). The mixture quickly adopted a brightly red colour, which then gradually faded with time. Vigorous stirring overnight. The following morning the reaction mixture was cooled to 0 °C before HCl (aq., 4 M, 200 mL) was slowly added (dropwise).
The resulting mixture was transferred to a separation funnel with the aid of dichloromethane (100 mL). The organic phase was separated and the aqueous phase was extracted further with dichloromethane (4 x 100 mL). The collected organic phases were dried with MgSO₄. Upon evaporation of the solvents the obtained residue was purified by column chromatography (silica gel, EtOAc-heptane, 0-40 %; upon reaching 40 % EtOAc formic acid was added to the eluent, 0.5 % v/v) to yield a mixture of 5,6-bis(4-chlorophenyl)-3-(methoxymethyl)pyrazine-2-carboxylic acid and 5,6-bis(4-chlorophenyl)-3-(1-methoxyethyl)pyrazine-2-carboxylic acid (1.130 g) as a brownish semi-solid

### Step B Methyl 5,6-bis(4-chlorophenyl)-3-(1-methoxyethyl)pyrazine-2-carboxylate

The mixture from step A (1.13 g, 2.90 mmol) was dissolved in dichloromethane (28 mL), whereupon MeOH (7 mL) was added. Trimethylsilyl diazomethane (1.8 mL, 2.0 M in hexanes, 3.6 mmol) was added dropwise at such a rate as to keep the evolution of nitrogen under control. Stirring overnight at ambient temperature. Next morning silica gel (ca 5 g) was added to the reaction mixture and the solvents were evaporated. The obtained solid residue was put on top of a pre-packed column (silica gel, heptane) and the product was eluted by two rounds of column chromatography: 1) silica gel, EtOAc-heptane, 0-25 %, methyl 5,6-bis(4-chlorophenyl)-3-(1-methoxyethyl)pyrazine-2-carboxylate elutes slightly faster than methyl 5,6-bis(4-chlorophenyl)-3-(methoxymethyl)pyrazine-2-carboxylate; 2) silica gel, dichloromethane-heptane, 0-100 %. Methyl 5,6-bis(4-chlorophenyl)-3-(1-methoxyethyl)pyrazine-2-carboxylate (9 mg, 0.02 mmol, 0.7 %) was obtained as a colourless viscous oil.
¹H NMR (500 MHz, CDCl₃) δ 7.47 (d, 2H, J=8.5 Hz), 7.44 (d, 2H, J=8.5 Hz), 7.38-7.30 (m, 4H), 5.00 (q, 1H, J=6.5 Hz), 4.04 (s, 3H), 3.38 (s, 3H), 1.66 (d, 3H, J=6.5 Hz).

### Step C 5,6-bis(4-chlorophenyl)-3-(1-methoxyethyl)-N-piperidin-1-ylpyrazine-2-carboxamide

The hydrochloride of piperidin-1 amine (0.683 g, 5.00 mmol) was suspended in dry toluene (5.0 mL) under Ar. Upon cooling to 0 °C trimethylaluminium (2.5 mL, 2 M in toluene, 5.0 mmol) was added dropwise. After the addition of Me₃Al was complete the ice bath was removed and the mixture was allowed to stir for 3 h at ambient temperature; consequently, a ca 0.67 M solution of aluminium amide was obtained. A part of this solution (0.75 mL, 0.50 mmol, corresponding to ca 25 eq.) was added to methyl 5,6-bis(4-chlorophenyl)-3-(1-methoxyethyl)pyrazine-2-carboxylate (9 mg, 0.02 mmol), which had been placed in a dry flask under Ar. Heating at 50 °C overnight. Next morning the reaction mixture was cooled to 0 °C and toluene (1 mL) was added before HCl (aq., 2 M, 10 mL) was slowly added (dropwise). The obtained mixture was transferred to a separation funnel with the aid of dichloromethane (30 mL) and water (30 mL). The organic phase was separated and the aqueous phase was extracted further with dichloromethane (3 x 10 mL). The collected organic phases were washed with water (10 mL) and brine (30 mL) before drying with MgSO₄. Upon evaporation of the solvents the obtained residue was purified by column chromatography (silica gel, EtOAc-heptane, 0-40 %) to yield 5,6-bis(4-chlorophenyl)-3-(1-methoxyethyl)-*N*-piperidin-1-ylpyrazine-2-carboxamide (6 mg, 0.01 mmol) as a white solid.
¹H NMR (500 MHz, CDCl₃) δ 8.48 (br s, 1H, N-*H*), 7.50 (d, 2H, J=8.5 Hz), 7.43 (d, 2H, J=8.5 Hz), 7.37 (d, 2H, J=8.5 Hz), 7.32 (d, 2H, J=8.5 Hz), 5.77 (q, 1H, J=6.3 Hz), 3.41 (s, 3H), 3.00-2.84 (m, 4H), 1.86-1.74 (m, 4H), 1.64 (d, 3H, J=6.3 Hz), 1.54-1.44 (m, 2H).
HRMS Calcd for [C₂₅H₂₆Cl₂N₄O₂+H]⁺: 485.1511. Found: 485.1526.

### Reference Example 14

### tert-butyl 5,6-bis(4-chlorophenyl)-3-{[(2-hydroxy-1-methylethyl)amino]carbonyl}-pyrazine-2-carboxylate

Triphenylphosphine (230 mg, 0.876 mmol) dissolved in 2 ml acetonitrile/pyridine 1:1 was added to a suspension of (tert-butoxycarbonyl)-5,6-bis(4-chlorophenyl)pyrazine-2-carboxylic acid (260 mg, 0.584 mmol), carbon tetrachloride (1 ml, 10.4 mmol), triethylamine (0.12 ml, 0.88 mmol) and DIEA (113 mg, 0.88 mmol) in 5 ml acetonitrile/pyridine 1:1 at room temperature. The mixture was stirred over the weekend to give a clear brown reaction mixture. It consisted to a large part of starting material. More triphenyl phosphine (250 mg, 0.95 mmol) was added and another portion of DIEA (227mg, 1.75 mmol) was added. The reaction mixture turned reddish. The mixture was stirred for 6 hrs at room temperature and then ice was added. After 30 minutes most of the solvent was removed at about 35 °C and the residue was partitioned between methylene chloride and 0.3 M KHS04. The organic extract was washed with sodium hydrogen carbonate solution and then dried (magnesium sulfate). Evaporation of the solvent gave a dark brown residue.

Flash chromatography using toluene/EtOAc with increasing amounts of EtOAc and then preparative HPLC gave tert-butyl 5,6-bis(4-chlorophenyl)-3-{[(2-hydroxy-1-methylethyl)amino]carbonyl}-pyrazine-2-carboxylate 19 mg, 6.5%.
¹H NMR (600 MHz, CDCl₃) δ 7.72 (d, 1H), 7.43 (d, 2H), 7.39 (d, 2H), 7.34 (d, 2H), 7.29 (d, 2H), 4.30-4.23 (m, 1H), 3.77 ("d", 1H), 3.69-3.64 (m, 1H), 2.66 (br s, 1H), 1.66 (s, 9H), 1.30 (d, 3H).
HRMS m/z calcd for [C25 H25 Cl2 N3 O4]H⁺ 502.1300, found 502.1297

### Example 15

### tert-butyl 5,6-bis(4-chlorophenyl)-3-{[(4,4-difluorocyclohexyl)amino]carbonyl}pyrazine-2-carboxylate

(tert-butoxycarbonyl)-5,6-bis(4-chlorophenyl)pyrazine-2-carboxylic acid (86 mg, 0.19 mmol), (4,4-difluorocyclohexyl)amine (37 mg, 0.29 mmol), and PyBOP (220 mg, 0.42 mmol) were mixed in pyridine (2 ml) and stirred for 3 hrs. Purification by preparative HPLC gave 68 mg (63%) of the target compound.
¹H NMR (400 MHz, CDCl₃) δ 7.53 (d, 1H), 7.44 (d, 2H), 7.40-7.34 (m, 4H), 7.31 (d, 2H), 4.19-4.08 (m, 1H), 2.21-2.06 (m, 4H), 2.01-1.83 (m, 2H), 1.75-1.63 (m, 11H).
HRMS m/z calcd for [C28 H27 C12 F2 N3 O3]H⁺ 562.1476, found 562.1516

### Example 16

### tert-butyl 5,6-bis(4-chlorophenyl)-3-[(pentylamino)carbonyl]pyrazine-2-carboxylate

(tert-butoxycarbonyl)-5,6-bis(4-chlorophenyl)pyrazine-2-carboxylic acid (76 mg, 0.17 mmol), pentan-1-amine (87 mg, 1 mmol), and PyBOP (181 mg, 0.348 mmol) were stirred in pyridine (2 ml) for 20 hrs. Purification by preparative HPLC gave 71 mg (81 %) of the target compound.
¹H NMR (400 MHz, CDCl₃, mixture of rotamers) δ 7.66 and 7.58 (t, 1H), 7.44 (d, 2H), 7.39 (d, 2H), 7.35 (d, 2H), 7.30 (d, 2H), 3.54-3.28 (m, 2H), 1.75-1.59 (m, 11H), 1.58-1.17 (m, 2H), 0.99-0.90 (m, 5H)
HRMS m/z calcd for [C27 H29 Cl2 N3 O3]H⁺ 514.1664, found 514.1663

### Example 17

### tert-butyl 5,6-bis(4-chlorophenyl)-3-{[(1-ethylpropyl)amino]carbonyl}pyrazine-2-carboxylate

(tert-butoxycarbonyl)-5,6-bis(4-chlorophenyl)pyrazine-2-carboxylic acid (100 mg, 0.22 mmol), (1-ethylpropyl)amine (29 mg, 0.34 mmol), and PyBOP (240 mg, 0.46 mmol) were stirred in pyridine (2 ml) for 25 hrs. Purification by preparative HPLC gave 95 mg (82%) of the title compound.
¹H NMR (400 MHz, CDCl₃) δ 7.44 (d, 2H), 7.39 (d, 2H), 7.35 (d, 2H), 7.30 (d, 2H), 4.07-3.96 (m, 1H), 1.74-1.61 (m, 11H), 1.59-1.46 (m, 2H), 0.96 (t, 6H)
HRMS m/z calcd for [C27 H29 C12 N3 O3]H⁺ 514.1664, found 514.1701

### Example 18

### tert-butyl 5,6-bis(4-chlorophenyl)-3-{[(4,4-difluoropiperidin-1-yl)amino]carbonyl}-pyrazine-2-carboxylate

(tert-butoxycarbonyl)-5,6-bis(4-chlorophenyl)pyrazine-2-carboxylic acid (121 mg, 0.27 mmol), 4,4-difluoropiperidin-1-amine (55 mg, 0.41 mmol), and PyBOP (283 mg, 0.543 mmol) were stirred in pyridine (2 ml) over the weekend. The mixture was diluted with methylene chloride and washed with dilute hydrochloric acid and bicarbonate solution, dried (magnesium sulfate) and the solvent was removed in vacuo. Purification of the residue by preparative HPLC gave 93 mg (61 %) of the target compound.
¹H NMR (400 MHz, CDCl₃) δ 8.39 (s, 1H), 7.43 (d, 2H), 7.40-7.34 (m, 4H), 7.31 (d, 2H), 3.13-3.07 (m, 4H), 2.28-2.15 (m, 4H), 1.68 (s, 9H)
HRMS m/z calcd for [C27 H26 Cl2 F2 N4 O3]H⁺ 563.1428, found 563.1418

### Example 19

### 5,6-bis(4-chlorophenly)-N-piperidin-1-yl-3-[(4-propyl-1H-1,2,3-triazol-1-yl)methyl]pyrazine-2-carboxamide

### Step A Ethyl 3-(azidomethyl)-5,6-bis(4-chlorophenyl)pyrazine-2-carboxylate

Ethyl 5,6-bis(4-chlorophenyl)-3-{[(methylsulfonyl)oxy]methyl}pyrazine-2-carboxylate (1360 mg, 2.86 mmol) was dissolved in DMF (10 ml) and NaN₃ (275.5 mg, 4.24 mmol) was added followed by one drop of water. The reaction mixture was stirred at rt for 2 h 20 min whereafter it was poured into water and the water phase was extracted three times with toluene. Then the combined organic phase was washed three times with water, dried (MgSO₄), filtered and evaporated. The residue was used without further purification (1163 mg, 96 %).

¹H NMR (400 MHz, CDCl₃) δ 7.50 (4H, m), 7.33 (4H, m), 4.91 (2H, s), 4.51 (2H, q), 1.46 (3H, t).

MS *mlz* 428, 430, 432 (M+H)⁺.

### Step B 3-(azidomethyl)-5,6-bis(4-chlorophenyl)pyrazine-2-carboxylic acid

Ethyl 3-(azidomethyl)-5,6-bis(4-chlorophenyl)pyrazine-2-carboxylate (1152 mg, 2.69 mmol) was dissolved in THF (10 ml) and LiOH (261.6 mg, 10.92 mmol) dissolved in water (5 ml) was added. The reaction mixture was stirred at rt for 1 h 15 min. The solvent was evaporated and more water was added. The water phase was acidified with 10 % HCl and extracted three times with DCM. The combined organic phase was then washed three times with water before the solvent was removed under reduced pressure. The residue was used without further purification (1.0 g, 95 %).

¹H NMR (400 MHz, (CD₃)₂SO) δ 7.48 (8H, m), 4.91 (2H, s).

MS *mlz* 400, 402, 404 (M+H)⁺.

### Step C 3-(azidomethyl)-5,6-bis(4-chlorophenyl)pyrazine-2-carbonyl chloride

3-(azidomethyl)-5,6-bis(4-chlorophenyl)pyrazine-2-carboxylic acid (1.0 g, 2.56 mmol) was dissolved in DCM (14 ml) and oxalylchloride (1.08 ml, 12.81 mmol) was added at rt followed by one drop of DMF which resulted in gas formation. The mixture was stirred at rt for 40 min whereafter the solvents were removed under reduced pressure. The residue was used without further purification (1.0 g, 95 %).
¹H NMR (400 MHz, CDCl₃) δ 7.58 (4H, m), 7.37 (4H, m), 4.86 (2H, s).
MS *m*/*z* 414, 416, 418 (M+H)⁺.

### Step D 3-(azidomethyl)-5,6-bis(4-chlorophenyl)-N-piperidin-1-ylpyrazine-2-carboxamide

A mixture of 1-aminopiperidine (687.1 mg, 5.03 mmol), TEA (1.36 ml, 9.75 mmol) and DCM (10 ml) was added to 3-(azidomethyl)-5,6-bis(4-chlorophenyl)pyrazine-2-carbonyl chloride (1.0 g, 2.44 mmol) dissolved in DCM (10 ml). The reaction mixture was stirred at rt for 2.5 days whereafter the organic phase was extracted twice with water before the solvent was removed under reduced pressure. The residue was purified by flash chromatography (SiO₂, DCM:ethyl acetate, 0 % to 7 % ethyl acetate) to yield the title compound (1005 mg, 85 %) as a pale yellow powder.
¹H NMR (400 MHz, CDCl₃) δ 8.54 (1H, s), 7.41 (8H, m), 5.12 (2H, s), 2.90 (4H, s), 1.78 (4H, m), 1.47 (2H, s).
MS *m*/*z* 482, 484, 486 (M+H)⁺.

### Step E 5,6-bis(4-chlorophenyl)-N-piperidin-1-yl-3-[(4-propyl-1H-1,2,3-triazol-1-yl)methyl]pyrazine-2-carboxamide

Pentyne (0.021 ml, 0.210 mmol) was added to 3-(azidomethyl)-5,6-bis(4-chlorophenyl)-N-piperidin-1-ylpyrazine-2-carboxamide, (50.6 mg, 0.105 mmol) dissolved in a 1:1 mixture of water and *tert*-butyl alcohol (1.4 ml). Sodium ascorbate was dissolved in a minimum amount of water and added to the reaction mixture. The copper sulphate also dissolved in a minimum amount of water was added to the reaction mixture which made it change colour from white to red. The reaction mixture was stirred at rt for 5 h whereafter one more eq of pentyn, 0.2 eq of sodium ascorbate, 0.1 eq of copper sulphate and THF (2 ml) were added. The temperature was increased to 40 °C. After three days the reaction mixture was diluted with DCM and washed three times with water before the solvents were removed under reduced pressure. The residue was purified by flash chromatography (SiO₂, DCM:ethyl acetate, 10 % to 100 % ethyl acetate) to yield the title compound (23.3 mg, 40 %).
¹H NMR (400 MHz, CDCl₃) δ 8.53 (1H, s), 7.61 (1H, s), 7.35 (4H, m), 7.25 (4H, m), 6.28 (2H, s), 2.88 (4H, s), 2.72 (2H, t), 1.78 (4H, m), 1.71 (2H, q), 1.47 (2H, s), 0.96 (3H, t).
MS *mlz* 550 (M+H)⁺.

### Reference Example 20

### 5,6-bis(4-chlorophenyl)-3-{[4-(1-hydroxyethyl)-1H-1,2,3-triazol-1-yl]methyl}-N-piperidin-1-ylpyrazine-2-carboxamide

### Reference Example 21

### 5,6-bis(4-chlorophenyl)-3-{[5-(1-hydroxyethyl)-1H-1,2,3-triazol-1-yl]methyl}-N-piperidin-1-ylpyrazine-2-carboxamide

1-butyn-3-ol (0.72 ml, 9.23 mmol) was added to a suspension of 3-(azidomethyl)-5,6-bis(4-chlorophenyl)-N-piperidin-1-ylpyrazine-2-carboxamide, (890 mg, 1.85 mmol) in toluene (8 ml). The reaction mixture was stirred at 65 °C for four days, added 5 more equivalent of 1-butyn-3-ol after 18 h. The solvent was removed under reduced pressure and the regio isomers were separated by preparatory HPLC (kromasil C8 column, ammonium acetate (aq, 0.1 M):acetonitrile) to yield Ex. 20 (460 mg, 45 %) and Ex. 21 (405 mg, 39 %) as pale yellow powders.
Ex. 20: ¹H NMR (500 MHz, CDCl₃) δ 8.56 (1H, s), 7.84 (1H, s), 7.38 (4H, m), 7.27 (4H, m), 6.33 (2H, s), 5.12 (1H, q), 2.91 (4H, s), 1.81 (4H, m), 1.62 (3H, d), 1.50 (2H, s).
MS *m*/*z* 552, 554, 556 (M+H)⁺.
Ex. 21: ¹H NMR (500 MHz, CDCl₃) δ 8.53 (1H, s), 7.72 (1H, s), 7.27 (8H, m), 6.43 (2H, AB-pattem), 5.07 (1H, q), 2.91 (4H, s), 1.80 (4H, m), 1.61 (3H, d), 1.49 (2H, s).
MS *m*/*z* 552, 554, 556 (M+H)⁺.

### Reference Example 22

### tert-butyl {[1-({5,6-bis(4-chlorophenyl)-3-[(piperidin-1-ylamino)carbonyl]pyrazin-2-yl}methyl)-1H-1,2,3-triazol-4-yl]methyl}carbamate

### Reference Example 23

### tert-butyl {[1-({5,6-bis(4-chlorophenyl)-3-[(piperidin-1-ylamino)carbonyl]pyrazin-2-yl}methyl)-1H-1,2,3-triazol-5-yl]methyl}carbamate

3-(azidomethyl)-5,6-bis(4-chlorophenyl)-N-piperidin-1-ylpyrazine-2-carboxamide, (92.3 mg, 0.191 mmol) was dissolved in toluene (1 ml) and *tert*-butyl prop-2-yn-1-ylcarbamate (148.5 mg, 0.957 mmol) was added. The reaction mixture was heated to 65 °C under N₂. After 16 h another 3 equivalents of *tert*-butyl prop-2-yn-1-ylcarbamate were added. After 24 h the temperature was decreased to rt for 2.5 days thenthe temperature was increased to 65 °C again and 3 more equivalents of *tert*-butyl prop-2-yn-1-ylcarbamate were added. After another 24 h the solvent was removed under reduced pressure. The isomers were separated by preparative HPLC (kromasil Cyano, heptan/ethyl acetate/FA/TEA 70/30/0.1/0.05) to yield Ex. 22 (9.4 mg, 8 %) and Ex. 23 (2.6 mg, 2 %) as pale yellow powders.
Ex. 22: ¹H NMR (400 MHz, CDCl₃) δ 8.56 (1H, s), 7.83 (1H, s), 7.37 (4H, m), 7.26 (4H, m), 6.32 (2H, s), 5.10 (1H, s), 4.44 (2H, d), 2.90 (4H, t), 1.80 (4H, m), 1.49 (2H, m), 1.44 (9H, s).
MS *mlz* 637, 639, 641 (M+H)⁺.
Ex.23: ¹H NMR (400 MHz, CDCl₃) δ 8.56 (1H, s), 7.70 (1H, s), 7.26 (8H, m), 6.35 (2H, s), 4.98 (1H, s), 4.42 (2H, d), 2.90 (4H, s), 1.79 (4H, m), 1.48 (2H, m), 1.32 (9H, s).
MS *m*/*z* 637, 639, 641 (M+H)⁺.

### Reference Example 24

### 3-{[4-(aminomethyl)-1H-1,2,3-triazol-1-yl]methyl}-5,6-bis(4-chlorophenyl)-N-piperidin-1-ylpyrazine-2-carboxamide hydrochloride

A mixture of thionyl chloride (0.05 ml, 0.674 mmol) and methanol (0.2 ml) prepared at subzero temperature was added to *tert*-butyl {[1-({5,6-bis(4-chlorophenyl)-3-[(piperidin-1-ylamino)carbonyl]pyrazin-2-yl}methyl)-1H-1,2,3-triazol-4-yl]methyl}carbamate (8.6 mg, 0.014 mmol) dissolved in methanol (0.05 ml) at subzero temperature. The ice bath was removed after the addition. After 2 h 20 min the solvent was removed under reduced pressure to yield the title compound (5.8 mg, 74 %) as a pale yellow powder.
¹H NMR (500 MHz, CD₃OD) δ 7.55 (1H, s), 7.35 (8H, m), 6.36 (2H, s), 4.29 (2H, s), 3.46 (4H, s), 1.96 (4H, s), 1.66 (2H, s).
MS *mlz* 537, 539, 541 (M+H)⁺.

### Reference Example 25:

### 3-{[5-(aminomethyl)-1H-1,2,3-triazol-1-yl]methyl}-5,6-bis(4-chlorophenyl)-N-piperidin-1-ylpyrazine-2-carboxamide hydrochloride

*Tert*-butyl {[1-({5,6-bis(4-chlorophenyl)-3-[(piperidin-1-ylamino)carbonyl]pyrazin-2-yl}methyl)-1H-1,2,3-triazol-5-yl]methyl}carbamate (2.6 mg, 0.004 mmol) was treated with thionyl chloride (0.05 ml, 0.689 mmol) in methanol (0.2 ml) as described in Ex 24, to give the title compound (1.9 mg, 80 %) as a pale yellow powder.
¹H NMR (400 MHz, CD₃OD) δ 7.96 (1H, s), 7.40 (9H, m), 6.47 (2H, s), 4.47 (2H, s), 3.49 (4H, s), 1.98 (4H, s), 1.67 (2H, s).
MS *m*/*z* 537, 539, 541 (M+H)⁺.

### Example 26 5,6-bis(4-chlorophenyl)-3-(phenoxymethyl)-N-piperidin-1-ylpyrazine-2-carboxamide

### Step A Ethyl 5,6-bis(4-chlorophenyl)-3-(phenoxymethyl)pyrazine-2-carboxylate

Ethyl 5,6-bis(4-chlorophenyl)-3-(hydroxymethyl)pyrazine-2-carboxylate (0.157 g, 0.389 mmol) was dissolved in dry THF (3.9 mL) under Ar. Phenol (0.055 g, 0.584 mmol) and triphenylphosphine 0.123 g, 0.467 mmol) were added. Upon cooling to 0°C diethyl azodicarboxylate (0.075 g, 0.43 mmol) was added dropwise. After stirring at 0°C for 2 h TLC analysis indicated full conversion of the starting material and the the flask was put in a freezer overnight awaiting purification. Next morning the reaction mixture was transferred to a bigger flask with the aid of dichloromethane. Silica gel (1 g) was added and the solvents were evaporated. The obtained solid residue was put on top of a pre-packed column (silica gel, heptane) and the products were eluted with an EtOAc-dichloromethane gradient (0-70 %). A second round of column chromatography gave ethyl 5,6-bis(4-chlorophenyl)-3-(phenoxymethyl)pyrazine-2-carboxylate as viscous colourless oil (0.129 g, 0.27 mmol, 69 %).
¹H NMR (500 MHz, CDCl₃) δ 7.48-7.40 (m, 4H), 7.35-7.29 (m, 6H), 7.02-6.98 (m, 3H), 5.58 (s, 2H), 4..44 (q, 7.0 Hz, 2H), 1.35 (t, 7.0 Hz, 3H).
HRMS Calcd for [C₂₆H₂₀Cl₂N₂O₃+H]⁺: 479.0929. Found: 479.0938.

### Step B 5,6-bis(4-chlorophenyl)-3-(phenoxymethyl)-N-piperidin-1-ylpyrazine-2-carboxamide

Piperidine-1-amine hydrochloride (0.221 g, 1.62 mmol) was dissolved in dichloromethane (1.5 mL) under Ar and trimethylaluminium (1.65 mL, 2.0 M in toluene, 3.3 mmol) was carefully added during ca 5 min. The solution was stirred at ambient temperature for 1.5 h and, as a result, a ca 0.51 M solution of an amide reagent was obtained. 0.7 mL (0.36 mmol, ca 2 eq.) of this stock solution was added to ethyl 5,6-bis(4-chlorophenyl)-3-(phenoxymethyl)pyrazine-2-carboxylate (0.085 g, 0.18 mmol) which had been placed in a dry round flask under Ar. A red solution was obtained, which was stirred at 50°C overnight. Next morning the reaction mixture was transferred to a separation funnel with the aid of dichloromethane (30 mL). HCl (aq, 2 M, 30 mL) was added. The organic phase was separated and the aqueous phase was extracted further with dichloromethane (3 x 10 mL). The collected organic phases were washed consecutively with H₂O (15 mL) and brine (30 mL) before drying with MgSO₄. Upon evaporation of the solvents the residue was purified by column chromatography (silica gel; EtoAc/heptane, 0-40 %) to yield 5,6-bis(4-chlorophenyl)-3-(phenoxymethyl)-*N*-piperidin-1-ylpyrazine-2-carboxamide as a crispy, yellowish solid (0.079 g, 0.15 mmol, 84 %).
¹H NMR (500 MHz, CDCl₃) δ 8.51 (br s, 1H), 7.42-7.22 (m, 10H), 7.03-6.93 (m, 3H), 5.87 (s, 2H), 2.92-2.89 (m, 4H), 1.82-1.75 (m, 4H), 1.50-1.46 (m, 2H).
HRMS Calcd for [C₂₉H₂₆Cl₂N₄O₂+H]⁺: 533.1511. Found: 533.1480.

### Example 27

### 5,6-bis(4-chlorophenyl)-3-(morpholin-4-ylmethyl)-N-piperidin-1-ylpyrazine-2-carboxamide

### Step A Ethyl 5,6-bis(4-chlorophenyl)-3-(morpholin-4-ylmethyl)pyrazine-2-carboxylate

Ethyl 5,6-bis(4-chlorophenyl)-3-formylpyrazine-2-carboxylate (0.115 g, 0.287 mmol) was dissolved in 1,2-dichloroethane (1.0 mL). Morpholine (0.027 g, 0.32 mmol) was added (yielding an orange-coloured solution) and finally sodium triacetoxyborohydride (0.091 g, 0.43 mmol). The reaction was allowed to stir overnight. Next morning TLC analysis indicated full conversion of the starting material. The reaction mixture was transferred to a separation funnel with the aid of EtOAc (30 mL). NaHCO₃ (aq., sat., 30 mL) was added. The organic phase was separated and the aqueous phase was extracted further with EtOAc (3 x 10 mL). The collected organic phases were washed with H₂O (15 mL) and brine (30 mL) before drying with MgSO₄. Upon evaporation of the solvents the residue was purified by column chromatography (silica gel; EtOAc-dichloromethane 0-20 %) to yield ethyl 5,6-bis(4-chlorophenyl)-3-(morpholin-4-ylmethyl)pyrazine-2-carboxylate as a viscous colourless oil (0.125 g, 0.264 mmol, 92 %).
¹H NMR (500 MHz, CDCl₃) δ 7.46-7.41 (m, 4H), 7.34-7.29 (m, 4H), 4.49 (q, 7.0 Hz, 2H), 3.99 (s, 2H), 3.69-3.66 (m, 4H), 2.55-2.52 (m, 4H), 1.47 (t, 7.3 Hz, 3H).
HRMS Calcd for [C₂₄H₂₃Cl₂N₃O₃+H]⁺: 472.1195. Found: 472.1182.

### Step B 5,6-bis(4-chlorophenyl)-3-(morpholin-4-ylmethyl)-N-piperidin-1-ylpyrazine-2-carboxamide

Piperidine-1-amine hydrochloride (0.221 g, 1.62 mmol) was dissolved in dichloromethane (1.5 mL) under Ar and trimethylaluminium (1.65 mL, 2.0 M in toluene, 3.3 mmol) was slowly added during ca 5 min. The solution was stirred at ambient temperature for 1.5 h and, as a result, a ca 0.51 M solution of an amide reagent was obtained. 0.8 mL (0.36 mmol, ca 2 eq.) of this stock solution was added to ethyl 5,6-bis(4-chlorophenyl)-3-(morpholin-4-ylmethyl)pyrazine-2-carboxylate (0.094 g, 0.20 mmol) which had been placed in a dry round flask under Ar. A red solution was obtained, which was stirred at 50°C overnight. Next morning the reaction mixture was transferred to a separation funnel with the aid of dichloromethane (30 mL). H₂O (30 mL) was added. The pH of the aqueous phase was adjusted to ca 7 with NaOH (aq, 2 M). The organic phase was separated and the aqueous phase was extracted further with dichloromethane (4 x 10 mL). The collected organic phases were washed with brine (30 mL) before drying with MgSO₄. Upon evapoartion of the solvents the residue was purified by column chromatography (silica gel; EtOAc-dichloromethane 0-100 %) to yield 5,6-bis(4-chlorophenyl)-3-(morpholin-4-ylmethyl)-*N*-piperidin-1-ylpyrazine-2-carboxamide as a viscous yellowish oil (0.077 g, 0.15 mmol, 73 %).
¹H NMR (500 MHz, CDCl₃) δ 8.63 (br s, 1H), 7.44-7.37 (m, 4H), 7.36-7.27 (m, 4H), 4.27 (s, 2H), 3.73-3.69 (m, 4H), 2.92-2.88 (m, 4H), 2.71-2.68 (m, 4H), 1.81-1.74 (m, 4H), 1.49-1.46 (m, 2H).
HRMS Calcd for [C₂₇H₂₉Cl₂N₅O₂+H]⁺: 526.1777. Found: 526.1781.

### Example 28

### 5,6-bis(4-chlorophenyl)-3-(piperidin-1-ylmethyl)-N-piperidin-1-ylpyrazine-2-carboxamide

### Step A Ethyl 5,6-bis(4-chlorophenyl)-3-(piperidin-1-ylmethyl)pyrazine-2-carboxylate

Ethyl 5,6-bis(4-chlorophenyl)-3-formylpyrazine-2-carboxylate (0.069 g, 0.17 mmol) was dissolved in 1,2-dichloroethane (0.6 mL). Piperidine (0.016 g, 0.19 mmol) was added (yielding an orange-coloured solution) and finally sodium triacetoxyborohydride (0.055 g, 0.26 mmol). The reaction was allowed to stir overnight. Next morning the reaction mixture was transferred to a separation funnel with the aid of EtOAc (30 mL). NaHCO₃ (aq., sat., 30 mL) was added. The organic phase was separated and the aqueous phase was extracted further with EtOAc (2 x 10 mL). The collected organic phases were washed with H₂O (10 mL) and brine (25 mL) before drying with MgSO₄. Upon evaporation of the solvents the residue was purified by column chromatography (silica gel; EtOAc-dichloromethane 0-50 %) to yield ethyl 5,6-bis(4-chlorophenyl)-3-(piperidin-1-ylmethyl)pyrazine-2-carboxylate as a yellowish solid (0.051 g, 0.11 mmol, 63 %).
¹H NMR (500 MHz, CDCl₃) δ 7.44-7.39 (m, 4H), 7.32-7.25 (m, 4H), 4.46 (q, 7.1 Hz, 2H), 3.90 (s, 2H), 2.55-2.35 (m, 4H), 1.57-1.48 (m, 4H), 1.48-1.35 (m, 5H).
HRMS Calcd for [C₂₅H₂₅Cl₂N₃O₂+H]⁺: 470.1402. Found: 470.1368.

### Step B 5,6-bis(4-chlorophenyl)-3-(piperidin-1-ylmethyl)-N-piperidin-1-ylpyrazine-2-carboxamide

Piperidine-1-amine hydrochloride (0.221 g, 1.62 mmol) was dissolved in dichloromethane (1.5 mL) under Ar and trimethylaluminium (1.65 mL, 2.0 M in toluene, 3.3 mmol) was added slowly during ca 5 min. The solution was stirred at ambient temperature for 1.5 h and, as a result, a ca 0.51 M solution of an amide reagent was obtained. 0.39 mL (0.20 mmol, ca 3 eq.) of this stock solution was added to ethyl 5,6-bis(4-chlorophenyl)-3-(piperidin-1-ylmethyl)pyrazine-2-carboxylate (0.032g, 0.068 mmol) which had been placed in a dry round flask under Ar. A red solution was obtained, which was stirred at 50°C overnight. Next morning the reaction mixture was cooled to 0°C. Dichloromethane (ca 3 mL) was added followed by HCl (aq., 2 M, ca 1 mL). The mixture was transferred to a separation funnel with the aid of dichloromethane (30 mL) and H₂O (30 mL) was added. The pH of the aqueous phase was adjusted to ca ca 8 with NaOH (aq, 2 M). The organic phase was separated and the aqueous phase was extracted further with dichloromethane (4 x 10 mL). The collected organic phases were washed with brine (30 mL) before drying with MgSO₄. Upon evaporation of the solvents the residue was purified by column chromatography (silica gel; EtOH-toluene 0-6 %) to yield 5,6-bis(4-chlorophenyl)-3-(piperidin-1-ylmethyl)-*N*-piperidin-1-ylpyrazine-2-carboxamide as a yellow, viscous oil (0.029 g). Further purification by preparative HPLC (CH₃CN-H₂O (NH₄OAc buffer), 75-100 % over 60 min) yielded the desired compound (0.017 g, 0.032 mmol, 48 %).
¹H NMR (500 MHz, CDCl₃) δ 9.43 (br s, 1H), 7.47-7.37 (m, 4H), 7.33-7.26 (m, 4H), 4.08 (s, 2H), 2.93-2.90 (m, 4H), 2.66-2.46 (m, 4H), 1.90-1.68 (m, 4H), 1.68-1.54 (m, 4H), 1.54-1.40 (m, 4H).
HRMS Calcd for [C₂₈H₃₁Cl₂N₅O+H]⁺: 524.1984. Found: 524.2031.

### Reference Example 29

### 5,6-bis(4-chlorophenyl)-3-[(cyclohex-2-en-1-yloxy)methyl]-N-piperidin-1-ylpyrazine-2-carboxamide

### Step A 5,6-bis(4-chlorophenyl)-3-[(cyclohex-2-en-1-yloxy)methyl]pyrazine-2-carboxylic acid

Ethyl 5,6-bis(4-chlorophenyl)-3-(hydroxymethyl)pyrazine-2-carboxylate (0.139 g, 0.345 mmol) was dissolved in dichloromethane (1.7 mL). 3-bromocyclohexene (0.139 g, 0.862 mmol) and tetrabutylammonium hydrogensulphate (0.012 g, 0.034 mmol) were added. Finally, NaOH (aq., 50 % w/w, 1.7 mL) was added immediately producing an intensely red solution. After vigorous stirring for 4 h another portion of 3-bromocyclohexene (0.139 g, 0.862 mmol) was added and the reaction was stirred overnight. Next morning the reaction mixture was transferred to a separation funnel with the aid of dichloromethane (30 mL). HCl (aq., 2 M, 30 mL) was added. The organic phase was separated and the aqueous phase was extracted further with dichloromethane (3 x 10 mL). The collected organic phases were washed with brine (30 mL) before drying with MgSO₄. Upon evaporation of the solvents the obtained residue was purified by column chromatography (silica gel). ). The products were eluted with two successive gradients: firstly, EtOAc-heptane 0-50 % and, secondly, EtOAc-heptane 50-60 % with HCOOH (1 % v/v) added to the eluent. 5,6-bis(4-chlorophenyl)-3-[(cyclohex-2-en-1-yloxy)methyl]pyrazine-2-carboxylic acid was obtained as a brownish, amorphous solid (0.084 g, 0.18 mmol, 53 %).
¹H NMR (500 MHz, CDCl₃) δ 7.50-7.31 (m, 8H), 5.90 (br s, 2H), 5.29 (d, 12.5 Hz, 1H), 5.22 (d, 12.5 Hz, 1H), 4.22-4.17 (m, 1H), 2.16-1.46 (m, 6H).

### Step B Methyl 5,6-bis(4-chlorophenyl)-3-[(cyclohex-2-en-1-yloxy)methyl]pyrazine-2-carboxylate

5,6-bis(4-chlorophenyl)-3-[(cyclohex-2-en-1-yloxy)methyl]pyrazine-2-carboxylic acid (0.073 g, 0.16 mmol) was dissolved in dichloromethane (1.6 mL). MeOH (0.4 mL) was added. (trimethylsilyl)diazomethane (105 µL, 0.21 mmol, 2 M in hexanes) was added dropwise at such a rate as to keep the gas evolution under control. After stirring overnight the reaction mixture was transferred to a bigger flask with the aid of dichloromethane. Silica gel (1 g) was added and the solvents were evaporated. The obtained residue was put on top of a pre-packed column (silica gel; heptane) and the products were eluted with an EtOAc-heptane gradient (0-10 %). Methyl 5,6-bis(4-chlorophenyl)-3-[(cyclohex-2-en-1-yloxy)methyl]pyrazine-2-carboxylate was obtained as a viscous, colourless oil (0.057 g, 0.12 mmol, 76 %).
¹H NMR (500 MHz, CDCl₃) δ 7.46-7.39 (m, 4H), 7.33-7.27 (m, 4H), 5.95-5.75 (m, 2H), 5.06 (d, 12.1 Hz, 1H), 5.00 (d, 12.1 Hz, 1H), 4.09-4.02 (m, 1H), 4.01 (s, 3 H), 2.20-1.40 (m, 6H).
HRMS Calcd for [C₂₅H₂₂Cl₂N₂O₃+H]⁺: 469.1086. Found: 469.1083.

### Step C 5,6-bis(4-chlorophenyl)-3-[(cyclohex-2-en-1-yloxy)methyl]-N-piperidin-1-ylpyrazine-2-carboxamide

Piperidine-1-amine hydrochloride (0.272 g, 2.00 mmol) was dissolved in dichloromethane (2.0 mL) under Ar and trimethylaluminium (2.0 mL, 2.0 M in toluene, 4.0 mmol) was carefully added during ca 5 min. The solution was stirred at ambient temperature for 1.5 h and, as a result, a ca 0.50 M solution of an amide reagent was obtained. 0.56 mL (0.28 mmol, ca 3 eq.) of this stock solution was added to methyl 5,6-bis(4-chlorophenyl)-3-[(cyclohex-2-en-1-yloxy)methyl]pyrazine-2-carboxylate (0.044g, 0.094 mmol) which had been placed in a dry round flask under Ar. A red solution was obtained, which was stirred at 50°C overnight. Next morning the reaction mixture was cooled to 0°C. Toluene (ca 3 mL) was added followed by HCl (aq., 2 M, ca 1 mL). The mixture was transferred to a separation funnel with the aid of dichloromethane (30 mL) and H₂O (30 mL) was added. The organic phase was separated and the aqueous phase was extracted further with dichloromethane (4 x 10 mL). The collected organic phases were washed with brine (30 mL) before drying with MgSO₄. Upon evaporation of the solvents the obtained residue was purified by column chromatography (silica gel; EtOAc-heptane 0-70 %) to yield 5,6-bis(4-chlorophenyl)-3-[(cyclohex-2-en-1-yloxy)methyl]-*N*-piperidin-1-ylpyrazine-2-carboxamide as a yellowish solid (0.036 g, 0.067 mmol, 71 %).
¹H NMR (500 MHz, CDCl₃) δ 8.43 (br s, -NH, 1H), 7.51-7.31 (m, 8H), 5.88 (br s, 2H), 5.24 (d, 12.5 Hz, 1H), 5.19 (d, 12.5 Hz, 1H), 4.16-4.12 (m, 1H), 3.00-2.80 (m, 4H), 2.20-1.40 (m, 12H).
HRMS Calcd for [C₂₉H₃₀Cl₂N₄O₂+H]⁺: 537.1824. Found: 537.1823.

### Reference Example 30

### 5,6-bis(4-chlorophenyl)-3-[(cyclohexyloxy)methyl]-N-piperidin-1-ylpyrazine-2-carboxamide

### Step A Ethyl 3-(bromomethyl)-5,6-bis(4-chlorophenyl)pyrazine-2-carboxylate

Ethyl 5,6-bis(4-chlorophenyl)-3-(hydroxymethyl)pyrazine-2-carboxylate (0.100 g, 0.248 mmol) was dissolved in dichloromethane (2.5 mL) and triphenylphosphine (0.072 g, 0.27 mmol) was added. Upon cooling to 0°C carbon tetrabromide (0.123 g, 0.372 g) was added in one portion. The solution gradually turned yellow. After stirring at 0°C for 1 h the ice bath was removed and the solution was allowed ro reach ambient temperature. Stirring overnight. Next morning the consumption of starting material was complete based on TLC. The reaction mixture was transferred to a bigger flask with the aid of dichloromethane. Silica gel (1 g) was added and the solvents were evaporated. The obtained residue was put on top of a pre-packed column (silica gel; heptane) and the products were eluted with a dichloromethane-heptane gradient (0-70 %). Ethyl 3-(bromomethyl)-5,6-bis(4-chlorophenyl)pyrazine-2-carboxylate was obtained as a colourless oil (0.061 g, 0.13 mmol, 53 %).
¹H NMR (500 MHz, CDCl₃) δ 7.52-7.42 (m, 4H), 7.38-7.28 (m, 4H), 5.05 (s, 2H), 4.54 (q, 7.2 Hz, 2H), 1.48 (t, 7.1 Hz, 3H).
HRMS Calcd for [C₂₀H₁₅Cl₂N₂O₂+H]⁺: 464.9772. Found: 464.9785.

### Step B 5,6-bis(4-chlorophenyl)-3-[(cyclohexyloxy)methyl]pyrazine-2-carboxylic acid

Ethyl 3-(bromomethyl)-5,6-bis(4-chlorophenyl)pyrazine-2-carboxylate (0.045 g, 0.097 mmol) was dissolved in dichloromethane (1.5 mL). Tetrabutylammonium hydrogensulphate (0.003 g, 0.01 mmol) and cyclohexanol (0.048 g, 0.48 mmol) were added. Finally, NaOH (aq., 50 % w/w, 0.5 mL) was added and the solution took on an orangy colour. The mixture was stirred vigorously overnight. Next morning the reaction mixture was transferred to a separation funnel with the aid of dichloromethane (30 mL). HCl (aq., 2 M, 30 ml) was added. The organic phase was separated and the aqueous phase was extracted further with dichloromethane (3 x 10 mL). The collected organic phases were washed with brine (30 mL) before drying with MgSO₄. The solvents were evaporated and the obtained crude residue, containing 5,6-bis(4-chlorophenyl)-3-[(cyclohexyloxy)methyl]pyrazine-2-carboxylic acid, was used as such in the next step.

### Step C Methyl 5,6-bis(4-chlorophenyl)-3-[(cyclohexy)methyl]pyrazine-2-carboxylate

Crude 5,6-bis(4-chlorophenyl)-3-[(cyclohexyloxy)methyl]pyrazine-2-carboxylic acid (see above) was dissolved in dichloromethane (2.0 mL). MeOH (0.5 mL) was added. Finally, (trimethylsilyl)diazomethane (100 µL, 0.2 mmol, 2 M in hexanes) was added dropwise at such a rate as to keep the gas evolution under control. After stirring overnight the reaction mixture was transferred to a bigger flask with the aid of dichloromethane. Silica gel (0.5 g) was added and the solvents were evaporated. The obtained residue was put on top of a pre-packed column (silica gel; heptane) and the products were eluted with an EtOAc-heptane gradient (0-25 %). Methyl 5,6-bis(4-chlorophenyl)-3-[(cyclohexyloxy)methyl]pyrazine-2-carboxylate was obtained as a colourless semi-solid (0.006 g, 0.013 mmol, 13 %).
¹H NMR (500 MHz, CDCl₃) δ 7.47-7.38 (m, 4H), 7.34-7.27 (m, 4H), 4.98 (s, 2H), 4.01 (s, 3H), 3.49-3.39 (m, 1H), 2.02-1.90 (m, 2H), 1.82-1.68 (m, 2H), 1.42-1.14 (m, 6H).

### Step D 5,6-bis(4-chlorophenyl)-3-[(cyclohexyloxy)methyl]-N-piperidin-1-ylpyrazine-2-carboxamide

Piperidine-1-amine hydrochloride (0.342 g, 2.50 mmol) was dissolved in dichloromethane (2.5 mL) under Ar and trimethylaluminium (2.5 mL, 2.0 M in toluene, 5.0 mmol) was carefully added during ca 5 min. The solution was stirred at ambient temperature for 1.5 h and, as a result, a ca 0.50 M solution of an amide reagent was obtained. 0.50 mL (0.25 mmol, ca 24 eq.) of this stock solution was added to methyl 5,6-bis(4-chlorophenyl)-3-[(cyclohexyloxy)methyl]pyrazine-2-carboxylate (0.005g, 0.011 mmol) which had been placed in a dry round flask under Ar. A red solution was obtained, which was stirred at 50°C overnight. Next morning the reaction mixture was cooled to 0°C. Dichloromethane (ca 3 mL) was added followed by HCl (aq., 2 M, ca 1 mL). The mixture was transferred to a separation funnel with the aid of dichloromethane (30 mL) and H₂O (30 mL) was added. The organic phase was separated and the aqueous phase was extracted further with dichloromethane (3 x 10 mL). The collected organic phases were washed with H₂O (10 mL) and brine (30 mL) before drying with MgSO₄. Upon evaporation of the solvents the obtained residue was purified by column chromatography (silica gel; EtOAc-heptane 0-50 %) to yield 5,6-bis(4-chlorophenyl)-3-[(cyclohexyloxy)methyl]-*N*-piperidin-1-ylpyrazine-2-carboxamide as a viscous, yellowish oil (0.004 g, 0.0074 mmol, 70 %).
¹H NMR (500 MHz, CDCl₃) δ 8.38 (br s, 1H), 7.48-7.42 (m, 2H), 7.42-7.27 (m, 6H), 5.23 (s, 2H), 3.63-3.52 (m, 1H), 2.98-2.81 (m, 4H), 2.12-1.96 (m, 2H), 1.86-1.70 (m, 4H), 1.50-1.14 (m, 10H).
HRMS Calcd for [C₂₉H₃₂Cl₂N₄O₂+H]⁺: 539.1981. Found: 539.1987.

### Reference Example 31

### 5,6-Bis(4-chlorophenyl)-N-(2-hydroxyethyl)-N'-piperidin-1-ylpyrazine-2,3-dicarboxamide;

PyBOP (228 mg, 0.439 mmol), dissolved in DCM (1 ml), was added to a mixture of 5,6-bis(4-chlorophenyl)-3-[piperidin-1-ylamino)carbonyl]pyrazine-2-carboxylic acid (120 mg, 0.255 mmol) and 2-aminoethanol (25 µl, 0.415 mmol) in DCM (4 ml) and TEA (0.5 ml), at 0°C. The reaction was continued at 0°C for 15 minutes and thereafter at room temperature 4 hours. A precipitate had been formed. The solvent was evaporated and the mixture dissolved in ethyl acetate, extracted with water and dried over MgSO₄. The product was purified by flash chromatography (SiO₂, toluene:ethyl acetate 1:9) and thereafter preparatory HPLC (kromasil C8 column, ammonium acetate (aq, 0.1M):acetonitrile, product came at about 80% acetonitrile) to give the title compound as a white powder (55 mg, 42%).
¹H NMR (400 MHz, THF-d8) δ 8.74 and 8.20 (s, 1H), 8.42-8.33 and 8.25-8.18 (m, 1H), 7.60-7.48 (m, 4H), 7.41-7.31 (m, 4H), 4.30-3.90 (br, 1H), 3.70-3.52 (m, 2H), 3.52-3.43 (m, 2H), 2.95-2.40 (m, 4H), 1.70-1.62 and 1.38-1-10 (m, 4H), 1.46-1.38 and 1.38-1.10 (m, 2H).
¹³C NMR (100 MHz, CDCl₃) δ 170.25, 165.84, 163.78, 162.61, 152.18, 151.22, 150.73, 148.96, 148.10, 143.88, 142.19, 138.32, 136.32, 136.21, 136.00, 135.61, 135.23, 135.10, 131.23, 129.09, 129.01, 61.86, 61.38, 56.90, 42.79, 42.46, 25.39, 25.18, 23.25, 23.04.
HRMS Calcd for [C₂₅H₂₅N₅O₃Cl₂+H]⁺: 514.141. Found: 514.144.

### Reference Example 32

### 5,6-bis(4-chlorophenyl)-N-(3-hydroxybutyl)-N'-piperidin-1-ylpyrazine-2,3-dicarboxamide;

PyBOP (245 mg, 0.470 mmol), dissolved in DCM (1 ml), was added to a mixture of 5,6 bis(4-chlorophenyl)-3-[piperidin-1-ylamino)carbonyl]pyrazine-2-carboxylic acid (136 mg, 0.289 mmol) and 4-amino-2-butanol (40 mg, 0.449 mmol) in DCM (4 ml) and TEA (1 ml), at 0°C. The reaction was continued at 0°C for 15 minutes and thereafter at room temperature 5 hours. The mixture was extraced with water and dried over MgSO₄. The residue was purified by flash chromatography (SiO₂, ethyl acetate) to give the title compound as a slightly yellow powder (104 mg, 67%).
¹H NMR (400 MHz, CDCl₃) δ 8.24 and 7.81 (t, 1H), 7.86 and 7.60 (s, 1H), 7.45-7.25 (m, 8H), 3.96-3.88 and 3.88-3.73 (m, 1H), 3.88-3.73 and 3.46-3.30 (m, 2H), 3.40-3.20 and 2.80-2.50 (br, 1H), 2.96-2.88 and 2.88-2.40 (m, 4H), 1.80-1.53 (m, 2H), 1.80-1.65 and 1.35-1.20 (m, 4H), 1.45-1.35 and 1.20-1.10 (m, 2H), 1.20-1.10 (m, 3H).
¹³C NMR (100 MHz, CDCl₃) δ 170.20, 165.13, 163.63, 152.32, 151.02, 150.80, 148.93, 148.31, 143.41, 142.95, 137.99, 136.28, 136.30, 136.19, 136.02, 135.74, 135.66, 135.32, 135.30, 131.27, 131.22, 129.13, 129.02, 65.74, 65.38, 56.92, 38.81, 38.47, 37.27, 36.78, 25.43, 23.60, 23.52, 23.36, 23.05.
HRMS Calcd for [C₂₇H₂₉N₅O₃Cl₂+H]⁺: 542.173. Found: 542.176.

### Reference Example 33

### 5,6-Bis(4-chlorophenyl)-N-(3-hydroxypropyl)-N'-piperidin-1-ylpyrazine-2,3-dicarboxamide

PyBOP (193 mg, 0.371 mmol), dissolved in DCM (1 ml), was added to a mixture of 5,6-bis(4-chlorophenyl)-3-[piperidin-1-ylamino)carbonyl]pyrazine-2-carboxylic acid (91 mg, 0.194 mmol), 1-amino-2-propanol (111 mg, 1.47 mmol) and TEA (1 ml), in DCM (3 ml) at 0°C. The reaction was continued at 0°C for 15 minutes and thereafter at room temperature 5 hours. The mixture was extracted with water and dried over MgSO₄. The residue was purified by flash chromatography (SiO₂, toluene:ethyl acetate, product came at about 80% ethyl acetate) to give the title compound as a slightly yellow powder (69 mg, 67%).
¹H NMR (400 MHz, CDCl₃) δ 8.20-8.12 and 7.60-7.52 (m, 1H), 7.73-7.68 and 7.26-7.21 (m, 1H), 7.44-7.24 (m 8H), 4.16-4.06 and 4.06-3.96 (m, 1H), 3.65-3.51 and 3.40-3.29 (m, 2H), 2.96-2.88 and 2.88-2.50 (m, 4H), 1.77-1.67 and 1.35-1.20 (m, 4H), 1.48-1.38 and 1.35-1.20 (m, 2H), 1.25-1.15 (m, 3H).
¹³C NMR (100 MHz, CDCl₃) δ170.12, 165.84, 163.71, 162.52, 152.25, 151.35, 150.68, 148.94, 148.24, 144.20, 142.12, 138.29, 136.36, 136.19, 136.01, 135.73, 135.63, 135.25, 135.15, 131.25, 131.20, 129.12, 129.01, 67.40, 66.81, 56.94, 47.58, 47.15, 25.38, 25.15, 23.29, 23.06, 21.04, 20.78.
HRMS Calcd for [C₂₆H₂₇N₅O₃Cl₂+H]⁺: 528.157. Found: 528.156.

### Example 34

### Tert-butyl 5,6-bis(4-methylphenyl)-3-[(piperidin-1-ylamino)carbonyl]pyrazine-2-carboxylate

### Step A 5,6- Bis(4-methylphenyl)pyrazine-2,3-dicarbonitrile

1,2-Bis(4-methylphenyl)ethane-1,2-dione (3.0 g, 12.59 mmol) and (2Z)-2,3-diaminobut-2-enedinitrile (1.5 g, 13.88 mmol) were refluxed 29 h in ethanol/water (3:1, 40 ml) and acetic acid (1 ml). The mixture was cooled to room temperature and 30 ml water was added. The product was collected by filtration and washed with water, ethanol and diethylether. Finally the product was purified by recrystallisation from DCM/heptane (3.0 g, 76%)
¹H NMR (400 MHz, CDCl₃) δ 7.45 (d, 4H), 7.16 (d, 4H), 2.39 (s, 6H).
MS m/z 309 (M-H)⁻.

### Step B 5,6-Bis(4-methylphenyl)pyrazine-2,3-dicarboxylic acid

5,6- Bis(4-methylphenyl)pyrazine-2,3-dicarbonitrile (1.5g, 4.83 mmol), potassium hydroxide (2.79 g, 49.78 mmol) and hydrogen peroxide (13 ml, 30%, aq) were suspended in water (100 ml) and heated to 35 °C were the temperature was maintained 1h to allow foam formation to decraese. The temperature was thereafter slowly increased to 65 °C and the reaction thereafter continued at that temperture 40 h. The mixture was cooled to room temperature and the pH decreased to 3 by the addition of HCl (aq). The product was collected by filtration and washed with water (450 ml) to give the title compound as a yellowish powder (1.4 g, 83%).
¹H NMR (400 MHz, CD₃OD) δ 7.40 (d, 4H), 7.11 (d, 4H), 2.32 (s, 6H).
MS m/z 349 (M+H)⁺.

### Step C 2,3-bis(4-methylphenyl)furo[3,4-b]pyrazine-5,7-dione

5,6-Bis(4-methylphenyl)pyrazine-2,3-dicarboxylic acid (590mg, 1.69mmol) was refluxed 15 h in acetylchloride (15 ml, 210.2 mmol). The solution was cooled to room temperature and the product dried by coevaporation with toluene at reduced pressure three times to give the title compound (559 mg, 99%).
¹H NMR (400 MHz, CDCl₃) δ 7.47 (d, 4H), 7.15 (d, 4H), 2.38 (s, 6H).

### Step D 3-(tert-butoxycarbonyl)-5,6-bis(4-methylphenyl)pyrazine-2-carboxylic acid

To a suspension of 2,3-bis(4-methylphenyl)furo[3,4-*b*]pyrazine-5,7-dione (160mg,0.484 mmol) in DCM (3ml) were added a solution of *Tert*-butanol (54 mg, 0.727 mmol) in DCM (1 ml) and 4-dimethylaminopyridine (74 mg, 0.605 mmol). The reaction was continued at room temperature 2.5 hours. The solvent was evaporated and toluene and HCl (2M) were added. The phases were separated and the organic phase extracted with water and dried over MgSO₄. The product contained about 30% 5,6-bis(4-methylphenyl)pyrazine-2,3-dicarboxylic acid but used without further purification in Step E (104 mg, 53% estimated yield).
¹H NMR (400 MHz, CDCl₃) δ 10.40-9.90 (br, 1H), 7.45-7.30 (m, 4H), 7.15-7.07 (m, 4H), 2.36 (s, 6H), 1.70 (s, 9H).
MS m/z 405 (M+H)⁺.

### Step E Tert-butyl 5,6-bis(4-methylphenyl)-3-[(piperidin-1-ylamino)carbonyl]pyrazine-2-carboxylate

PyBOP (312 mg, 0.599 mmol), dissolved in DCM (1ml), was added to a mixture of 3-(*tert*-butoxycarbonyl)-5,6-bis(4-methylphenyl)pyrazine-2-carboxylic acid (105 mg, 0.259 mmol) and piperidin-1-aminium chloride (78 mg, 0.569 mmol) and TEA (1 ml), in DCM (4 ml) at 0°C. The reaction was continued at 0°C for 15 minutes and thereafter at room temperature 4 hours. The mixture was extraced with water and dried over MgSO₄. It was purified by flash chromatography (SiO₂, toluene:ethyl acetate 9:1) to give the title compound as a yellowish powder (115 mg, 91%).
¹H NMR (400 MHz, CDCl₃) δ 8.36 (s, 1H), 7.48-7.32 (m, 4H), 7.27-7.07 (m, 4H), 2.94-2.82 (m, 4H), 2.38 (s, 3H), 2.34 (s, 3H), 1.82-1.72 (m, 4H), 1.68 (s, 9H), 1.50-1.40 (m, 2H).
HRMS Calcd for [C₂₉H₃₄N₄O₃+H]⁺: 487.271. Found: 487.271.

### Example 35

### 5,6-Bis(4-methylphenyl)-N-piperidin-1-yl-3-(1H-tetrazol-1-ylmethyl)pyrazine-2-carboxamide

### Step A 3-Ethoxycarbonyl)-5,6-bis(4-methylphenyl)pyrazine-2-carboxylic acid

A mixture of 2,3-bis(4-methylphenyl)furo[3,4-b] pyrazine-5,7-dione (399 mg, 1.21 mmol) and ethanol (10 ml, 173.8 mmol) in DCM (5 ml) was stirred at room temperature for 28 hours. The product was dried by cooevaporation with toluene at reduced pressure. The product contained about 10% 5,6-bis(4-methylphenyl)pyrazine-2,3-dicarboxylic acid but used without further purification in the next step (451 mg, 89%).
¹H NMR (400 MHz, CDCl₃) δ 10.80-10.50 (br, 1H), 7.45-7.30 (m, 4H), 7.15-7.00 (m, 4H), 4.55 (q, 2H), 2.34 (s, 6H), 1.45 (t, 3H).
MS m/z 377 (M+H)⁺.

### Step B Ethyl 3-(hydroxymethyl)-5,6-bis(4-methylphenyl)pyrazine-2-carboxlate

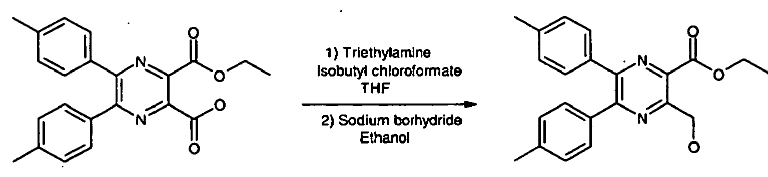

3-Ethoxycarbonyl)-5,6-bis(4-methylphenyl)pyrazine-2-carboxylic acid (451 mg, 1.08 mmol) was dissolved in THF and cooled to -78°C. TEA (210 µl, 1.51 mmol) and isobutyl chloroformate (182 µl, 1.40 mmol) were added dropwise and the reaction continued at - 78°C for 80 minutes and thereafter at 0°C for 70 minutes. The mixture was thereafter added to a freshly prepared suspension of sodium borohydride (106 mg, 2.80 mmol) in ethanol (10 ml) which had been prepared at 0°C. The reaction was continued at 0°C for 1.5 hours and then quenched by addition of acetic acid (3 ml). It was allowed to warm to room temperature and DCM and water were added. The organic phase was washed with NaHCO₃ (10%, aq) and water and dried over MgSO₄. The solvent was removed at reduced pressure at room temperature. The residue was purified by flash chromatography (SiO₂, DCM:ethyl acetate, product came at about 1% ethyl acetate) to give the title compound (151 mg, 38%).
¹H NMR (400 MHz, CDCl₃) δ 7.46-7.40 (m, 4H), 7.16-7.10 (m, 4H), 5.15 (d, 2H), 4.49 (q, 2H), 2.36 (s, 3H), 2.35 (s, 3H), 1.46 (t, 3H).
MS m/z 363 (M+H)⁺.

### Step C Ethyl 5,6-bis(4-methylphenyl)-3-(1H-tetrazol-1-ylmethyl)pyrazine-2-carboxylate

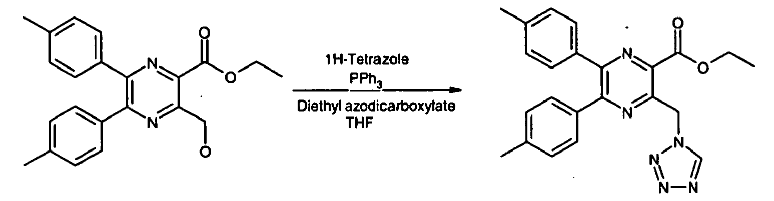

To a mixture of Ethyl 3-(hydroxymethyl)-5,6-bis(4-methylphenyl)pyrazine-2-carboxylate (85 mg, 0.234 mmol), 1*H*-tetrazole (26 mg, 0.371 mmol) and triphenylphosphine (74 mg, 0.283 mmol) in dry THF (3 ml) at 0°C under N₂(g) was added diethyl azodicarboxylate (49 µl, 0.260 mmol) dropwise and stirring was continued at 0°C under N₂(g) for 3 hours and thereafter at room temperature under N₂(g) for 2 hours. The solvent was evaporated and the product purified by preparatory HPLC (kromasil C8 column, ammonium acetate (aq, 0.1M):acetonitrile, product came at about 100% acetonitrile) to give the title compound (20 mg, 20%).
¹H NMR (400 MHz, CDCl₃) δ 8.91 (s, 1H), 7.41 (d, 2H), 7.23 (d, 2H), 7.12 (d, 2H), 7.06 (d, 2H), 6.26 (s, 2H), 4.52 (q, 2H), 2.35 (s, 3H), 2.33 (s, 3H), 1.47 (t, 3H).
MS m/z 415 (M+H)⁺.

### Step D 5,6.Bis(4-methylphenyl)-N-piperidin-1-yl-3-(1H-tetrazol-1-ylmethyl)pyrazine-2-carboxamide

Trimethylaluminium solution (2 ml, 2M in toluene) was added to a solution of piperidin-1-aminium chloride (272 mg, 2.72 mmol) in DCM (2ml). It was stirred for 1h at room temperature under N₂(g). 290µl of the solution was added to ethyl 5,6-bis(4-methylphenyl)-3-(1*H*-tetrazol-1-ylmethyl)pyrazine-2-carboxylate (20 mg, 0.048 mmol) and stirring was continued at room temperature under N₂(g) for 5 hours. The mixture was cooled to 0°C, diluted with DCM (2ml) and quenched with HCl (conc.). The mixture was thereafter neutralised with NaOH (aq). The phases were separated and the aqueousphase extracted with DCM. The combined organic phase was washed with water and dried over MgSO₄. Finally the product was purified by flash chromatography (SiO₂, toluene:ethyl acetate, product came at about 40% ethyl acetate) to give the title compound as a yellowish powder (11 mg, 50%).
¹H NMR (400 MHz, CDCl₃) δ 9.01 (s, 1H), 8.66 (s, 1H), 7.35 (d, 2H), 7.23 (d, 2H), 7.17 (d, 2H), 7.07 (d, 2H), 6.41 (s, 2H), 2.92-2.84 (m, 4H), 2.39 (s, 3H), 2.33 (s, 3H), 1.85-1.75 (m, 4H), 1.55-1.45 (m, 2H).
HRMS Calcd for [C₂₆H₂₈N₈O+H]⁺: 469.246. Found: 469.247.

### Example 36

### 5,6-Bis(4-methylphenyl)-N-piperidin-1-yl-3-(2H-tetrazol-2-ylmethyl)pyrazine-2-carboxamide

### Step A Ethyl 5,6-bis(4-methylphenyl)-3-(2H-tetrazol-2-ylmethyl)pyrazine-2-carboxylate

The titled compound (28 mg, 28%) was formed as a isomer in Step C Example 35 and was isolated by preparatory HPLC (kromasil C8 column, ammonium acetate (aq, 0.1M):acetonitrile, product came at about 100% acetonitrile)
¹H NMR (400 MHz, CDCl₃) δ 8.58 (s, 1H), 7.41 (d, 2H), 7.19 (d, 2H), 7.12 (d, 2H), 7.02 (d, 2H), 6.50 (s, 2H), 4.50 (q, 2H), 2.35 (s, 3H), 2.30 (s, 3H), 1.46 (t, 3H).
MS m/z 415 (M+H)⁺.

### Step B 5,6-Bis(4-methylphenyl)-N-piperidin-1-yl-3-(2H-tetrazol-2-ylmethyl)pyrazine-2-carboxamide

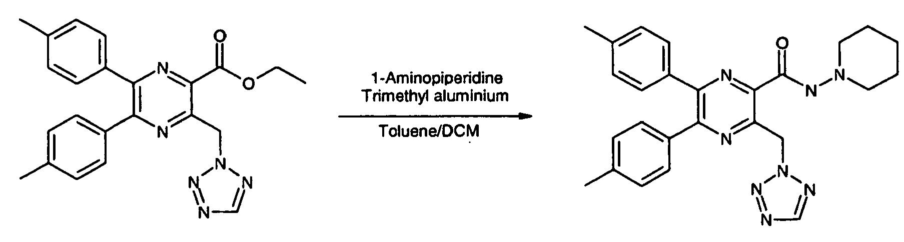

It was prepared as described in Example 35 step D. Purification was performed by preparatory HPLC (kromasil C8 column, ammonium acetate (aq, 0.1M):acetonitrile, product came at about 100% acetonitrile) to give the title compound as a white powder (13 mg, 42%).
¹H NMR (400 MHz, CDCl₃) δ8.85-8.65 (br, 1H), 8.59 (s, 1H), 7.35 (d, 2H), 7.16 (d, 2H), 7.08 (d, 2H), 6.99 (d, 2H), 6.68 (s, 2H), 2.96-2.88 (m, 4H) 2.38 (s, 3H), 2.29 (s, 3H), 1.83-1.74 (m, 4H), 1.52-1.43 (m, 2H).
HRMS Calcd for [C₂₆H₂₈N₈O+H]⁺: 469.246. Found: 469.246.

### Example 37

### 5,6-Bis(4-chlorophenyl)-N-piperidin-1-yl-3-(2H-tetrazol-2-ylmethyl)pyrazine-2-carboxamide

### Step A Ethyl 5,6-bis(4-chlorophenyl)-3-(2H-tetrazol-2-ylmethyl)pyrazine-2-carboxylate and ethyl 5,6-bis(4-chlorophenyl)-3-(1H-tetrazol-1-ylmethyl)pyrazine-2-carboxlate

To a solution of ethyl 5,6-bis(4-chlorophenyl)-3-(hydroxymethyl)pyrazine-2-carboxylate (0.32 g, 0.80 mmol) in tetrahydrofuran (10 ml) were added 1H-tetrazol (84 mg, 1.20 mmol) and triphenylphosphine (0.25 g, 0.96 mmol). Upon cooling to 0°C, diethyl azodicarboxylate (0.16 ml, 0.84 mmol) was added dropwise. The reaction mixture was stirred at 0°C for 1h. The solvent was removed under reduced pressure and separation by prepHPLC gave two isomers:ethyl 5,6-bis(4-chlorophenyl)-3-(2*H*-tetrazol-2-ylmethyl)pyrazine-2-carboxylate (180 mg, 50%) as a white solid.
¹H NMR (400 MHz, CDCl3) δ 8.60 (s, 1H), 7.46 (d, 2H), 7.33 (d, 2H), 7.22 (d, 4H), 6.52 (s, 2H), 4.53 (q, 2H), 1.47 (t, 3H).
MS *m*/*z* 455 (M+H)⁺.
and ethyl 5,6-bis(4-chlorophenyl)-3-(1*H*-tetrazol-1-ylmethyl)pyrazine-2-carboxylate (88 mg, 24%) as a white solid.
¹H NMR (400 MHz, CDCl3) δ 8.89 (s, 1H), 7.46 (d, 2H), 7.34 (d, 2H), 7.27 (d, 4H), 6.29 (s, 2H), 4.55 (q, 2H), 1.49 (t, 3H).
MS *m*/*z* 455 (M+H)⁺.

### Step B 5,6-bis(4-chlorophenyl)-N-piperidin-1-yl-3-(2H-tetrazol-2-ylmethyl)pyrazine-2-carboxamide

Piperidine-1-amine hydrochloride (0.54 g, 3.98 mmol) was dissolved in dichloromethane (4.0 ml) under Ar and trimethylaluminium (2.0 ml, 2.0 M in toluene, 4 mmol) was carefully added during 5 min. The solution was stirred at ambient temperature for 1.5 h. 2.4 ml (1.2 mmol) of this solution was added to ethyl 5,6-bis(4-chlorophenyl)-3-(2H-tetrazol-2-ylmethyl)pyrazine-2-carboxylate (180 mg, 0.40 mmol) and the reaction mixture was stirred at 45°C overnight. It was cooled to 0°C and quenched with HCl (aq, 2 M, 2.5 mL). The mixture was diluted with dichloromethane and neutralized with addition of KOH (aq, 2 M). The organic phase was separated and the aqueous phase was extracted further with dichloromethane. The collected organic phases were washed with H₂O before drying with Na₂SO₄. The solvent was removed under reduced pressure and purification by preparative HPLC gave the title compound (152 mg, 76%) as a pale yellow solid.
¹H NMR (400 MHz, CDCl3) δ 8.60 (s, 1H), 8.59 (s, 1H), 7.36 (d, 4H), 7.19 (d, 2H), 7.09 (d, 2H), 6.68 (s, 2H), 2.93-2.89 (m, 4H), 1.82-1.76 (m, 4H), 1.50-1.45 (m, 2H).
MS *mlz* 509 (M+H)⁺.

### Example 38

### 5,6-bis(4-chlorophenyl)-N-piperidin-1-yl-3-(1H-tetrazol-1-ylmethyl)pyrazine-2-carboxamide

Piperidine-1-amine hydrochloride (0.54 g, 3.98 mmol) was dissolved in dichloromethane (4.0 ml) under Ar and trimethylaluminium (4.0 ml, 2.0 M in toluene, 8 mmol) was carefully added during 5 min. The solution was stirred at ambient temperature for 1.5 h. 0.52 ml (0.26 mmol) of this stock solution was added to ethyl 5,6-bis(4-chlorophenyl)-3-(1*H*-tetrazol-1-ylmethyl)pyrazine-2-carboxylate, from Ex. 37, Step A (35 mg, 0.08 mmol) and the reaction solution was stirred at 45°C overnight. The reaction solution was cooled to 0°C and quenched with HCl (aq, 2 M, 0.5 mL). The mixture was diluted with dichloromethane and neutralized with addition of KOH (aq, 2 M). The organic phase was separated and the aqueous phase was extracted further with dichloromethane. The collected organic phases were washed with H₂O before drying with Na₂SO₄. The solvent was removed under reduced pressure and purification by preparative HPLC gave the title compound (12 mg, 27%) as a white solid.
¹H NMR (400 MHz, CDCl3) δ 8.99 (s, 1H), 8.58 (s, 1H), 7.37 (d, 4H), 7.25 (s, 4H), 6.41 (s, 2H), 2.92-2.86 (m, 4H), 1.83-1.77 (m, 4H), 1.52-1.46 (m, 2H).
MS *mlz* 509 (M+H)⁺.

### Example 39

### 5,6-bis(4-chlorophenyl)-N-(4,4-difluorocyclohexyl)-3-(2H-tetrazol-2-ylmethyl)pyrazine-2-carboxamide

(4,4-difluorocyclohexyl)amine (0.54 g, 4 mmol) was dissolved in dichloromethane (4.0 ml) under Ar and trimethylaluminium (2.0 ml, 2.0 M in toluene, 6 mmol) was carefully added. The solution was stirred at ambient temperature for 1.5 h. 1.32 ml (0.65 mmol) of this stock solution was added to ethyl 5,6-bis(4-chlorophenyl)-3-(2*H*-tetrazol-2-ylmethyl)pyrazine-2-carboxylate (100 mg, 0.22 mmol) and the reaction mixture was stirred at 45°C overnight. The reaction mixture was cooled to 0°C and quenched with 2M HCl . The mixture was diluted with dichloromethane and neutralised by addition of 2M KOH. The organic phase was separated and the aqueous phase was extracted further with dichloromethane. The combined organic phases were washed with H₂O before drying with Na₂SO₄. The solvent was removed under reduced pressure and purification by preparative HPLC gave the title compound (106 mg, 89%) as a solid.
¹H NMR (400 MHz, CDCl3) δ 8.61 (s, 1H), 7.86 (d, 1H), 7.37 (s, 4H), 7.22 (d, 2H), 7.14 (d, 2H), 6.70 (s, 2H), 4.19-4.06 (m, 1H), 2.24-1.66 (m, 8H).
MS *m*/*z* 544 (M+H)⁺.

### Example 40

### 5,6-bis(4-chlorophenyl)-N-(4,4-difluoropiperidin-1-yl)-3-(2H-tetrazol-2-ylmethyl)pyrazine-2-carboxamide

### Step A 5,6-bis(4-chlorophenyl)-3-(2H-tetrazol-2-ylmethyl)pyrazine-2-carboxylic acid

To a solution of ethyl 5,6-bis(4-chlorophenyl)-3-(2*H*-tetrazol-2-ylmethyl)pyrazine-2-carboxylate (200 mg, 0.44 mmol) in acetonitrile were added a solution of lithium hydroxide (42 mg, 1.76 mmol) in water (3.0 ml) and tetrahydrofuran (3 ml). The reaction solution was stirred in room temperature overnight. The solvent was removed under reduced pressure andwater was added to the residue. The aqueous phase was acidified by adding 1M HCl and extracted with dichloromethane and the collected organic phases were evaporated to give the title compound (187 mg, 100%) as a white solid.
MS *m*/*z* 427 (M+H)⁺.

### Step B 5,6-bis(4-chlorophenyl)-N-(4,4-difluoropiperidin-1-yl)-3-(2H-tetrazol-2-ylmethyl)pyrazine-2-carboxamide

To a solution of 5,6-bis(4-chlorophenyl)-3-(2*H*-tetrazol-2-ylmethyl)pyrazine-2-carboxylic acid (79 mg, 0.19 mmol) and 4,4-difluoropiperidin-1-amine (38 mg, 0.28 mmol) in pyridine (2.0 ml) was benzotriazol-1-yl-oxytri-pyrrolidinophosphonium hexafluorophosphate (192 mg, 0.37 mmol) carefully added. The reaction mixture was stirred in room temperature overnight. The mixture was diluted with dichloromethane and the organic phase was washed with 2M HCl (aq) and NaHCO₃ (aq). The solvent was removed under reduced pressure and purification by preparatory HPLC gave the title compound (27 mg, 27%) as a solid.
¹H NMR (400 MHz, CDCl3) δ 8.72 (s, 1H), 8.59 (s, 1H), 7.36 (s, 4H), 7.20 (d, 2H), 7.11 (d, 2H), 6.67 (s, 2H), 3.14-3.10 (m, 4H), 2.28-2.18 (m, 4H).
MS *m*/*z* 545 (M+H)⁺.

### Reference Example 41

### 5,6-bis(4-chlorophenyl)-3-[(2-methoxyethoxy)methyl]-N-piperidin-1-ylpyrazine-2-carboxamide

### Step A 5,6-bis(4-chlorophenyl)-3-[(2-methoxyethoxy)methyl]pyrazine-2-carboxylic acid

Ethyl 5,6-bis(4-chlorophenyl)-3-(hydroxymethyl)pyrazine-2-carboxylate (0.119 g, 0.294 mmol) was dissolved in dichloromethane (1.4 mL). 1-Bromo-2-methoxyethane (1.075 g, 7.35 mmol) and tetrabutylammonium hydrogensulphate (10 mg, 0.029 mmol) and then NaOH (aq., 50 % w/w, 1.4 mL) were added. The mixture quickly adopted an orangy colour, which then gradually faded with time. Vigorous stirring overnight. The following morning the reaction mixture was cooled to 0 °C before HCl (aq., 4 M, 30 mL) was slowly added (dropwise). The resulting mixture was transferred to a separation funnel with the aid of dichloromethane (30 mL). The organic phase was separated and the aqueous phase was extracted further with dichloromethane (4 x 15 mL). The collected organic phases were dried with MgSO₄. Upon evaporation of the solvents the obtained residue was purified by column chromatography (silica gel, EtOAc-heptane, 0-70 %; upon reaching 70 % EtOAc formic acid was added to the eluent, 1 % v/v) to yield 5,6-bis(4-chlorophenyl)-3-[(2-methoxyethoxy)methyl]pyrazine-2-carboxylic acid (64 mg, 0.15 mmol, 48 %) of as a yellow, viscous oil. This material was used as such in the next step.
¹H NMR (500 MHz, CDCl₃) δ 7.51 (d, 2H, J=8.5 Hz), 7.44-7.28 (m, 6H), 5.27 (s, 2H), 3.92-3.88 (m, 2H), 3.67-3.63 (m, 2H), 3.40 (s, 3H).

### Step B Methyl 5,6-bis(4-chlorophenyl)-3-[(2-methoxyethoxy)methyl]pyrazine-2-carboxylate

To a solution of 5,6-bis(4-chlorophenyl)-3-[(2-methoxyethoxy)methyl]pyrazine-2-carboxylic acid (61 mg, 0.14 mmol) in dichloromethane (1.6 mL) was added MeOH (0.4 mL). Trimethylsilyl diazomethane (0.088 mL, 2.0 M in hexanes, 0.18 mmol) was added dropwise at such a rate as to keep the evolution of nitrogen under control. Stirring at ambient temperature overnight. Next morning silica gel (ca 0.5 g) was added and the solvents were evaporated. The obtained solid residue was put on top of a pre-packed column (silica gel, heptane) and the product was eluted with an EtOAc-heptane gradient (0-70 %). The methyl 5,6-bis(4-chlorophenyl)-3- [(2-methoxyethoxy)methyl]pyrazine-2-carboxylate (52 mg, 0.12 mmol, 83 %) was obtained as a colourless, viscous oil.
¹H NMR (500 MHz, CDCl₃) δ 7.48 (d, 2H, J=8.5 Hz), 7.44 (d, 2H, J=8.5 Hz), 7.34-7.31 (m, 4H), 5.08 (s, 2H), 4.04 (s, 3H), 3.81-3.78 (m, 2H), 3.63-3.60 (m, 2H), 3.41 (s, 3H).

### Step C 5,6-bis(4-chlorophenyl)-3-[(2-methoxyethoxy)methyl]-N-piperidin-1-ylpyrazine-2-carboxamide

The hydrochloride of piperidin-1 amine (0.683 g, 5.00 mmol) was suspended in dry toluene (5.0 mL) under Ar. Upon cooling to 0 °C trimethylaluminium (2.5 mL, 2 M in toluene, 5.0 mmol) was added dropwise. After the addition of Me₃Al was complete the ice bath was removed and the mixture was allowed to stir for 3 h at ambient temperature; consequently, a ca 0.67 M solution of aluminium amide was obtained. A part of this solution (3.5 mL, 2.3 mmol, corresponding to ca 6.8 eq.) was added to methyl 5,6-bis(4-chlorophenyl)-3- [(2-methoxyethoxy)methyl]pyrazine-2-carboxylate (0.154 g, 0.344 mmol), which had been placed in a dry flask under Ar. Heating at 50 °C overnight. Next morning the reaction mixture was cooled to 0 °C and toluene (4 mL) was added before HCl (aq., 2 M, 4 mL) was slowly added (dropwise). After stirring for a couple of hours the obtained mixture was transferred to a separation funnel with the aid of dichloromethane (60 mL) and water (60 mL). The organic phase was separated and the aqueous phase was extracted further with dichloromethane (3 x 20 mL). The collected organic phases were washed with water (25 mL) and brine (60 mL) before drying with MgSO₄. Upon evaporation of the solvents the the obtained residue was purified by column chromatography (silica gel, EtOAc-heptane, 0-70 %) to yield 5,6-bis(4-chlorophenyl)-3-[(2-methoxyethoxy)methyl]-*N*-piperidin-1-ylpyrazine-2-carboxamide (0.150 g, 0.291 mmol, 84 %) as a viscous, yellowish oil.
¹H NMR (500 MHz, CDCl₃) δ 8.45 (br s, 1H, N-*H*), 7.49 (d, 2H, J=8.5 Hz), 7.41 (d, 2H, J=8.5 Hz), 7.36 (d, 2H, J=8.5 Hz), 7.31 (d, 2H, J=8.5 Hz), 5.30 (s, 2H), 3.90-3.87 (m, 2H), 3.67-3.64 (m, 2H), 3.41 (s, 3H), 3.00-2.80 (m, 4H), 1.88-1.74 (m, 4H), 1.56-1.42 (m, 2H).
HRMS Calcd for [C₂₆H₂₈Cl₂N₄O₃+H]⁺: 515.1617. Found: 515.1570.

### Reference Example 42

### 5,6-bis(4-chlorophenyl)-3-[(5-cyclopropyl-2H-tetrazol-2-yl)methyl]-N-piperidin-1-ylpyrazine-2-carboxamide

### Step A Ethyl 5,6-bis(4-chlorophenyl)-3-[(5-cyclopropyl-2H-tetrazol-2-yl)methyl]pyrazine-2-carboxylate and ethyl 5,6-bis(4-chlorophenyl)-3-[(5-cyclopropyl-1H-tetrazol-1-yl)methyl]pyrazine-2-carboxylate

To a solution of ethyl 5,6-bis(4-chlorophenyl)-3-(hydroxymethyl)pyrazine-2-carboxylate (0.23 g, 0.57 mmol) in tetrahydrofuran (5 ml) were added 5-cyclopropyl-2H-tetrazole (94 mg, 0.86 mmol) and triphenylphosphine (0.18 g, 0.68 mmol). Upon cooling to 0°C, diethyl azodicarboxylate (104µl, 0.60 mmol) was added drop wise. The reaction solution was stirred at 0°C for 2h. The solvent was removed under reduced pressure and separation by preparatory HPLC gave ethyl 5,6-bis(4-chlorophenyl)-3-[(5-cyclopropyl-2H-tetrazol-2-yl)methyl]pyrazine-2-carboxylate (165 mg, 58%), a solid.
¹H NMR (400 MHz, CDCl3) δ 7.47 (d, 2H), 7.33 (d, 2H), 7.25 (d, 4H), 6.38 (s, 2H), 4.52 (q, 2H), 2.26-2.19 (m, 1H), 1.47 (t, 3H), 1.12-1.07 (m, 4H).
MS *m*/*z* 495 (M+H)⁺.
and ethyl 5,6-bis(4-chlorophenyl)-3-[(5-cyclopropyl-1*H*-tetrazol-1-yl)methyl]pyrazine-2-carboxylate (72 mg, 26%) as a solid.
¹H NMR (400 MHz, CDC13) δ 7.47 (d, 2H), 7.34 (d, 2H), 7.23 (d, 4H), 6.25 (s, 2H), 4.56 (q, 2H), 1.92-1.85 (m, 1H), 1.50 (t, 3H), 1.23-1.19 (m, 2H), 1.10-1.05 (m, 2H).
MS *m*/*z* 495 (M+H)⁺.

### Step B 5,6-bis(4-chlorophenyl)-3-[(5-cyclopropyl-2H-tetrazol-2-yl)methyl]-N-piperidin-1-ylpyrazine-2-carboxamide

Piperidine-1-amine hydrochloride (0.82 g, 5.99 mmol) was dissolved in dichloromethane (6.0 ml) under Ar and trimethylaluminium (3.0 ml, 2.0 M in toluene, 6 mmol) was carefully added. The solution was stirred at ambient temperature for 1.5 h. 2.0 ml (1.0 mmol) of this stock solution was added to ethyl 5,6-bis(4-chlorophenyl)-3-[(5-cyclopropyl-2H-tetrazol-2-yl)methyl]pyrazine-2-carboxylate (165 mg, 0.33 mmol) and the reaction mixture was stirred at 45°C overnight. It was cooled to 0°C and quenched with 2M HCl . The mixture was diluted with dichloromethane and neutralized with addition of 2M KOH. The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (163 mg, 89%) as a white solid.
¹H NMR (400 MHz, CDCl3) δ 8.57 (s, 1H), 7.37 (d, 4H), 7.20 (d, 2H), 7.13 (d, 2H), 6.55 (s, 2H), 2.93-2.89 (m, 4H), 2.27-2.20 (m, 1H), 1.82-1.76 (m, 4H), 1.52-1.45 (m, 2H), 1.11-1.08 (m, 4H).
MS *m*/*z* 549 (M+H)⁺.

### Reference Example 43

### 5,6-bis(4-chlorophenyl)-3-[(5-cyclopyl-1H-tetrazol-1-yl)methyl]-N-piperidin-1-ylpyrazine-2-carboxamide

Piperidine-1-amine hydrochloride (0.82 g, 5.99 mmol) was dissolved in dichloromethane (6.0 ml) under Ar and trimethylaluminium (3.0 ml, 2.0 M in toluene, 6 mmol) was carefully added. The solution was stirred at ambient temperature for 1.5 h. 0.9 ml (0.45 mmol) of this stock solution was added to ethyl 5,6-bis(4-chlorophenyl)-3-[(5-cyclopropyl-1*H*-tetrazol-1-yl)methyl]pyrazine-2-carboxylate, from Ex. 42, Step A (72 mg, 0.15 mmol) and the reaction mixture was stirred at 45°C overnight. It was cooled to 0°C and quenched with 2M HCl . The mixture was diluted with dichloromethane and neutralized by addition of 2M KOH. The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (66 mg, 83%) as a pale yellow solid.
¹H NMR (400 MHz, CDCl3) δ 8.58 (s, 1H), 7.38 (d, 4H), 7.23 (d, 2H), 7.15 (d, 2H), 6.41 (s, 2H), 2.93-2.90 (m, 4H), 1.92-1.78 (m, 5H), 1.52-1.46 (m, 2H), 1.20-1.15 (m, 2H), 1.06-1.01 (m, 2H).
MS *m*/*z* 549 (M+H)⁺.

### Example 44

### 5,6-bis(4-chlorophenyl)-3-[(5-methyl-2H-tetrazol-2-yl)methyl]-N-piperidin-1-ylpyrazine-2-carboxamide

### Step A Ethyl 5,6-bis(4-chlorophenyl)-3-[(5-methyl-2H-tetrazol-2-yl)methyl]pyrazine-2-carboxylate and ethyl 5,6-bis(4-chlorophenyl)-3-[(5-methyl-1H-tetrazol-1-yl)methyl]pyrazine-2-carboxylate

To a solution of ethyl 5,6-bis(4-chlorophenyl)-3-(hydroxymethyl)pyrazine-2-carboxylate (0.23 g, 0.57 mmol) in tetrahydrofuran (5 ml) were added 5-methyl-1*H*-tetrazole (71 mg, 0.85 mmol) and triphenylphosphine (178 mg, 0.68 mmol). Upon cooling to 0°C, diethyl azodicarboxylate (104µl, 0.60 mmol) was added dropwise. The reaction solution was stirred at 0°C for 2h. The solvent was removed under reduced pressure and separation by preparatory HPLC gave ethyl 5,6-bis(4-chlorophenyl)-3-[(5-methyl-2H-tetrazol-2-yl)methyl]pyrazine-2-carboxylate (117 mg, 44%), as a solid.
¹H NMR (400 MHz, CDcl3) δ 7.47 (d, 2H), 7.33 (d, 2H), 7.25 (s, 4H), 6.41 (s, 2H), 4.52 (q, 2H), 2.57 (s, 3H), 1.47 (t, 3H).
MS *m*/*z* 469 (M+H)⁺.
and ethyl 5,6-bis(4-chlorophenyl)-3-[(5-methyl-1*H*-tetrazol-1-yl)methyl]pyrazine-2-carboxylate (79 mg, 30%) as a solid.
¹H NMR (400 MHz, CDCl3) δ 7.45 (d, 2H), 7.33 (d, 2H), 7.25 (d, 2H), 7.18 (d, 2H), 6.15 (s, 2H), 4.55 (q, 2H), 2.59 (s, 3H), 1.49 (t, 3H).
MS *m*/*z* 469 (M+H)⁺.

### Step B 5,6-bis(4-chlorophenyl)-3-[(5-methyl-2H-tetrazol-2-yl)methyl]-N-piperidin-1-ylpyrazine-2-carboxamide

Piperidine-1-amine hydrochloride (0.82 g, 5.99 mmol) was dissolved in dichloromethane (6.0 ml) under Ar and trimethylaluminium (3.0 ml, 2.0 M in toluene, 6 mmol) was carefully added. The solution was stirred at ambient temperature for 1.5 h. 1.5 ml (0.75 mmol) of this stock solution was added to ethyl 5,6-bis(4-chlorophenyl)-3-[(5-methyl-2*H-*tetrazol-2-yl)methyl]pyrazine-2-carboxylate (117 mg; 0.25 mmol) and the reaction mixture was stirred at 45°C overnight. It was cooled to 0°C and quenched with 2M HCl . The mixture was diluted with dichloromethane and neutralized by addition of 2M KOH. The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (114 mg, 87%) as a white solid.
¹H NMR (400 MHz, CDC13) δ 8.57 (s, 1H), 7.36 (d, 4H), 7.20 (d, 2H), 7.14 (d, 2H), 6.59 (s, 2H), 2.92-2.89 (m, 4H), 2.57 (s, 3H), 1.82-1.76 (m, 4H), 1.51-1.46 (m, 2H).
MS *m*/*z* 523 (M+H)⁺.

### Example 45

### 5,6-bis(4-chlorophenyl)-3-[(5-methyl-1H-tetrazol-1-yl)methyl]-N-piperidin-1-ylpyrazine-2-carboxamide

Piperidine-1-amine hydrochloride (0.82 g, 5.99 mmol) was dissolved in dichloromethane (6.0 ml) under Ar and trimethylaluminium (3.0 ml, 2.0 M in toluene, 6 mmol) was carefully added. The solution was stirred at ambient temperature for 1.5. 1.0 ml (0.50 mmol) of this stock solution was added to ethyl 5,6-bis(4-chlorophenyl)-3-[(5-methyl-1*H-*tetrazol-1-yl)methyl]pyrazine-2-carboxylate, from Ex. 44, Step A (78 mg, 0.17 mmol) and the reaction mixture was stirred at 45°C overnight. It was cooled to 0°C and quenched with 2M HCl. The mixture was diluted with dichloromethane and neutralized by addition of 2M KOH. The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (75 mg, 86%) as a pale yellow solid.
¹H NMR (400 MHz, CDCl3) δ 8.58 (s, 1H), 7.37 (d, 4H), 7.23 (d, 2H), 7.12 (d, 2H), 6.29 (s, 2H), 2.93-2.89 (m, 4H), 2.56 (s, 3H), 1.83-1.77 (m, 4H), 1.52-1.45 (m, 2H).
MS *m*/*z* 523 (M+H)⁺.

### Example 46

### tert-butyl 6-(4-chlorophenyl)-5-(4-methylphenyl)-3-[(piperidin-1-ylaminol-carbonyl]pyrazine-2-carboxylate

### Step A 5-(4-chlorophenyl)-6-(4-methylphenyl)pyrazine-2,3-dicarbonitrile

1-(4-chlorophenyl)-2-(4-methylphenyl)ethane-1,2-dione (4 g, 15.5 mmol), diaminomaleonitrile (1.84 g, 17.0 mmol) and acetic acid (1.5 ml) in ethanol (30 ml) and water (20 ml) were heated at 75 °C overnight. The reaction mixture was cooled, and water was added. The precipitate was filtered and washed with ethanol. The crude product was dissolved in methylene chloride and treated with activated charcoal, then filtered through celite. The solid was recrystallized from methylene chloride/heptane to give the subtitle compound (4.5 g, 88%) as pale yellow crystals.
¹H NMR (400 MHz, CDC13) δ 7.51 (d, 2H), 7.43 (d, 2H), 7.33 (d, 2H), 7.19 (d, 2H), 2.40 (s, 3H).

### Step B 5-(4-chlorophenyl)-6-(4-methylphenyl)pyrazine-2,3-dicarboxylic acid 5-(4-chlorophenyl)-6-(4-methylphenyl)pyrazine-2,3-dicarbonitrile (4.50 g, 12.2 mmol) and

KOH (6.84 g, 0.12 mol) in water (25 ml) was added hydrogen peroxide (35%, 5.0 ml) followed by a few drops of nonanol to reduce foaming. The reaction mixture was heated at reflux for 2h, cooled and washed once with ether and acidified to pH 4 with 2M HCl. The precipitate was filtered, washed with water and dried under reduced pressure to give the crude product. The crude product was purifed by esterification by refluxing with hydrogen chloride/methanol (36 ml) followed by preparatory HPLC, giving 2.73 g of the methyl ester. The resulting methyl ester treated with lithium hydroxide (6.60 g, 27.5 mmol) in acetonitrile (30 ml) and water (20 ml) at ambient temperature for 2 h. The acetonitrile was removed under reduced pressure and the aqueous solution was washed with diethyl ether. Acidification with hydrochloric acid (2M) gave the subtitle compound ( 2.52 g, 56 %) as a solid which was isolated by filtration.
MS *m*/*z* 369 (M+H)⁺.

### Step C 2-(4-chlorophenyl)-3-(4-methylphenyl)furo[3,4-b]pyrazine-5,7-dione

5-(4-chlorophenyl)-6-(4-methylphenyl)pyrazine-2,3-dicarboxylic acid (1.50 g, 4.07 mmol) and acetyl chloride (5.0 ml) were heated at reflux overnight. The acetyl chloride was removed under reduced pressure to give the subtitle compound (1.73 g, 100%) as a solid.
¹H NMR (400 MHz, CDCl3) δ 7.54 (d, 2H), 7.46 (d, 2H), 7.43 (d, 2H), 7.26 (d, 2H), 2.40 (s, 3H).

### Step D 3-(tert-butoxycarbonyl)-5-(4-chlorophenyl)-6-(4-methylphenyl)pyrazine-2-carboxylic acid and 3-(tert-butoxycarbonyl)-6-(4-chlorophenyl)-5-(4-methylphenyl)-pyrazine-2-carboxylic acid

2-(4-chlorophenyl)-3-(4-methylphenyl)furo[3,4-*b*]pyrazine-5,7-dione (150 mg, 0.43 mmol) was mixed with *tert*-butanol (51 mg, 0.68 mmol) and DMAP (522 mg, 4.28 mmol) in dichloromethane (2.0 ml). After 2h the solvent was removed under reduced pressure and the residue was dissolved in toluene. The organic phase was separated and washed with H₂O before drying with MgSO₄. The solvent was removed under reduced pressure and separation by preparatory HPLC gave 3-(*tert*-butoxycarbonyl)-5-(4-chlorophenyl)-6-(4-methylphenyl)pyrazine-2-carboxylic acid (50 mg, 28%), a white solid.
¹H NMR (400 MHz, CDCl3) δ 8.49 (s, OH), 7.43 (d, 2H), 7.35 (d, 2H), 7.22 (d, 2H), 7.06 (d, 2H), 2.31 (s, 3H), 1.60 (s, 9H).
MS *m*/*z* 425 (M+H)⁺
and 3-(*tert*-butoxycarbonyl)-6-(4-chlorophenyl)-5-(4-methylphenyl)pyrazine-2-carboxylic acid (41 mg, 23%) as a white solid.
¹H NMR (400 MHz,CDCl3) δ 8.45 (s, OH), 7.47 (d, 2H), 7.38 (d, 2H), 7.24 (d, 2H), 7.11 (d, 2H), 2.34 (s, 3H), 1.63 (s, 9H).
MS *m*/*z* 425 (M+H)⁺

### Step E tert-butyl 6-(4-chlorophenyl)-5-(4-methylphenyl)-3-[(piperidin-1-ylamino)-carbonyl]pyrazine-2-carboxylate

To a solution of 3-(*tert*-butoxycarbonyl)-5-(4-chlorophenyl)-6-(4-methylphenyl)pyrazine-2-carboxylic acid (50 mg, 0.12 mmol) and aminopiperidine (19 mg, 0.19 mmol) in dichloromethane (2.0 ml) was triethylamine (262 µl, 1.9 mmol) added. The resulting solution was cooled to 0°C and benzotriazol-1-yl-oxytri-pyrrolidinophosphonium hexafluorophosphate (98 mg, 0.19 mmol), dissolved in dichloromethane, was carefully added. The reaction was continued at 0°C for 30 minutes, then in room temperature overnight. The mixture was diluted with dichloromethane and the organic phase washed with H₂O before drying with MgSO₄. The solvent was removed under reduced pressure and preparatory HPLC gave the title compound (26 mg, 44%) as a white solid.
¹H NMR (400 MHz, CDCl3) δ 8.34 (s, NH), 7.45 (d, 2H), 7.33 (d, 2H), 7.28 (d, 2H), 7.18 (d, 2H), 2.90-2.86 (m, 4H), 2.40 (s, 3H), 1.80-1.74 (m, 4H), 1.68 (s, 9H), 1.49-1.42 (m, 2H).
MS *m*/*z* 425 (M+H)⁺

### Example 47

### tert-butyl 5-(4-chlorophenyl)-6-(4-methylphenyl)-3-[(piperidin-1-ylamino)carbonyl]-pyrazine-2-carboxylate

To a solution of 3-(*tert*-butoxycarbonyl)-6-(4-chlorophenyl)-5-(4-methylphenyl)pyrazine-2-carboxylic acid from Ex. 46, Step D (39 mg, 0.09 mmol) and aminopiperidine (15 mg, 0.15 mmol) in dichloromethane (2.0 ml) was triethylamine (205 µl, 1.5 mmol) added. The resulting solution was cooled to 0°C and benzotriazol-1-yl-oxytri-pyrrolidinophosphonium hexafluorophosphate (76 mg, 0.15 mmol), dissolved in dichloromethane, was carefully added. The reaction was continued at 0°C for 30 minutes, then in room temperature overnight. The mixture was diluted with dichloromethane and the organic phase washed with H₂O before drying with MgSO₄. The solvent was removed under reduced pressure and preparatory HPLC gave the title compound (28 mg, 60%) as a white solid.
¹H NMR (400 MHz, CDCl3) δ 8.29 (s, NH), 7.41-7.32 (m, 6H), 7.13 (d, 2H), 2.92-2.87 (m, 4H), 2.36 (s, 3H), 1.80-1.75 (m, 4H), 1.68 (s, 9H), 1.51-1.43 (m, 2H).
MS *m*/*z* 425 (M+H)⁺.

### Example 48 6-(4-chlorophenyl)-5-(4-methylphenyl)-N-piperidin-1-yl-3-(2H-tetrazol-2-ylmethyl)-pyrazine-2-carboxamide

### Step A 5-(4-chlorophenyl)-3-(ethoxycarbonyl)-6-(4-methylphenyl)pyrazine-2-carboxylic acid and 6-(4-chlorophenyl)-3-(ethoxycarbonyl)-5-(4-methylphenyl)pyrazine-2-carboxylic acid

2-(4-chlorophenyl)-3-(4-methylphenyl)furo[3,4-*b*]pyrazine-5,7-dione (1.40 g, 4.0 mmol) was suspended in EtOH (45 ml). The suspended material gradually went into solution. After stirring overnight the solvent was removed under reduced pressure to give a mixture of the titled compounds (1.42, 90%).
MS *m*/*z* 397 (M+H)⁺

### Step B Ethyl 6-(4-chlorophenyl)-3-(hydroxymethyl)-5-(4-methylphenyl)pyrazine-2-carboxylate and ethyl 5-(4-chlorophenyl)-3-(hydroxymethyl)-6-(4-methylphenyl)pyrazine-2-carboxylate

The mixture of 5-(4-chlorophenyl)-3-(ethoxycarbonyl)-6-(4-methylphenyl)pyrazine-2-carboxylic acid and 6-(4-chlorophenyl)-3-(ethoxycarbonyl)-5-(4-methylphenyl)pyrazine-2-carboxylic acid (1.42 g, 3.59 mmol) was dissolved in tetrahydrofuran (15 mL) under Ar. Upon cooling to -20°C triethylamine (0.70 ml, 5.02 mmol) and then *iso-*butylchloroformiate (0.56 ml, 4.30 mmol) were added dropwise. After the addition the temperature was adjusted to 0°C. After stirring at 0°C for 1 h, the reaction mixture was re cooled to -20°C and 4.33 ml of a pre-prepared solution of sodiumborohydride (0.940 g, 24.8 mmol) in pre-chilled H₂O (10 mL) was added dropwise to the reaction mixture. After stirring at -20°C for 1.5 h the reaction was quenched by the dropwise addition of HCl (aq., 2 M). The reaction mixture was allowed to stir for 1 h (during that time the temperature increased to -10°C) before it was diluted with dichloromethane and washed with H₂O. The organic phase was separated and the aqueous phase was extracted further with dichloromethane. The collected organic phases were washed with brine before drying with MgSO₄. The solvent was removed under reduced pressure and with preparatory HPLC the two compounds were separated to give the Ethyl 6-(4-chlorophenyl)-3-(hydroxymethyl)-5-(4-methylphenyl)pyrazine-2-carboxylate₋(372 mg, 27%) , a white solid.
¹H NMR (400 MHz, CDC13) δ 7.48 (d, 2H), 7.41 (d, 2H), 7.29 (d, 2H), 7.15 (d, 2H), 5.15 (d, 2H), 4.50 (q, 2H), 4.39 (t, OH), 2.37 (s, 3H), 1.46 (t, 3H).
MS *m*/*z* 383 (M+H)⁺
and ethyl 5-(4-chlorophenyl)-3-(hydroxymethyl)-6-(4-methylphenyl)pyrazine-2-carboxylate (362 mg, 26%) as a white solid.
¹H NMR (400 MHz, CDC13) δ 7.49 (d, 2H), 7.40 (d, 2H), 7.31 (d, 2H), 7.15 (d, 2H), 5.16 (d, 2H), 4.51 (q, 2H), 4.29 (t, OH), 2.37 (s, 3H), 1.46 (t, 3H).
MS *m*/*z* 383 (M+H)⁺

### Step C Ethyl 6-(4-chlorophenyl)-5-(4-methylphenyl)-3-(2H-tetrazol-2-ylmethyl)pyrazine-2-carboxylate and ethyl 6-(4-chlorophenyl)-5-(4-methylphenyl)-3-(1H-tetrazol-1-ylmethyl)pyrazine-2-carboxylate

To a solution of ethyl 6-(4-chlorophenyl)-3-(hydroxymethyl)-5-(4-methylphenyl)pyrazine-2-carboxylate (161 mg, 0.42 mmol) in tetrahydrofuran (5 ml) were added 1H-tetrazol (44 mg, 0.63 mmol) and triphenylphosphine (132 mg, 0.51 mmol). Upon cooling to 0°C, diethyl azodicarboxylate (0.8 ml, 0.46 µmol) was added dropwise. The reaction mixture was stirred at 0°C for 1.5 h. The solvent was removed under reduced pressure and with preparatory HPLC the two compounds were separated to give ethyl 6-(4-chlorophenyl)-5-(4-methylphenyl)-3-(2*H*-tetrazol-2-ylmethyl)pyrazine-2-carboxylate (74 mg, 41 %), a white solid.
¹H NMR (400 MHz) δ 8.59 (s, 1H), 7.47 (d, 2H), 7.30 (d, 2H), 7.16 (d, 2H), 7.02 (d, 2H), 6.51 (s, 2H), 4.52 (q, 2H), 2.32 (s, 3H), 1.47 (t, 3H).
MS *m*/*z* 435 (M+H)⁺.
and ethyl 6-(4-chlorophenyl)-5-(4-methylphenyl)-3-(1*H*-tetrazol-1-ylmethyl)pyrazine-2-carboxylate (40 mg, 22 %) as a solid.
¹H NMR (400 MHz, CDCl3) δ 8.91 (s, 1H), 7.47 (d, 2H), 7.30 (d, 2H), 7.21 (d, 2H), 7.09 (d, 2H), 6.27 (s, 2H), 4.54 (q, 2H), 2.34 (s, 3H), 1.48 (t, 3H).
MS *m*/*z* 435 (M+H)⁺.

### Step D 6-(4-chlorophenyl)-5-(4-methylphenyl)-N-piperidin-1-yl-3-(2H-tetrazo1-2-ylmethyl)pyrazine-2-carboxamide

Piperidine-1-amine hydrochloride (0.81 g, 6.0 mmol) was dissolved in dichloromethane (6.0 ml) under Ar and trimethylaluminium (3.0 ml, 2.0 M in toluene, 6 mmol) was carefully added during 5 min. The solution was stirred at ambient temperature for 1.5 h 1.0 ml (0.50 mmol) of this stock solution was added to ethyl 6-(4-chlorophenyl)-5-(4-methylphenyl)-3-(2*H*-tetrazol-2-ylmethyl)pyrazine-2-carboxylate (74 mg, 0.17 mmol) and the reaction mixture was stirred at 45°C overnight. It was cooled to 0°C and quenched with HCl (aq, 2 M, 2.5 mL). The mixture was diluted with dichloromethane and neutralized with addition of KOH (aq, 2 M). The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (60 mg, 72%) as a white solid.
¹H NMR (400 MHz, CDC13) δ 8.61 (s, NH), 8.58 (s, 1H), 7.39 (d, 2H), 7.32 (d, 2H), 7.05 (d, 2H), 7.00 (d, 2H), 6.68 (s, 2H), 2.94-2.87 (m, 4H), 2.29 (s, 3H), 1.82-1.75 (m, 4H), 1.50-1.43 (m, 2H).
MS *mlz* 489 (M+H)⁺.

### Example 49

### 5-(4-chlorophenyl)-6-(4-methylphenyl)-N-piperidin-1-yl-3-(2H-tetrazol-2-ylmethyl)-pyrazine-2-carboxamide

### Step A Ethyl 5-(4-chlorophenyl)-6-(4-methylphenyl)-3-(2H-tetrazol-2-ylmethyl)pyrazine-2-carboxylate and ethyl 5-(4-chlorophenyl)-6-(4-methylphenyl)-3-(1H-tetrazol-1-ylmethyl)pyrazine-2-carboxylate

To a solution of ethyl 5-(4-chlorophenyl)-3-(hydroxymethyl)-6-(4-methylphenyl)pyrazine-2-carboxylate (156 mg, 0.41 mmol) in tetrahydrofuran (5 ml) were added 1H-tetrazol (43 mg, 0.61 mmol) and triphenylphosphine (128 mg, 0.49 mmol). Upon cooling to 0°C, diethyl azodicarboxylate (82 µl, 0.45 µmol) was added drop wise. The reaction solution was stirred at 0°C for 1.5 h. The solvent was removed under reduced pressure and with preparatory HPLC the two compounds were separated to ethyl 5-(4-chlorophenyl)-6-(4-methylphenyl)-3-(2*H*-tetrazol-2-ylmethyl)pyrazine-2-carboxylate (105 mg, 59 %), a solid.
¹H NMR (400 MHz, CDCl3) δ 8.62 (s, 1H), 7.42 (d, 2H), 7.23 (d, 2H), 7.22 (d, 2H), 7.17 (d, 2H), 6.54 (s, 2H), 4.55 (q, 2H), 2.39 (s, 3H), 1.49 (t, 3H).
MS *m*/*z* 435 (M+H)⁺.
and ethyl 5-(4-chlorophenyl)-6-(4-methylphenyl)-3-(1*H*-tetrazol-1-ylmethyl)pyrazine-2-carboxylate (63 mg, 36 %) as solid.
¹H NMR (400 MHz,CDCl3) δ 8.92 (s, 1H), 7.42 (d, 2H), 7.31 (d, 2H), 7.27 (d, 2H), 7.18 (d, 2H), 6.30 (s, 2H), 4.56 (q, 2H), 2.39 (s, 3H), 1.50 (t, 3H).
MS *m*/*z* 435 (M+H)⁺.

### Step B 5-(4-chlorophenyl)-6-(4-methylphenyl)-N-piperidin-1-yl-3-(2H-tetrazol-2-ylmethyl)pyrazine-2-carboxamide

Piperidine-1-amine hydrochloride (0.81 g, 6.0 mmol) was dissolved in dichloromethane (6.0 ml) under Ar and trimethylaluminium (3.0 ml, 2.0 M in toluene, 6 mmol) was carefully added during 5 min. The solution was stirred at ambient temperature for 1.5 h. 1.45 ml (0.73 mmol) of this stock solution was added to ethyl 5-(4-chlorophenyl)-6-(4-methylphenyl)-3-(2*H*-tetrazol-2-ylmethyl)pyrazine-2-carboxylate (105 mg, 0.24 mmol) and the reaction mixture was stirred at 45°C overnight. It was cooled to 0°C and quenched with HCl (aq, 2 M, 2.5 mL). The mixture was diluted with dichloromethane and neutralized with addition of KOH (aq, 2 M). The organic phase was separated and the aqueous phase was extracted further with dichloromethane. The collected organic phases were washed with H₂O before drying with Na₂SO₄. The solvent was removed under reduced pressure and purification by preparatory HPLC gave the title compound (74 mg, 63%) as a pale yellow solid.
¹H NMR (400 MHz, CDCl3) δ 8.68 (s, NH), 8.58 (s, 1H), 7.31 (d, 2H), 7.19-7.09 (m, 6H), 6.68 (s, 2H), 2.93-2.88 (m, 4H), 2.38 (s, 3H), 1.81-1.75 (m, 4H), 1.51-1.42 (m, 2H).
MS *m*/*z* 489 (M+H)⁺.

### Reference Example 50

### Tert-butyl 5,6-bis(4-chlorophenyl)-3-{[(2-hdyroxyethyl)(methyl)amino]carbonyl}-pyrazine-2-carboxylate

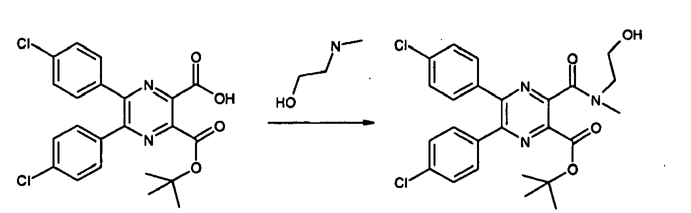

PyBOP (185 mg, 0.356 mmol), dissolved in DCM (1 ml), was added to a mixture of 3-(*tert*-butoxycarbonyl)-5,6-bis(4-chlorophenyl)pyrazine-2-carboxylic acid (100 mg, 0.225 mmol) and 2-(methylamino)ethanol (34 mg, 0.449 mmol) in DCM (4 ml) and TEA (0.5 ml), at 0°C. Stirring was continued at 0°C for 15 minutes and thereafter at room temperature for 22 hours. The reaction mixture was washed with water and dried over MgSO₄. The product was purified by flash chromatography (SiO₂, toluene:ethyl acetate 6:4) to give the subtitle compound as a slightly yellow powder (76 mg, 67%).
¹H NMR (400 MHz, CDCl₃) δ 7.48-7.28 (m, 8H), 4.02-3.96 and 3.80-3.75 (m, 2H), 3.75-3.70 and 3.58-3.52 (m, 2H), 3.40-3.32 and 3.12-3.04 (m, 1H), 3.21 and 3.05 (s, 3H), 1.62 and 1.60 (s, 9H).
¹³C NMR (100 MHz, CDCl₃) δ 168.21, 167.29, 163.57, 163.37, 151.95, 150.79, 150.42, 148.35, 148.11, 141.92, 140.07, 136.54, 136.39, 136.16, 135.65, 135.49, 135.40, 135.15, 131.39, 131.25, 129.25, 129.10, 84.50, 60.48, 59.52, 52.92, 51.23, 38.17, 32.97, 28.20, 28.14.
HRMS Calcd for [C₂₅H₂₅N₃O₄Cl₂+H]⁺: 502.130. Found: 502.131.

### Example 51

### 5,6-Bis-(4-chloro-phenyl)-3-propoxy-pyrazine-2-carboxylic acid piperidin-1-ylamide

### Step A 5,6-Bis-(4-chloro-phenyl)-3-hydroxy-pyrazine-2-carboxylic acid methyl ester

A solution of 5,6-bis-(4-chloro-phenyl)-3-hydroxy-pyrazine-2-carboxylic acid (730 mg, 2.02 mmol) and boron trifluoride ethyl etherate (10 ml) in methanol (50 ml) was refluxed for 3 hours, cooled to room temperature, added saturated sodium hydrogen carbonate (200 ml) and extracted with CH₂Cl₂ (3x100 ml). The combined organic layers were washed with brine, dried (Na₂SO₄), filtered and concentrated to afford 740 mg (98%) of the title compound.
¹H NMR (CDCl₃): δ 11.2 (1H, s), 7.5-7.3 (8H, m), 4.1 (3H, s).

### Step B 5,6-Bis-(4-chloro-phenyl)-3-propoxy-pyrazine-2-carboxylic acid methyl ester

A solution of 5,6-bis-(4-chloro-phenyl)-3-hydroxy-pyrazine-2-carboxylic acid methyl ester (301 mg, 0.80 mmol), cesium carbonate (340 mg, 1.04 mmol), 1-bromopropane (95 µl, 1.04 mmol) and a few crystals of potassium iodide in DMF was heated at 60°C for 5 hours, cooled to room temperature and evaporated to dryness. Flash chromatography (silica, hexane:EtOAc 90: 10) afforded 315 mg (94%) of the title compound.
¹H NMR (CDCl₃): δ 7.5-7.3 (8H, m), 4.5 (2H, t), 4.1 (3H, s), 1.9 (2H, q), 1.1 (3H, t).

### Step C 5,6-Bis-(4-chloro-phenyl)-3-propoxy-pyrazine-2-carboxylic acid

To a solution of 5,6-bis-(4-chloro-phenyl)-3-propoxy-pyrazine-2-carboxylic acid methyl ester (315 mg, 0.76 mmol) in methanol (5 ml) was added a solution of potassium hydroxide (251 mg, 3.8 mmol) in water (5 ml) and refluxed for 1 hour. The mixture was cooled to room temperature, acidified with 1M hydrochloric acid, extracted with CH₂Cl₂ (3x20 ml), dried (Na₂SO₄), filtered and concentrated to afford 263 mg (86%) of the title compound.

### Step D 5,6-Bis-(4-chloro-phenyl)-3-propoxy-pyrazine-2-carboxylic acid piperidin-1-ylamide

To a solution of 5,6-bis-(4-chloro-phenyl)-3-propoxy-pyrazine-2-carboxylic acid (263 mg, 0.65 mmol) in 10 ml CH₂Cl₂ was added 2 drops of DMF followed by oxalyl chloride (111 µl, 1.31 mmol). The mixture was refluxed for 2 hours, cooled to room temperature and evaporated to dryness. The residue was dissolved in 10 ml CH₂Cl₂ and cooled to 0°C.

Triethylamine (182 µl, 1.31 mmol) was added followed by 1-aminopiperidine (78 µl, 0.72 mmol) and the mixture was stirred at room temperature for 1.5 hours. Water (25 ml) was added, the mixture extracted with CH₂Cl₂ (2x30 ml), dried (Na₂SO₄), filtered and concentrated. Flash chromatography (silica, hexane:EtOAc 3:1) afforded 240 mg (76%) of the title compound as a pale yellow solid.
¹H NMR (CDCl₃): δ 8.3 (1H, broad s), 7.5-7.3 (8H, m), 4.5 (2H, t), 3.1-2.9 (4H, m), 2.0-1.7 (6H, m), 1.6-1.4 (2H, m), 1.1 (3H, t).
MS m/z 508 (M+Na)
HPLC: 99.6%.

### Example 52

### 5,6-bis(4-chlorophenyl)-N-piperidin-1-yl-3-(2H-tetrazol-5-ylmethyl)pyrazine-2-carboxamide

Dibutyltin oxide (12.7 mg, 0.051 mmol) and trimethylsilyl azide (0.134 ml, 1.016 mmol) were added to 5,6-bis(4-chlorophenyl)-3-(cyanomethyl)-*N*-piperidin-1-ylpyrazine-2-carboxamide (79.0 mg, 0.169 mmol) dissolved in toluene (0.34 ml). The reaction mixture was refluxed for 28 h, adding another 6 eq of the trimethylsilyl azide (0.134 ml, 1.016 mmol) after 20 h. The solvent was removed under reduced pressure and the residue was dissolved in methanol and re-concentrated under reduced pressure twice. The residue was dissolved in ethyl acetate and extracted several times with sat. NaHCO₃. The combined aqueous phasees was acidified (10 % HCl) and extracted twice with ethyl acetate. The solvent was removed under reduced pressure from the combined organic phase. The product was purified by preparative HPLC (kromasil C8 column, ammonium acetate (aq, 0.1 M):acetonitrile) to yield the title compound (20.0 mg, 23 %) as a white solid after freeze drying.
¹H NMR (400 MHz, DMSO-d6) δ 9.66 (1H, s), 7.43 (8H, m), 4.85 (2H, s), 2.77 (4H, t), 1.55 (4H, m), 1.34 (2H, m).
HRMS Calcd for [C₂₄H₂₂Cl₂N₈O+H]⁺: 509.137. Found: 509.138.

### Example 53

### 5,6-bis(4-chlorophenyl)-N-piperidin-1-yl-3-(1H-tetrazol-5-yl)pyrazine-2-carboxamide

### Step A 5,6-bis(4-chlorophenyl)-3-(1H-tetrazol-5-yl)pyrazine-2-carbonitrile

5,6-bis(4-chlorophenyl)pyrazine-2,3-dicarbonitrile (204 mg, 0.581 mmol), sodium azide (47 mg, 0.723 mmol), and triethylammonium chloride (154 mg, 1.12 mmol) were heated in N-methylpyrrolidinone (10 ml) at 170 degrees for 10 minutes in a microwave heating apparatus. Purification by preparative HPLC gave the title compound, 162 mg, 71 %.

¹H NMR (400 MHz, CD₃OD) δ 7.67 (d, 2H), 7.58 (d, 2H), 7.44 (d, 2H), 7.42 (d, 2H)
MS *m*/*z* calcd for [C 18 H9 C12 N7]H⁺ 394, found 394

### Step B 5,6-bis(4-chlorophenyl)-3-(1H-tetrazol-5-yl)pyrazine-2-carboxylic acid

5,6-bis(4-chlorophenyl)-3-(1*H*-tetrazol-5-yl)pyrazine-2-carbonitrile (155 mg, 0.393 mmol), potassium hydroxide (300 mg, 5.4 mmol), and 30% hydrogen peroxide (0.5 ml) were heated in 5 ml of water at 140 degrees for 10 minutes in a microvawe heating apparatus.

The reaction mixture was acidified with 0.5M hydrochloric acid and extracted with methylene chloride. Drying (magnesium sulfate) and removal of the solvent in vacuo gave the title compound, 124 mg, 76%.

¹H NMR (400 MHz, CD₃OD) δ 7.56 (d, 2H), 7.53 (d, 2H), 7.37 (d, 2H), 7.35 (d, 2H)
MS *m*/*z* calcd for [C18 H10 C12 N6 O2]H⁺413, found 413

### Step C 5,6-bis(4-chlorophenyl)-N-piperidin-1-yl-3-(1H-tetrazol-5-yl)pyrazine-2-carboxamide

5,6-bis(4-chlorophenyl)-3-(1*H*-tetrazol-5-yl)pyrazine-2-carboxylic acid (78 mg, 0.19 mmol), piperidin-1-amine hydrochloride (45 mg, 0.33 mmol), and PyBOP (123 mg, 0.24 mmol) were mixed in pyridine (1.5 ml) and stirred overnight at room temperature. Purification by preparative HPLC gave the target compound, 17 mg (18%).
¹H NMR (400 MHz, mix of CDCl₃ and CD₃OD) δ 7.48-7.33 (m, 4H), 7.30-7.20 (m, 4H), 2.73-2.62 (m, 4H), 1.62-1.53 (m, 4H), 1.38-1.29 (m, 2H)
HRMS *m*/*z* calcd for [C23H20C12N8O]H⁺ 495.1215, found 495.1219

### Example 54

### 5,6-bis(4-chlorophenyl)-3-[(5-morpholin-4-yl-2H-tetrazol-2-yl)methyl]-N-pineridin-1-ylpyrazine-2-carboxamide and

### Example 55

### 5,6-bis(4-chlorophenyl)-3-[(5-morpholin-4-yl-1H-tetrazol-1-yl)methyl]-N-piperidin-1-ylpyrazine-2-carboxamide

### Step A 5,6-bis(4-chlorophenyl)-3-(hydroxymethyl)-N-piperidin-1-ylpyrazine-2-carboxamide

Piperidine-1-amine hydrochloride (0.82 g, 6.0 mmol) was dissolved in dichloromethane (6.0 ml) under Ar and trimethylaluminium (3.0 ml, 2.0 M in toluene, 6.0 mmol) was carefully added. The solution was stirred at ambient temperature for 1.5 h and, as a result, a ca 0.5 M solution of an amide reagent was obtained. 8.1 ml (4.05 mmol) of this stock solution was added to ethyl 5,6-bis(4-chlorophenyl)-3-(hydroxymethyl)pyrazine-2-carboxylate (546 mg, 1.35 mmol) and the reaction mixture was stirred at 45°C overnight. It was cooled to 0°C and quenched with HCl (aq, 2 M, 8.0 mL). The mixture was diluted with dichloromethane and neutralized with addition of KOH (aq, 2 M). The organic phase was separated and the aqueous phase was extracted further with dichloromethane. The collected organic phases were washed with H₂O before drying with Na₂SO₄. The solvent was removed under reduced pressure and purification by preparatory HPLC gave the titled compound (436 mg, 70%) as a white solid.
¹H NMR (400 MHz, CDCl₃) δ 8.66 (s, 1H), 7.45-7.31 (m, 8H), 5.18 (d, 2H), 4.88 (t, OH), 2.92-2.89 (m, 4H), 1.82-1.76 (m, 4H), 1.51-1.46 (m, 2H).
MS *m*/*z* 457 (M+H)⁺.

### Step B 5,6-bis(4-chlorophenyl)-3-[(5-morpholin-4-yl-1H-tetrazol-1-yl)methyl]-N-piperidin-1-ylpyrazine-2-carboxamide

To a suspension of 5,6-bis(4-chlorophenyl)-3-(hydroxymethyl)-*N*-piperidin-1-ylpyrazine-2-carboxamide (99 mg, 0.22 mmol) in tetrahydrofuran (2 ml) were added 5-(4-morpholino)tetrazole (50 mg, 0.32 mmol) and triphenylphosphine (68 mg, 0.26 mmol). Upon cooling to 0°C, diethyl azodicarboxylate (40µl, 0.22 mmol) was added dropwise. The reaction mixture was stirred at 0°C for 1h. The solvent was removed under reduced pressure and separation by preparative HPLC gave Ex 54, 5,6-bis(4-chlorophenyl)-3-[(5-morpholin-4-yl-2*H*-tetrazol-2-yl)methyl]-*N*-piperidin-1-ylpyrazine-2-carboxamide (75mg, 58%) and Ex. 55, 5,6-bis(4-chlorophenyl)-3-[(5-morpholin-4-yl-1*H*-tetrazol-1-yl)methyl]-*N*-piperidin-1-ylpyrazine-2-carboxamide (4mg, 3%) as solids.
Ex. 54: ¹H NMR (400 MHz, CDCl₃) δ 8.57 (s, 1H), 7.37 (d, 4H), 7.20 (d, 4H), 6.46 (s, 2H), 3.83-3.80 (m, 4H), 3.50-3.47 (m, 4H), 2.92-2.89 (m, 4H), 1.81-1.75 (m, 4H), 1.51-1.45 (m, 2H).
MS *mlz* 594 (M+H)⁺.
Ex. 55: ¹H NMR (400 MHz, CDCl₃) δ 8.58 (s, 1H), 7.38 (d, 4H), 7.23 (d, 2H), 7.16 (d, 2H), 6.22 (s, 2H), 3.70-3.67 (m, 4H), 3.28-3.25 (m, 4H), 2.93-2.89 (m, 4H), 1.83-1.77 (m, 4H), 1.52-4.45 (m, 2H).
MS *m*/*z* 594 (M+H)⁺.

### Example 56

### 5,6-bis(4-chlorophenyl)-N-piperidin-1-yl-3-[(5-pyrrolidin-1-yl-2H-tetrazol-2-yl)methyl]pyrazine-2-carboxamide

### Example 57

### 5,6-bis(4-chlorophenyl)-N-piperidin-1-yl-3-[(5-pyrrolidin-1-yl-1H-tetrazol-1-yl)methyl]pyrazine-2-carboxamide

To a suspension of 5,6-bis(4-chlorophenyl)-3-(hydroxymethyl)-*N*-piperidin-1-ylpyrazine-2-carboxamide (148 mg, 0.32 mmol) in tetrahydrofuran (3.5 ml) were added 5-(1-pyrrolidino)tetrazole (68 mg, 0.49 mmol) and triphenylphosphine (102 mg, 0.39 mmol). Upon cooling to 0°C, diethyl azodicarboxylate (60µl, 0.34 mmol) was added dropwise. The reaction mixture was stirred at 0°C for 45min. The solvent was removed under reduced pressure and the residue was separated by preparative HPLC to give, **EX. 56,** 5,6-bis(4-chlorophenyl)-*N*-piperidin-1-yl-3-[(5-pyrrolidin-1-yl-2*H*-tetrazol-2-yl)methyl]pyrazine-2-carboxamide (122 mg, 65%) and **Ex. 57,** 5,6-bis(4-chlorophenyl)-*N*-piperidin-1-yl-3-[(5-pyrrolidin-1-yl-1*H*-tetrazol-1-yl)methyl]pyrazine-2-carboxamide (26 mg, 14%) as solids.
**Ex. 56^{:1}H** NMR (400 MHz, CDCl₃) δ 8.57 (s, 1H), 7.37 (d, 4H), 7.20 (s, 4H), 6.44 (s, 2H), 3.52-3.48 (m, 4H), 2.93-2.88 (m, 4H), 2.01-1.98 (m, 4H), 1.81-1.75 (m, 4H), 1.50-1.44 (m, 2H).
MS *m*/*z* 578 (M+H)⁺.
**Ex. 57**:¹H NMR (400 MHz, CDCl₃) δ 8.56 (s, 1H), 7.38 (d, 4H), 7.22 (d, 4H), 6.32 (s, 2H), 3.56-3.52 (m, 4H), 2.93-2.88 (m, 4H), 1.89-1.77 (m, 8H), 1.53-1.45 (m, 2H).
MS *m*/*z* 578 (M+H)⁺.

### Reference Example 58

### 5,6-bis(4-chlorophenyl)-3-{[5-(methylthio)-2H-tetrazol-2-yl]methyl}-N-piperidin-1-ylpyrazine-2-carboxamide

### Reference Example 59

### 5,6-bis(4-chlorophenyl)-3-{[5-(methylthio)-1H-tetrazol-1-yl]methyl}-Npiperidin-1-ylpyrazine-2-carboxamide

To a suspension of 5,6-bis(4-chlorophenyl)-3-(hydroxymethyl)-*N*-piperidin-1-ylpyrazine-2-carboxamide (150 mg, 0.33 mmol) in tetrahydrofuran (3.5 ml) were added 5-(methylthio)-1H-tetrazole (57 mg, 0.49 mmol) and triphenylphosphine (103 mg, 0.39 mmol). Upon cooling to 0°C, diethyl azodicarboxylate (60µl, 0.33 mmol) was added drop wise. The reaction mixture was stirred at 0°C for 30min. The solvent was removed under reduced pressure and the residue was separation by preparated HPLC to give **Ex. 58,** 5,6-bis(4-chlorophenyl)-3-{[5-(methylthio)-2*H*-tetrazol-2-yl]methyl}-*N*-piperidin-1-ylpyrazine-2-carboxamide (111 mg, 61%) and Ex. 59, 5,6-bis(4-chlorophenyl)-3-{[5-(methylthio)-1*H*-tetrazol-1-yl]methyl}-*N*-piperidin-1-ylpyrazine-2-carboxamide (37 mg, 20%) as solids.
**Ex. 58:** ¹H NMR (400 MHz, CDCl₃) δ 8.60 (s, 1H), 7.37 (d, 4H), 7.21 (d, 2H), 7.15 (d, 2H), 6.59 (s, 2H), 2.93-2.88 (m, 4H), 2.67 (s, 3H), 1.81-1.76 (m, 4H), 1.50-1.45 (m, 2H).
MS *m*/*z* 555 (M+H)⁺.
**Ex. 59**:¹H NMR (400 MHz, CDCl₃) δ 8.96 (s, 1H), 7.73 (d, 4H), 7.58 (d, 2H), 7.51 (d, 2H), 6.63 (s, 2H), 3.29-3.25 (m, 4H), 3.09 (s, 3H), 2.18-2.13 (m, 4H), 1.87-1.81 (m, 2H).
MS *m*/*z* 555 (M+H)⁺.

### Reference Example 60

### 5,6-bis(4-chlorophenyl)-N-(4,4-difluorocyclohexyl)-3-(methoxymethyl)pyrazine-2-carboxamide

### Step A 5,6-bis(4-chlorophenyl)-3-(methoxymethyl)pyrazine-2-carboxylic acid

Ethyl 5,6-bis(4-chlorophenyl)-3-(hydroxymethyl)pyrazine-2-carboxylate (1.44 g, 3.58 mmol) was dissolved in dichloromethane (18 mL), iodomethane (5.086 g, 35.8 mmol) and tetrabutylammonium hydrogensulphate (0.122 g, 0.358 mmol) and thenn NaOH (aq., 50 % w/w, 18 mL) were added. The solution quickly adopted a brightly red colour, which then gradually faded with time. Vigorous stirring overnight. The following morning the reaction mixture was cooled to 0 °C before HCl (aq., 4 M, 200 mL) was slowly added (dropwise). The resulting mixture was transferred to a separation funnel with the aid of dichloromethane (100 mL). The organic phase was separated and the aqueous phase was extracted further with dichloromethane (4 x 100 mL). The collected organic phases were dried with MgSO₄. Upon evaporation of the solvents the obtained residue was purified by column chromatography (silica gel, EtOAc-heptane, 0-40 %; upon reaching 40 % EtOAc formic acid was added to the eluent, 0.5 % v/v) to yield crude 5,6-bis(4-chlorophenyl)-3-(methoxymethyl)pyrazine-2-carboxylic acid (1.130 g) as a brownish semi-solid. The material was used without further purification in the next step.

### Step B Methyl 5,6-bis(4-chlorophenyl)-3-(methoxymethyl)pyrazine-2-carboxylate

To a mixture of 5,6-bis(4-chlorophenyl)-3-(methoxymethyl)pyrazine-2-carboxylic acid (1.13 g, 2.90 mmol) in dichloromethane (28 mL) and MeOH (7 mL) was added Trimethylsilyl diazomethane (1.8 mL, 2.0 M in hexanes, 3.6 mmol) dropwise at such a rate as to keep the evolution of nitrogen under control. Stirring overnight at ambient temperature. Next morning silica gel (ca 5 g) was added to the reaction mixture and the solvents were evaporated. The obtained solid residue was put on top of a pre-packed column (silica gel, heptane) and the products were eluted with an EtOAc-heptane gradient (0-25 %). Methyl 5,6-bis(4-chlorophenyl)-3-(methoxymethyl)pyrazine-2-carboxylate (1.058 g, 2.62 mmol, 90 %) was obtained as a colourless viscous oil.

¹H NMR (500 MHz, CDCl₃) δ 7.54-7.40 (m, 4H), 7.40-7.30 (m, 4H), 4.98 (s, 2H), 4.04 (s, 3H), 3.53 (s, 3H).

### Step C 5,6-bis(4-chlorophenyl)-N-(4,4-difluorocyclohexyl)-3-(methoxymethyl)pyrazine-2-carboxamide

(4,4-difluorocyclohexyl)amine (0.676 g, 5.00 mmol) was dissolved in dry toluene (5.0 mL) under Ar. Upon cooling to 0 °C trimethylaluminium (2.5 mL, 2.0 M in toluene, 5.0 mmol) was added dropwise. After the addition of Me₃Al was complete the ice bath was removed and the solution was allowed to stir for 2 h at ambient temperature. It was then added to a solution of methyl 5,6-bis(4-chlorophenyl)-3-(methoxymethyl)pyrazine-2-carboxylate (0.441 g, 1.09 mmol) in toluene (3.0 mL) under Ar. Heating at 50°C for 20 h. The following morning the reaction mixture was cooled to 0 °C and HCl (aq., 2 M, 10 mL) was slowly added (dropwise). The obtained mixture was transferred to a separation funnel with the aid of dichloromethane (100 mL) and water (100 mL). The organic phase was separated and the aqueous phase was extracted further with dichloromethane (3 x 30 mL). The collected organic phases were washed with water (40 mL) and brine (70 mL) before drying with MgSO₄. Upon evaporation of the solvents the obtained residue was purified by column chromatography (silica gel, EtOAc-dichloromethane, 0-6 %) to yield 5,6-bis(4-chlorophenyl)-*N*-(4,4-difluorocyclohexyl)-3-(methoxymethyl)pyrazine-2-carboxamide (0.511 g, 1.01 mmol, 92 %) as a yellowish powder.
¹H NMR (500 MHz, CDCl₃) δ 7.83 (d, 1H, J=8.1 Hz, N-*H*), 7.49 (d, 2H, J=8.5 Hz), 7.41 (d, 2H, J=8.7 Hz), 7.37 (d, 2H, J=8.7 Hz), 7.33 (d, 2H, J=8.5 Hz), 5.23 (s, 2H), 4.19-4.04 (m, 1H), 3.61 (s, 3H), 2.26-2.04 (m, 4H), 2.04-1.82 (m, 2H), 1.82-1.65 (m, 2H).
HRMS Calcd for [C₂₅H₂₃Cl₂F₂N₃O₂+H]⁺: 506.1213. Found: 506.1208.

### Example 61

### 5,6-bis(4-chlorophenyl)-N-(4,4-difluorocyclohexyl)-3-{[(4-fluorobenzyl)oxy]methyl}pyrazine-2-carboxamide

### Step A 5,6-bis(4-chlorophenyl)-3-{[(4-fluorobenzyl)oxy]methyl}pyrazine-2-carboxylic acid

Ethyl 5,6-bis(4-chlorophenyl)-3-(hydroxymethyl)pyrazine-2-carboxylate (0.561 g, 1.39 mmol) was dissolved in dichloromethane (7 mL). *p*-fluorobenzyl bromide (0.659 g, 3.49 mmol) and tetrabutylammonium hydrogensulphate (47 mg, 0.14 mmol) and then NaOH (aq., 50 % w/w, 7 mL) were added. The mixture quickly adopted a brightly red colour, which then gradually faded with time. Vigorous stirring overnight. The following morning the reaction mixture was cooled to 0 °C before HCl (aq., 4 M, 100 mL) was slowly added (dropwise). The resulting mixture was transferred to a separation funnel with the aid of dichloromethane (50 mL). The organic phase was separated and the aqueous phase was extracted further with dichloromethane (4 x 50 mL). The collected organic phases were dried with MgSO₄. Upon evaporation of the solvents the obtained residue was purified by column chromatography (silica gel, EtOAc-heptane, 0-40 %; upon reaching 40 % EtOAc formic acid, 1 % v/v, was added to the eluent) to yield crude 5,6-bis(4-chlorophenyl)-3-{[(4-fluorobenzyl)oxy]methyl}pyrazine-2-carboxylic acid (0.534 g) as a brownish oil. The material was used as such in the next step without further purification.

### Step B Methyl 5,6-bis(4-chloronhenyl)-3-{[(4-fluorobenzyl)oxy]methyl}pyrazine-2-carboxylate

To a mixture of 5,6-bis(4-chlorophenyl)-3-{[(4-fluorobenzyl)oxy]methyl}pyrazine-2-carboxylic acid (0.534 g, 1.10 mmol) in dichloromethane (12 mL) and MeOH (3 mL) was added Trimethylsilyl diazomethane (0.69 mL, 1.38 mmol, 2 M in hexanes) at such a rate (ca 5 min) as to keep the evolution of nitrogen under control. After the addition was complete the mixture was stirred at ambient temperature overnight. The following morning a few drops of acetic acid were added in order to destroy the excess of trimethylsilyl diazomethane. Silica gel (ca 3 g) was added and the solvents were evaporated. The obtained residue was put on top of a pre-packed column (silica gel, heptane) and the produts were eluted using an EtOAc-heptane gradient (0-20 %). Methyl 5,6-bis(4-chlorophenyl)-3-{[(4-fluorobenzyl)oxy]methyl}pyrazine-2-carboxylate (0.384 g, 0.772 mmol, 70 %) was obtained as a colourless, viscous oil.
¹H NMR (500 MHz, CDCl₃) δ 7.52-7.40 (m, 4H), 7.40-7.30 (m, 6H), 7.08-7.00 (m, 2H), 5.08 (s, 2H), 4.67 (s, 2H), 3.98 (s, 3H).

### Step C 5,6-bis(4-chlorophenyl)-N-(4,4-difluorocyclohexyl)-3-{[(4-fluorobenzyl)oxy]methyl}pyrazine-2-carboxamide

(4,4-difluorocyclohexyl)amine (0.691 g, 5.11 mmol) was dissolved in dry toluene (5.0 mL) under Ar. Upon cooling to 0 °C trimethylaluminium (2.5 mL, 2 M in toluene, 5.0 mmol) was added dropwise. After the addition of Me₃Al was complete the ice bath was removed and the mixture was allowed to stir for 2 h at ambient temperature; consequently, a ca 0.67 M solution of aluminium amide was obtained. A part of this solution (0.9 mL, 1.3 mmol, corresponding to ca 8.6 eq.) was added to methyl 5,6-bis(4-chlorophenyl)-3-{[(4-fluorobenzyl)oxy]methyl}pyrazine-2-carboxylate (35 mg, 0.070 mmol), which had been placed in a dry flask under Ar. Heating at 50 °C for ca 19 h. Upon cooling the reaction mixture to 0 °C toluene (1 mL) was added before HCl (aq., 2 M, 1 mL) was slowly added (dropwise). After stirring at 0 °C for 1 h the obtained mixture was transferred to a separation funnel with the aid of dichloromethane (30 mL) and water (30 mL). The organic phase was separated and the aqueous phase was extracted further with dichloromethane (3 x 10 mL). The collected organic phases were washed with water (10 mL) and brine (30 mL) before drying with MgSO₄. Upon evaporation of the solvents the obtained residue was purified by column chromatography (silica gel, EtOAc-dichloromethane, 0-3 %) to yield 5,6-bis(4-chlorophenyl)-*N*-(4,4-difluorocyclohexyl)-3-{[(4-fluorobenzyl)oxy]methyl}pyrazine-2-carboxamide (33 mg, 0.055 mmol, 78 %) as a white solid.
¹H NMR (500 MHz, CDCl₃) δ 7.79 (d, 1H, J=8.2 Hz, N-*H*), 7.47 (d, 2H, J=8.4 Hz), 7.44-7.35 (m, 6H), 7.33 (d, 2H, J=8.5 Hz), 7.08-6.98 (m, 2H), 5.33 (s, 2H), 4.76 (s, 2H), 4.19-4.03 (m, 1H), 2.24-2.04 (m, 4H), 2.04-1.84 (m, 2H), 1.80-1.64 (m, 2H).
HRMS Calcd for [C₃₁H₂₆Cl₂F₃N₃O₂+H]⁺: 600.1432. Found: 600.1445.

### Example 62

### 5,6-bis(4-chlorophenyl)-3-[(4,4-difluoroperidin-1-yl)methyl]-N-piperidine-1-ylpyrazine-2-carboxamide

### Step A Ethyl 5,6-bis(4-chlorophenyl)-3-[(4,4-difluoropiperidin-1-yl)methyl]pyrazine-2-carboxylate

Ethyl 5,6-bis(4-chlorophenyl)-3-formylpyrazine-2-carboxylate (92 mg, 0.23 mmol) was dissolved in 1,2-dichloroethane (0.8 mL) and 4,4-difluoropiperidine (56 mg, 0.46 mmol) was added. Finally, sodium triacetoxyborohydride (73 mg, 0.34 mmol) was added and the reaction mixture was allowed to stir overnight at ambient temperature. The following morning the reaction mixture was transferred to a separation funnel with the aid of EtOAc (30 mL) and NaHCO₃ (aq., sat., 30 mL). The organic phase was separated and the aqueous phase was extracted further with EtOAc (3 x 10 mL). The collected organic phases were washed with water (15 mL) and brine (30 mL) before drying with MgSO₄. Upon evaporation of the solvents the obtained residue was purified by column chromatography (silica gel, EtOAc-heptane, 0-20 %) to yield ethyl 5,6-bis(4-chlorophenyl)-3-[(4,4-difluoropiperidin-1-yl)methyl]pyrazine-2-carboxylate (89mg, 0.18 mmol) as a colourless sticky oil.
¹H NMR (500 MHz, CDCl₃) δ 7.50-7.40 (m, 4H), 7.38-7.30 (m, 4H), 4.48 (q, 2H, J=7.2 Hz), 4.05 (s, 2H), 2.72-2.54 (m, 4H), 2.05-1.90 (m, 4H), 1.47 (t, 3H, J=7.1 Hz).

### Step B 5,6-bis(4-chlorophenyl)-3-[(4,4-difluoropieridin-1-yl)methyl]-N-piperidine-1-ylpyrazine-2-carboxamide

The hydrochloride of piperidin-1 amine (0.683 g, 5.00 mmol) was suspended in dry toluene (5.0 mL) under Ar. Upon cooling to 0 °C trimethylaluminium (2.5 mL, 2 M in toluene, 5.0 mmol) was added dropwise. After the addition of Me₃Al was complete the ice bath was removed and the mixture was allowed to stir for 3 h at ambient temperature; consequently, a ca 0.67 M solution of aluminium amide was obtained. A part of this solution (2.0 mL, 1.3 mmol, corresponding to ca 7.4 eq.) was added to ethyl 5,6-bis(4-chlorophenyl)-3-[(4,4-difluoropiperidin-1-yl)methyl]pyrazine-2-carboxylate (0.092 g, 0.18 mmol), which had been placed in a dry flask under Ar. Heating at 50 °C for 18 h. Upon cooling to 0 °C toluene (ca 2 mL) was added before HCl (aq., 2 M, 2 mL) was slowly added (dropwise). The obtained mixture was allowed to stir at 0 °C for ca 3 h before it was transferred to a separation funnel with the aid of dichloromethane (30 mL) and water (30 mL). The pH of the aqueous phase was adjusted to 6-7 with NaOH (aq., 2 M). The organic phase was separated and the aqueous phase was extracted further with dichloromethane (3 x 10 mL). The collected organic phases were washed with water (10 mL) and brine (30 mL) before drying with MgSO₄. Upon evaporation of the solvents the obtained residue was purified by two rounds of column chromatography: 1) silica gel, EtOAc-heptane, 0-50 %; 2) silica gel , EtOAc-dichloromethane, 0-30 %. This yielded 5,6-bis(4-chlorophenyl)-3-[(4,4-difluoropiperidin-1-yl)methyl]-*N*-piperidine-1-ylpyrazine-2-carboxamide (82 mg, 0.15 mmol, 80 %) as a viscous, yellowish oil.
¹H NMR (500 MHz, CDCl₃) δ 8.51 (br s, 1H, N-*H*), 7.48-7.40 (m, 4H), 7.36 (d, 2H, J=8.4 Hz), 7.33 (d, 2H, J=8.4 Hz), 4.36 (s, 2H), 2.98-2.86 (m, 4H), 2.86-2.78 (m, 4H), 2.10-1.94 (m, 4H), 1.88-1.74 (m, 4H), 1.56-1.42 (m, 2H).
HRMS Calcd for [C₂₈H₂₉Cl₂F₂N₅O+H]⁺: 560.1796. Found: 560.1794.

### Example 63

### 5,6-bis(4-chlorophenyl)-N-(4,4-difluorocyclohexyl)-3-[(4,4-difluoropiperidin-1-yl)methyl]pyrazine-2-carboxamide

(4,4-difluorocyclohexyl)amine (0.691 g, 5.11 mmol) was dissolved in dry toluene (5.0 mL) under Ar. Upon cooling to 0 °C trimethylaluminium (2.5 mL, 2 M in toluene, 5.0 mmol) was added dropwise. After the addition of Me₃Al was complete the ice bath was removed and the mixture was allowed to stir for 2 h at ambient temperature; consequently, a ca 0.67 M solution of aluminium amide was obtained. A part of this solution (2.2 mL, 1.5 mmol, corresponding to ca 8 eq.) was added to ethyl 5,6-bis(4-chlorophenyl)-3-[(4,4-difluoropiperidin-1-yl)methyl]pyrazine-2-carboxylate (0.092 g, 0.18 mmol), which had been placed in a dry round flask under Ar. Heating at 50 °C for 19 h. Upon cooling to 0 °C toluene (ca 2 mL) was added before HCl (aq., 2 M, 2 mL) was slowly added (dropwise). The obtained mixture was allowed to stir at 0 °C for 1 h before it was transferred to a separation funnel with the aid of dichloromethane (30 mL) and water (30 mL). The organic phase was separated and the aqueous phase was extracted further with dichloromethane (3 x 10 mL). The collected organic phases were washed with water (10 mL) and brine (30 mL) before drying with MgSO₄. Upon evaporation of the solvents the obtained residue was purified by column chromatography (silica gel, EtOAc-dichloromethane, 0-14 %) to yield 5,6-bis(4-chlorophenyl)-*N*-(4,4-difluorocyclohexyl)-3-[(4,4-difluoropiperidin-1-yl)methyl]pyrazine-2-carboxamide (84 mg, 0.14 mmol, 78 %) as a white solid.
¹H NMR (500 MHz, CDCl₃) δ 7.91 (br s, 1H, J=8.1 Hz, N-*H*), 7.45 (d, 2H, J=8.5 Hz), 7.42 (d, 2H, J=8.5 Hz), 7.37 (d, 2H, J=8.5 Hz), 7.33 (d, 2H, J=8.5 Hz), 4.38 (s, 2H), 4.18-4.04 (m, 1H), 2.90-2.76 (m, 4H), 2.26-1.86 (m, 12H).
HRMS Calcd for [C₂₉H₂₈Cl₂F₄N₄O+H]⁺: 595.1655. Found: 595.1611.

### Reference Example 64

### 5,6-bis(4-chlorophenyl)-N-(4,4-difluoropiperidin-1-yl)-3-(methoxymethyl)pyrazine-2-carboxamide

The hydrochloride of 4,4-difluoropiperidin-1-amine (0.863 g, 5.00 mmol) was suspended in dry toluene (5.0 mL) in a dry round flask under Ar. Upon cooling to 0 °C trimethylaluminium (2.5 mL, 2.0 M in toluene, 5.0 mmol) was added dropwise. When the addition of Me₃Al was complete the flask was removed from the ice bath and the mixture was allowed to stir at ambient temperature. After stirring for 2 h the cognac-coloured solution (not entirely homogeneous: a few solid lumps remained) was added to a solution of methyl 5,6-bis(4-chlorophenyl)-3-(methoxymethyl)pyrazine-2-carboxylate (0.407 g, 1.00 mmol) in dry toluene (3.0 mL) under Ar. Heating at 50 °C for 20 h. Upon cooling the reaction mixture to 0 °C HCl (aq., 2 M, 10 mL) was slowly added (dropwise). The obtained mixture was transferred to a separation funnel with the aid of dichloromethane (100 mL) and water (100 mL). The organic phase was separated and the aqueous phase was extracted further with dichloromethane (3 x 30 mL). The collected organic phases were washed with water (40 mL) and brine before drying with MgSO₄. Upon evaporation of the solvents the obtained residue was purified by column chromatography (silica gel, EtOAc-dichloromethane, 0-15 %) to yield 5,6-bis(4-chlorophenyl)-*N*-(4,4-difluoropiperidin-1-yl)-3-(methoxymethyl)pyrazine-2-carboxamide (0.401 g, 0.79 mmol, 78 %) as a slightly yellowish solid.
¹H NMR (500 MHz, CDCl₃) δ 8.63 (br s, 1H, N-*H*), 7.48 (d, 2H, J=8.7 Hz), 7.40 (d, 2H, J=8.7 Hz), 7.37 (d, 2H, J=8.7 Hz), 7.32 (d, 2H, J=8.7 Hz), 5.20 (s, 2H), 3.60 (s, 3H), 3.17-3.07 (m, 4H), 2.30-2.17 (m, 4H).
HRMS Calcd for [C₂₄H₂₂Cl₂F₂N₄O₂+H]⁺: 507.1166. Found: 507.1165.

### Pharmacological Activity

Compounds of the present invention are active against the receptor product of the CB1 gene. The compounds of the present invention are active at the CB1 receptor (IC50 <1 micromolar). Most preferred compounds have IC50 <200 nanomolar. The affinity of the compounds of the invention for central cannabinoid receptors is demonstrable in methods described in Devane et al" Molecular Pharmacology, 1988, 34,605 or those described in WO01/70700 or EP 656354. Alternatively the assay may be performed as follows.

10µg of membranes prepared from cells stably transfected with the CB1 gene were suspended in 200µl of 100mM NaCl, 5mM MgCl₂, 1mM EDTA, 50mM HEPES (pH 7.4), 1mM DTT, 0.1% BSA and 100µM GDP. To this was added an EC80 concentration of agonist (CP55940), the required concentration of test compound and 0.1µCi [³⁵S]-GTPγS. The reaction was allowed to proceed at 30°C for 45 min. Samples were then transferred on to GF/B filters using a cell harvester and washed with wash buffer (50mM Tris (pH 7.4), 5mM MgCl₂, 50mM NaCl). Filters were then covered with scintilant and counted for the amount of [³⁵S]-GTPγS retained by the filter.

Activity is measured in the absence of all ligands (minimum activity) or in the presence of an EC80 concentration of CP55940 (maximum activity). These activities are set as 0% and 100% activity respectively. At various concentrations of novel ligand, activity is calculated as a percentage of the maximum activity and plotted. The data are fitted using the equation y=A+((B-A)/1+((C/x) ÙD)) and the IC50 value determined as the concentration required to give half maximal inhibition of GTPγS binding under the conditions used.

Selected examples of the present invention exhibit the following data:
- Example 9:: 1.8 nM
- Example 27:: 13.5 nM
- Example 35: 1.4 nM

The compounds of the present invention may provide additional benefits in terms of potency, selectivity, bioavailability, half-life in plasma, blood brain permeability, plasma protein binding or solubility compared to representative reference CB1 antagonists/inverse agonist agents.

## Claims

1. A compound of formula (I) 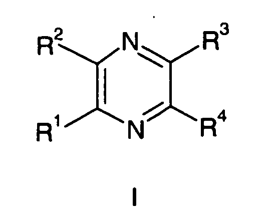 and pharmaceutically acceptable salts thereof, in which
R¹ and R² independently represent phenyl, thienyl or pyridyl each of which is independently optionally substituted by one or more groups represented by Z;
Z represents a C₁₋₈alkyl group, a C₁₋₆alkoxy group, hydroxy, halo, trifluoromethyl, trifluoromethylthio, trifluoromethoxy, trifluoromethylsulphonyl, nitro, mono or di C₁₋₃alkylamido, C₁₋₃alkylsulphonyl, C₁₋₃alkylsulphonyloxy, C₁₋₃alkoxycarbonyl, carboxy, cyano, carbamoyl, mono or di C₁₋₃alkyl carbamoyl, sulphamoyl, acetyl, an aromatic heterocyclic group which is optionally substituted by one or more halo, C₁₋₄alkyl , trifluoromethyl or trifluoromethoxy and a saturated or partially unsaturated 5 to 8 membered heterocyclic group containing one or more heteroatoms selected from nitrogen, oxygen or sulphur wherein the heterocyclic group is optionally substituted by one or more C₁₋₃alkyl groups, hydroxy, fluoro, benzyl or an amino group -NR^{x}R^{y} in which R^{x} and R^{y} independently represent **H** or C₁₋₄alkyl ;
R³ represents a group of formula X-Y-NR⁵R⁶
in which X is CO or SO₂
and Y is absent or represents NH optionally substituted by a C₁₋₃alkyl group
and R⁵ and R⁶ independently represent :
a C₁₋₆alkyl group;
an (amino)C₁₋₄alkyl- group in which the amino is optionally substituted by one or more C₁₋₃alkyl groups;
a group (C₃₋₁₂cycloalkyl)(CH₂)_{g}- wherein g is 0,1, 2 or 3 wherein the cycloalkyl is optionally substituted by one or more fluoro, hydroxy, C₁₋₃alkyl C₁₋₃alkoxy, trifluoromethyl or trifluoromethoxy;
a group -(CH₂)ᵣ(phenyl )ₛ in which r is 0,1, 2, 3 or 4, s is 1 when r is 0 otherwise s is 1 or 2 and the phenyl groups are optionally independently substituted by one or more groups represented by Z;
naphthyl;
anthracenyl;
a saturated or partially unsaturated 5 to 8 membered heterocyclic group containing one or more heteroatoms selected from nitrogen, oxygen or sulphur wherein the heterocyclic group is optionally substituted by one or more C₁₋₃alkyl groups, hydroxy, fluoro, trifluoromethyl , benzyl or an amino group -NR^{x}R^{y} in which R^{x} and R^{y} independently represent H or C₁₋₄alkyl ;
1-adamantylmethyl;
a group - (CH₂)ₜ Het in which t is 0,1, 2, 3 or 4, and the alkylene chain is optionally substituted by one or more C₁₋₃alkyl groups and Het represents an aromatic heterocyclic group optionally substituted by one, two or three groups selected from a C₁₋₅alkyl group, a C₁₋₅alkoxy group or halo;
or R⁵ represents H and R⁶ is as defined above;
or R⁵ and R⁶ together with the nitrogen atom to which they are attached represent a saturated or partially unsaturated 5 to 8 membered heterocyclic group containing one nitrogen and optionally one of the following : oxygen, sulphur or an additional nitrogen; wherein the heterocyclic group is optionally substituted by one or more more C₁₋₃alkyl groups, hydroxy, fluoro, trifluoromethyl, trifluoromethoxy , benzyl, C₁₋₆alkanoyl or an amino group -NR^{x}R^{y} in which R^{x} and R^{y} independently represent H or C₁₋₄alkyl ;
R⁴ represents a group of formula (CH₂)ₙCOOR⁷
in which n is 0, 1, 2, 3 or 4; and R⁷ represents a C₄₋₁₂alkyl group, a C₃₋₁₂cycloalkyl group or a (C₃₋₁₂cycloalkyl)C₁₋₃alkyl- group each of which is optionally substituted by one or more of the following: a C₁₋₆alkyl group; fluoro, amino or hydroxy, or
R⁷ represents a group -(CH₂)ₐphenyl in which a is 0, 1, 2, 3 or 4 and the phenyl group is optionally substituted by one or more groups represented by Z which may be the same or different or
R⁷ represents a saturated or partially unsaturated 5 to 8 membered heterocyclic group containing one or more of the of the following : oxygen, sulphur or nitrogen; wherein the heterocyclic group is optionally substituted by one or more C₁₋₃alkyl groups, C₁₋₃acyl groups, hydroxy, amino or benzyl; or
R⁴ represents a group of formula -(CH₂)ₒ-O-(CH₂)ₚ- R⁸ in which o and p independently represent an integer 0, 1, 2, 3 or 4 and R⁸ represents a C₁₋₁₂alkyl group with the proviso that R⁴ is not a C₁₋₃alkoxymethyl group, or R⁸ represents phenyl optionally independently substituted by one or more Z groups or R⁸ represents an aromatic heterocyclic group or a saturated or partially unsaturated 5 to 8 membered heterocyclic group containing one or more of one following : oxygen, sulphur or nitrogen wherein each of these rings is optionally substituted by one or more groups represented by Z which may be the same or different ;
R⁴ represents a C₄₋₁₂alkyl group optionally substituted by one or more fluoro, hydroxy, or amino; or
R⁴ represents a group of formula -(CH₂)_{q}R⁹ in which q is 0, 1, 2, 3 or 4 and R⁹ represents a C₃₋₁₂cycloalkyl group, phenyl, an aromatic heterocyclic group or a saturated or partially unsaturated 5 to 8 membered heterocyclic group containing one or more of one following : oxygen, sulphur or nitrogen wherein each of these rings is optionally substituted by one or more groups represented by Z which may be the same or different ; or
R⁴ represents a group of formula -(CH₂)ₘ-O-(CO)- R¹⁰ in which m represents an integer 0, 1, 2, 3 or 4 , in which R¹⁰ represents a C₁₋₁₂alkyl group optionally substituted by one or more fluoro, hydroxy, or amino or R¹⁰ represents a group of formula -(CH₂)_{q}R⁹ in which q and R⁹ is as previously described,
wherein the term aromatic heterocyclic group means an aromatic 5-, 6-, or 7-membered monocyclic ring or a 9- or 10-membered bicyclic ring, with up to five ring heteroatoms selected from oxygen, nitrogen and sulphur.

2. A formula I as claimed in claim 1 as represented by formula IIa 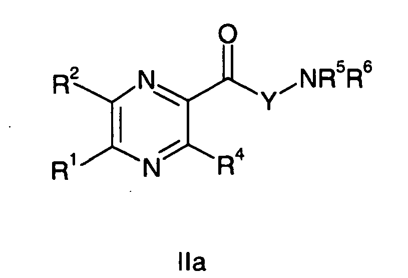 wherein R¹ and R² independently represent phenyl optionally independently substituted by halo or pyridyl,
R⁴ represents a C₄₋₈alkyl group, a group CH₂OR⁸ in which R⁸ is a C₄₋₈alkyl group, a group CO₂R⁷ in which R⁷ represents a C₄₋₈alkyl group,
and Y is represents NH and
R⁵ represents H and
R⁶ represents perfluorophenyl or phenyl optionally substituted by trifluoromethyl or R⁵ and R⁶ together with the nitrogen to which they are attached represent piperidino, morpholino or piperazino each of which is optionally substituted by one or more C₁₋₃alkyl groups, hydroxy, fluoro, trifluoromethyl, trifluoromethoxy, benzyl, C₁₋₆alkanoyl or an amino group -NR^{x}R^{y} in which R^{x} and R^{y} independently represent H or C₁₋₄alkyl;
or Y is absent and
R⁵ represents H or a C₁₋₆alkyl group optionally substituted by amino;
R⁶ represents tetrahydropyranyl or 4- piperidinyl optionally substituted by one or more C₁₋₃alkyl groups, hydroxy, fluoro, trifluoromethyl, trifluoromethoxy, benzyl, C₁₋₅alkanoyl or an amino group -NR^{x}R^{y} in which R^{x} and R^{y} independently represent H or C₁₋₄alkyl or a C₁₋₆alkyl group optionally substituted by amino;
or
R⁵ and R⁶ together with the nitrogen to which they are attached represent piperidino, morpholino or piperazino each of which is optionally substituted by C₁₋₃alkyl or fluoro.

3. A compound of formula I as claimed in claim 1 as represented by formula IIb 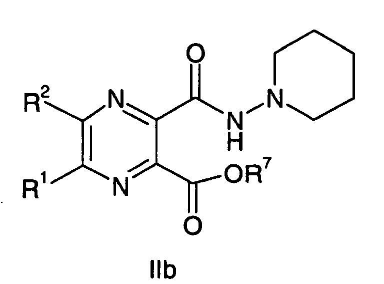 wherein R¹ and R² represent phenyl independently optionally substituted by one or more chloro; and
R⁷ represents butyl, tert-butyl, cyclohexyl or benzyl.

4. A compound according to claim 1 selected from one or more of the following:
*tert*-butyl 5,6-bis(4-chlorophenyl)-3-[(piperidin-1-ylamino)carbonyl]pyrazine-2-carboxylate;
butyl 5,6-bis(4-chlorophenyl)-3-[(piperidin-1-ylamino)carbonyl]pyrazine-2-carboxylate;
cyclohexyl 5,6-bis(4-chlorophenyl)-3-[(piperidin-1-ylamino)carbonyl]pyrazine-2-carboxylate;
benzyl 5,6-bis(4-chlorophenyl)-3-[(piperidin-1-ylamino)carbonyl]pyrazine-2-carboxylate;
tert-butyl 5,6-bis(4-chlorophenyl)-3-({[cis-2-hydroxycyclohexyl]amino}carboxyl)-pyrazine-2-carboxylate;
tert-butyl 5,6-bis(4-chlorophenyl)-3-({[trans-2-hydroxycyclohexyl]amino}carbonyl)-pyrazine-2-carboxylate;
tert-butyl 5,6-bis(4-chlorophenyl)-3-({2-[4-(trifluoromethyl)phenyl]hydrazino}carbonyl)-pyrazine-2-carboxylate;
tert-butyl 5,6-bis(4-chlorophenyl)-3-[(morpholin-4-ylamino)carbonyl]pyrazine-2-carboxylate;
tert-butyl 5,6-bis(4-chlorophenyl)-3-({2-[4-(tert-butylhydrazino}carbonyl)pyrazine-2-carboxylate;
tert-butyl 5,6-bis(4-chlorophenyl)-3-{[(4,4-difluorocyclohexyl)amino]carbonyl}pyrazine-2-carboxylate;
tert-butyl 5,6-bis(4-chlorophenyl)-3-[(pentylamino)carbonyl]pyrazine-2-carboxylate;
tert-butyl 5,6-bis(4-chlorophenyl)-3-{[(1-ethylpropyl)amino]carbonyl}pyrazine-2-carboxylate;
tert-butyl 5,6-bis(4-chlorophenyl)-3-{[(4,4-difluoropiperidin-1-yl)amino]carbonyl}-pyrazine-2-carboxylate;
5,6-bis(4-chlorophenyl)-N-piperidin-1-yl-3-[(4-propyl-1H-1,2,3-triazol-1-yl)methyl]pyrazine-2-carboxamide;
5,6-bis(4-chlorophenyl)-3-(phenoxymethyl)-*N*-piperidin-1-ylpyrazine-2-carboxamide;
5,6-bis(4-chlorophenyl)-3-(morpholin-4-ylmethyl)-*N*-piperidin-1-ylpyrazine-2-carboxamide;
5,6-bis(4-chlorophenyl)-3-(piperidin-1-ylmethyl)-*N*-piperidin-1-ylpyrazine-2-carboxamide;
*Tert*-butyl 5,6-bis(4-methylphenyl)-3-[( piperidin-1-ylamino)carbonyl]pyrazine-2-carboxylate;
5,6-bis(4-methylphenyl)-*N*-piperidin-1-yl -3 -(1*H*-tetrazol-1-ylmethyl)pyrazine-2-carboxamide;
5,6-bis(4-methylphenyl)-*N*-piperidin-1-yl -3 -(2*H*-tetrazol-2-ylmethyl)pyrazine-2-carboxamide;
5,6-bis(4-chlorophenyl)-*N*-piperidin-1-yl -3 -(2*H*-tetrazol-2-ylmethyl)pyrazine-2-carboxamide;
5,6-bis(4-chlorophenyl)-*N*-piperidin-1-yl-3-(1*H*-tetrazol-1-ylmethyl)pyrazine-2-carboxamide;
5,6-bis(4-chlorophenyl)-*N*-(4,4-difluorocyclohexyl)-3-(2*H*-tetrazol-2-ylmethyl)pyrazine-2-carboxamide;
5,6-bis(4-chlorophenyl)-*N*-(4,4-difluoropiperidin-1-yl)-3-(2*H*-tetrazol-2-ylmethyl)pyrazine-2-carboxamide;
5,6-bis(4-chlorophenyl)-3-[(5-methyl-2*H*-tetrazol-2-yl)methyl]-*N*-piperidin-1-ylpyrazine-2-carboxamide;
5,6-bis(4-chlorophenyl)-3-[(5-methyl-1*H*-tetrazol-1-yl)methyl]-*N*-piperidin-1-ylpyrazine-2-carboxamide;
*tert*-butyl 6-(4-chlorophenyl)-5-(4-methylphenyl)-3-[(piperidin-1-ylamino)carbonyl]pyrazine-2-carboxylate;
*tert*-butyl 5-(4-chlorophenyl)-6-(4-methylphenyl)-3-[(piperidin-1-ylamino)carbonyl]pyrazine-2-carboxylate;
6-(4-chlorophenyl)-5-(4-methylphenyl)-*N*-piperidin-1-yl-3-(2*H*-tetrazol-2-ylmethyl)pyrazine-2-carboxamide;
5-(4-chlorophenyl)-6-(4-methylphenyl)-*N*-piperidin-1-yl-3-(2*H*-tetrazol-2-ylmethyl)pyrazine-2-carboxamide;
5,6-bis-(4-chloro-phenyl)-3-propoxy-pyrazine-2-carboxylic acid piperidin-1-ylamide;
5,6-bis(4-chlorophenyl)-*N*-piperidin-1-yl-3-(2*H-*tetrazol-5-ylmethyl)pyrazine-2-carboxamide
5,6-bis(4-chlorophenyl)-*N*-piperidin-1-yl-3-(1*H*-tetrazol-5-yl)pyrazine-2-carboxamide;
5,6-bis(4-chlorophenyl)-3-[(5-morpholin-4-yl-2*H*-tetrazol-2-yl)methyl]-*N*-piperidin-1-ylpyrazine-2-carboxamide;
5,6-bis(4-chlorophenyl)-3-[(5-morpholin-4-yl-1*H*-tetrazol-1-yl)methyl]-*N*-piperidin-1-ylpyrazine-2-carboxamide;
5,6-bis(4-chlorophenyl)-*N*-piperidin-1-yl-3-[(5-pyrrolidin-1-yl-2*H*-tetrazol-2-yl)methyl]pyrazine-2-carboxamide;
5,6-bis(4-chlorophenyl)-*N*-piperidin-1-yl-3-[(5-pyrrolidin-1-yl-1*H*-tetrazol-1-yl)methyl]pyrazine-2-carboxamide;
5,6-bis(4-chlorophenyl)-*N*-(4,4-difluorocyclohexyl)-3-{[(4-fluorobenzyl)oxy]methyl}pyrazine-2-carboxamide;
5,6-bis(4-chlorophenyl)-3-[(4,4-difluoropiperidin-1-yl)methyl]-*N*-piperidine-1-ylpyrazine-2-carboxamide; or
5,6-bis(4-chlorophenyl)-*N*-(4,4-difluorocyclohexyl)-3-[(4,4-difluoropiperidin-1-yl)methyl]pyrazine-2-carboxamide;
and pharmaceutically acceptable salts thereof.

5. A compound according to claim 1 selected from one or more of the following:
*tert*-butyl 5,6-bis(4-chlorophenyl)-3-[(piperidin-1-ylamino)carbonyl]pyrazine-2-carboxylate;
butyl 5,6-bis(4-chlorophenyl)-3-[(piperidin-1-ylamino)carbonyl]pyrazine-2-carboxylate;
cyclohexyl 5,6-bis(4-chlorophenyl)-3-[(piperidin-1-ylamino)carbonyl]pyrazine-2-carboxylate; and
benzyl 5,6-bis(4-chlorophenyl)-3-[(piperidin-1-ylamino)carbonyl]pyrazine-2-carboxylate and pharmaceutically acceptable salts thereof.

6. A compound of formula I as claimed in any previous claim for use as a medicament.

7. A pharmaceutical formulation comprising a compound of formula I, as defined in any of claims 1 to 5, and a pharmaceutically acceptable adjuvant, diluent or carrier.

8. The use of a compound according to any of claims 1 to 5 in the preparation of a medicament for the treatment or prophylaxis of obesity, psychiatric disorders such as psychotic disorders, schizophrenia and bipolar disorders, anxiety, anxio-depressive disorders, depression, cognitive disorders, memory disorders, obsessive-compulsive disorders, anorexia, bulimia, attention disorders, epilepsy, and related conditions, and neurological disorders such as dementia, neurological disorders, Parkinson's Disease, Huntington's Chorea and Alzheimer's Disease, immune, cardiovascular, reproductive and endocrine disorders, septic shock, diseases related to the respiratory and gastrointestinal systems, and extended abuse, addiction and/or relapse indications.

9. The use according to claim 8 in the preparation of a medicament for the treatment or prophylaxis of obesity.

## Patentansprüche

1. Verbindungen der Formel (I) 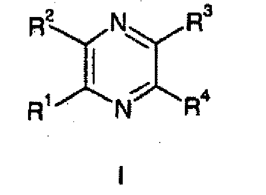 und deren pharmazeutisch annehmbare Salze, wobei
R¹ und R² unabhängig voneinander für Phenyl, Thienyl oder Pyridyl stehen, die jeweils unabhängig voneinander gegebenenfalls durch eine oder mehrere durch Z wiedergegebene Gruppen substituiert sind;
Z für eine C₁₋₈-Alkylgruppe, eine C₁₋₆-Alkoxygruppe, Hydroxy, Halogen, Trifluormethyl, Trifluormethylthio, Trifluormethoxy, Trifluormethylsulfonyl, Nitro, Mono- oder Di-C₁₋₃-alkylamido, C₁₋₃-Alkylsulfonyl, C₁₋₃-Alkylsulfonyloxy, C₁₋₃-Alkoxycarbonyl, Carboxyl, Cyano, Carbamoyl, Mono- oder Di-C₁₋₃-alkylcarbamoyl, Sulfamoyl, Acetyl, eine aromatische heterocyclische Gruppe, die gegebenenfalls durch einen oder mehrere Halogen-, C₁₋₄-Alkyl-, Trifluormethyl- oder Trifluormethoxyreste substituiert ist, oder eine gesättigte oder teilweise ungesättigte 5- bis 8-gliedrige heterocyclische Gruppe mit einem oder mehreren Heteroatomen ausgewählt aus Stickstoff, Sauerstoff und Schwefel, wobei die heterocyclische Gruppe gegebenenfalls durch eine oder mehrere C₁₋₃-Alkylgruppen, Hydroxy, Fluor, Benzyl oder eine Aminogruppe -NR^{x}R^{y}, in welcher R^{x} und R^{y} unabhängig voneinander für H oder C₁₋₄-Alkyl stehen, steht;
R³ für eine Gruppe der Formel X-Y-NR⁵R⁶ steht,
wobei X für CO oder SO₂ steht
und Y fehlt oder für gegebenenfalls durch eine C₁₋₃-Alkylgruppe substituiertes NH steht
und R⁵ und R⁶ unabhängig voneinander für folgendes stehen:
eine C₁₋₆-Alkylgruppe;
eine (Amino)-C₁₋₄-alkylgruppe, wobei die Aminogruppe gegebenenfalls durch eine oder mehrere C₁₋₃-Alkylgruppen substituiert ist;
eine Gruppe (C₃₋₁₂-Cycloalkyl) (CH₂)_{g}-, wobei g für 0, 1, 2 oder 3 steht, wobei der Cycloalkylrest gegebenenfalls durch einen oder mehrere Fluor-, Hydroxyl-, C₁₋₃-Alkyl-, C₁₋₃-Alkoxy-, Trifluormethyl- oder Trifluormethoxyreste substituiert ist;
eine Gruppe - (CH₂)ᵣ(Phenyl)ₛ, wobei r für 0, 1, 2, 3 oder 4 steht, s für 1 steht, wenn r für 0 steht, und ansonsten s für 1 oder 2 steht und die Phenylgruppen gegebenenfalls unabhängig voneinander substituiert durch eine oder mehrere durch Z wiedergegebene Gruppen sind;
Naphthyl;
Anthracenyl;
eine gesättigte oder teilweise ungesättigte 5- bis 8-gliedrige heterocyclische Gruppe, die ein oder mehrere Heteroatome ausgewählt aus Stickstoff, Sauerstoff und Schwefel enthält, wobei die heterocyclische Gruppe gegebenenfalls durch einen oder mehrere C₁₋₃-Alkylgruppen, Hydroxy, Fluor, Trifluormethyl, Benzyl oder eine Aminogruppe -NR^{x}R^{y}, in welcher R^{x} und R^{y} unabhängig voneinander für H oder C₁₋₄-Alkyl stehen, substituiert ist; 1-Adamantylmethyl;
eine Gruppe -(CH₂)ₜ-Het, in welcher t für 0, 1, 2, 3 oder 4 steht und die Alkylenkette gegebenenfalls durch eine oder mehrere C₁₋₃-Alkylgruppen substituiert ist und Het für eine aromatische heterocyclische Gruppe steht, die gegebenenfalls durch 1, 2 oder 3 Gruppen, ausgewählt aus einer C₁₋₅-Alkylgruppe, einer C₁₋₅-Alkoxygruppe und Halogen substituiert ist, steht;
oder R⁵ für H steht und R⁶ wie oben definiert ist;
oder R⁵ und R⁶ zusammen mit dem Stickstoffatom, an das sie gebunden sind, für eine gesättigte oder teilweise ungesättigte 5- bis 8-gliedrige heterocyclische Gruppe mit einem Stickstoff und gegebenenfalls einem der folgenden: Sauerstoff, Schwefel oder einem zusätzlichen Stickstoff stehen; wobei die heterocyclische Gruppe gegebenenfalls durch eine oder mehrere C₁₋₃-Alkylgruppen, Hydroxy, Fluor, Trifluormethyl, Trifluormethoxy, Benzyl, C₁₋₆-Alkanoyl oder eine Aminogruppe -NR^{x}R^{y}, in welcher R^{x} und R^{y} unabhängig voneinander für H oder C₁₋₄-Alkyl stehen, substituiert ist;
R⁴ für eine Gruppe der Formel (CH₂)ₙCOOR⁷ steht, wobei n für 0, 1, 2, 3 oder 4 steht; und R⁷ für eine C₄₋₁₂-Alkylgruppe, eine C₃₋₁₂-Cycloalkylgruppe oder eine (C₃₋₁₂-Cycloalkyl)-C₁₋₃-alkylgruppe steht, die jeweils gegebenenfalls durch eine oder mehrere der folgenden substituiert sind: eine C₁₋₆-Alkylgruppe, Fluor, Amino oder Hydroxy, oder
R⁷ für eine Gruppe -(CH₂)ₐ-Phenyl steht, wobei a für 0, 1, 2, 3 oder 4 steht und die Phenylgruppe gegebenenfalls durch eine oder mehrere durch Z wiedergegebene Gruppen, die gleich oder verschieden sein können, substituiert ist, oder
R⁷ für eine gesättigte oder teilweise ungesättigte 5- bis 8-gliedrige heterocyclische Gruppe mit einem oder mehreren der folgenden: Sauerstoff, Schwefel oder Stickstoff steht; wobei die heterocyclische Gruppe gegebenenfalls durch eine oder mehrere C₁₋₃-Alkylgruppen, C₁₋₃-Acylgruppen, Hydroxy, Amino oder Benzyl substituiert ist; oder
R⁴ für eine Gruppe der Formel -(CH₂)ₒ-O-(CH₂)ₚ-R⁸ steht, in welcher o und p unabhängig voneinander für eine ganze Zahl 0, 1, 2, 3 oder 4 stehen und R⁸ für eine C₁₋₁₂-Alkylgruppe steht, mit der Maßgabe, daß R⁴ nicht für eine C₁₋₃-Alkoxymethylgruppe steht oder R⁸ für gegebenenfalls unabhängig durch eine oder mehrere Z-Gruppen substituiertes Phenyl steht oder R⁸ für eine aromatische heterocyclische Gruppe oder eine gesättigte oder teilweise ungesättigte 5- bis 8-gliedrige heterocyclische Gruppe mit einem oder mehreren der folgenden: Sauerstoff, Schwefel oder Stickstoff steht, wobei diese Ringe jeweils gegebenenfalls durch eine oder mehrere durch Z wiedergegebene Gruppen, die gleich oder verschieden sein können, substituiert sind;
R⁴ für eine C₄₋₁₂-Alkylgruppe, gegebenenfalls substituiert durch eine oder mehrere Fluor-, Hydroxyl- oder Aminogruppen, steht; oder
R⁴ für eine Gruppe der Formel -(CH₂)_{q}R⁹ steht, in welcher q für 0, 1, 2, 3 oder 4 steht und R⁹ für eine C₃₋₁₂-Cycloalkylgruppe, Phenyl, eine aromatische heterocyclische Gruppe oder eine gesättigte oder teilweise ungesättigte, 5- bis 8-gliedrige heterocyclische Gruppe mit einem oder mehreren der folgenden: Sauerstoff, Schwefel oder Stickstoff steht, wobei diese Ringe jeweils gegebenenfalls durch eine oder mehrere durch Z wiedergegebene Gruppen, die gleich oder verschieden sein können, substituiert sind; oder
R⁴ für eine Gruppe der Formel -(CH₂)ₘ-O-(CO)-R¹⁰ steht, in welcher m für eine ganze Zahl 0, 1, 2, 3 oder 4 steht, in welcher R¹⁰ für eine C₁₋₁₂-Alkylgruppe steht, die gegebenenfalls durch eine oder mehrere Fluor-, Hydroxyl- oder Aminogruppen substituiert ist, oder R¹⁰ für eine Gruppe der Formel -(CH₂)_{q}R⁹ steht, in welcher q und R⁹ wie oben beschrieben sind;
wobei der Ausdruck aromatische heterocyclische Gruppe einen aromatischen 5-, 6- oder 7-gliedrigen monocyclischen Ring oder einen 9- oder 10-gliedrigen bicyclischen Ring mit bis zu fünf Ringheteroatomen ausgewählt aus Sauerstoff, Stickstoff und Schwefel bedeutet.

2. Verbindung der Formel I nach Anspruch 1, wiedergegeben durch die Formel IIa, 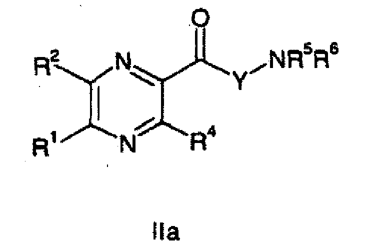 wobei R¹ und R² unabhängig voneinander für Phenyl stehen, das gegebenenfalls unabhängig durch Halogen oder Pyridyl substituiert ist,
R⁴ für eine C₄₋₈-Alkylgruppe, eine Gruppe CH₂OR⁸, in welcher R⁸ für eine C₄₋₈-Alkylgruppe steht, eine Gruppe CO₂R⁷, in welcher R⁷ für eine C₄₋₈-Alkylgruppe steht, steht,
und Y für NH steht und
R⁵ für H steht und
R⁶ für Perfluorphenyl oder gegebenenfalls durch Trifluormethyl substituiertes Phenyl steht oder R⁵ und R⁶ zusammen mit dem Stickstoff, an den sie gebunden sind, für Piperidino, Morpholino oder Piperazino stehen, die jeweils gegebenenfalls durch eine oder mehrere C₁₋₃-Alkylgruppen, Hydroxy, Fluor, Trifluormethyl, Trifluormethoxy, Benzyl, C₁₋₆-Alkanoyl oder eine Aminogruppe -NR^{x}R^{y}, in welcher R^{x} und R^{y} unabhängig voneinander für H oder C₁₋₄-Alkyl stehen, substituiert sind;
oder Y fehlt und
R⁵ für H oder eine gegebenenfalls durch Amino substituierte C₁₋₆-Alkylgruppe steht;
R⁶ für Tetrahydropyranyl oder 4-Piperidinyl, gegebenenfalls substituiert durch eine oder mehrere C₁₋₃-Alkylgruppen, Hydroxy, Fluor, Trifluormethyl, Trifluormethoxy, Benzyl, C₁₋₆-Alkanoyl oder eine Aminogruppe -NR^{x}R^{y}, in welcher R^{x} und R^{y} unabhängig voneinander für H oder C₁₋₄-Alkyl stehen, oder eine C₁₋₆-Alkylgruppe, gegebenenfalls substituiert durch Amino, steht; oder
R⁵ und R⁶ zusammen mit dem Stickstoff, an den sie gebunden sind, für Piperidino, Morpholino oder Piperazino stehen, die jeweils gegebenenfalls durch C₁₋₃-Alkyl oder Fluor substituiert sind.

3. Verbindungen der Formel I nach Anspruch 1, wiedergegeben durch die Formel IIb 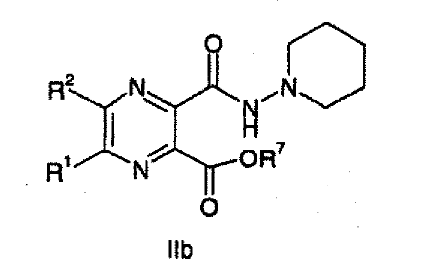 In welcher R¹ und R² unabhängig voneinander für Phenyl stehen, das gegebenenfalls durch eine oder mehrere Chlorgruppen substituiert ist; und
R⁷ für Butyl, tert.-Butyl, Cyclohexyl oder Benzyl steht.

4. Verbindungen nach Anspruch 1, ausgewählt aus einer oder mehreren der folgenden:
5,6-Bis(4-chlorphenyl)-3-[(piperidin-1-ylamino)carbonyl]pyrazin-2-carbonsäure-tert.-butylester;
5,6-Bis(4-chlorphenyl)-3-[(piperidin-1-ylamino)carbonyl]pyrazin-2-carbonsäurebutylester;
5,6-Bis(4-chlorphenyl)-3-[(piperidin-1-ylamino)carbonyl]pyrazin-2-carbonsäurecyclohexylester;
5,6-Bis(4-chlorphenyl)-3-[(piperidin-1-ylamino)carbonyl]pyrazin-2-carbonsäurebenzylester;
5,6-Bis(4-chlorphenyl)-3-({[cis-2-hydroxycyclohexyl]amino}carbonyl)pyrazin-2-carbonsäure-tert.-butylester;
5,6-Bis(4-chlorphenyl)-3-({[trans-2-hydroxycyclohexyl]amino}carbonyl)pyrazin-2-carbonsäure-tert.-butylester;
5,6-Bis-(4(chlorphenyl)-3-({2-[4-(trifluormethyl)phenyl]hydrazino}carbonyl)pyrazin-2-carbonsäure-tert.-butylester;
5,6-Bis(4-chlorphenyl)-3-[(morpholin-4-ylamino)carbonyl]pyrazin-2-carbonsäure-tert.-butylester;
5,6-Bis(4-chlorphenyl)-3-({2-[4-(tert.-butylhydrazino}carbonyl)pyrazin-2-carbonsäure-tert.-butylester;
5,6-Bis(4-chlorphenyl)-3-{[(4,4-difluorcyclohexyl)amino]carbonyl}pyrazin-2-carbonsäure-tert.-butylester;
5,6-Bis(4-chlorphenyl)-3-[(pentylamino)carbonyl]pyrazin-2-carbonsäure-tert.-butylester;
5,6-Bis(4-chlorphenyl)-3-{[(1-ethylpropyl)amino]carbonyl}pyrazin-2-carbonsäure-tert.-butylester;
5,6-Bis(4-chlorphenyl)-3-{[(4,4-difluorpiperidin-1-yl)amino]carbonyl}pyrazin-2-carbonsäure-tert.-butylester;
5,6-Bis(4-chlorphenyl)-*N*-piperidin-1-yl-3-[(4-propyl-1*H*-1,2,3-triazol-1-yl)methyl]pyrazin-2-carbonsäureamid;
5,6-Bis(4-chlorphenyl)-3-(phenoxymethyl)-*N-*piperidin-1-ylpyrazin-2-carbonsäureamid;
5,6-Bis(4-chlorphenyl)-3-(morpholin-4-ylmethyl)-*N-*piperidin-1-ylpyrazin-2-carbonsäureamid;
5,6-Bis(4-chlorphenyl)-3-(piperidin-1-ylmethyl)-*N-*piperidin-1-ylpyrazin-2-carbonsäureamid;
5,6-Bis(4-methylphenyl)-3-[(piperidin-1-ylamino)carbonyl]pyrazin-2-carbonsäure-tert.-butylester;
5,6-Bis(4-methylphenyl)-*N*-piperidin-1-yl-3-(1*H-*tetrazol-1-ylmethyl)pyrazin-2-carbonsäureamid;
5,6-Bis(4-methylphenyl)-*N*-piperidin-1-yl-3-(2*H-*tetrazol-2-ylmethyl)pyrazin-2-carbonsäureamid;
5,6-Bis(4-chlorphenyl)-*N*-piperidin-1-yl-3-(2*H-*tetrazol-2-ylmethyl)pyrazin-2-carbonsäureamid;
5,6-Bis(4-chlorphenyl)-*N*-piperidin-1-yl-3-(1*H-*tetrazol-1-ylmethyl)pyrazin-2-carbonsäureamid;
5,6-Bis(4-chlorphenyl)-*N*-(4,4-difluorcyclohexyl)-3-(2*H*-tetrazol-2-ylmethyl)pyrazin-2-carbonsäureamid;
5,6-Bis(4-chlorphenyl)-*N*-(4,4-difluorpiperidin-1-yl)-3-(2*H*-tetrazol-2-ylmethyl)pyrazin-2-carbonsäureamid;
5,6-Bis(4-chlorphenyl)-3-[(5-methyl-2*H*-tetrazol-2-yl)methyl]-*N*-piperidin-1-ylpyrazin-2-carbonsäureamid;
5,6-Bis(4-chlorphenyl)-3-[(5-methyl-1*H*-tetrazol-1-yl)methyl]-*N*-piperidin-1-ylpyrazin-2-carbonsäureamid;
6-(4-Chlorphenyl)-5-(4-methylphenyl)-3-[(piperidin-1-ylamino)carbonyl]pyrazin-2-carbonsäure-tert.-butylester;
5-(4-Chlorphenyl)-6-(4-methylphenyl)-3-[(piperidin-1-ylamino)carbonyl]pyrazin-2-carbonsäure-tert.-butylester;
6-(4-Chlorphenyl)-5-(4-methylphenyl)-*N*-piperidin-1-yl-3-(2*H*-tetrazol-2-ylmethyl)pyrazin-2-carbonsäureamid;
5-(4-Chlorphenyl)-6-(4-methylphenyl)-N-piperidin-1-yl-3-(2*H*-tetrazol-2-ylmethyl)pyrazin-2-carbonsäureamid;
5,6-Bis(4-chlorphenyl)-3-propoxy-pyrazin-2-carbonsäurepiperidin-1-ylamid;
5,6-Bis(4-chlorphenyl)-*N*-piperidin-1-yl-3-(2*H-*tetrazol-5-ylmethyl)pyrazin-2-carbonsäureamid;
5,6-Bis(4-chlorphenyl)-*N*-piperidin-1-yl-3-(1*H-*tetrazol-5-yl)pyrazin-2-carbonsäureamid;
5,6-Bis(4-chlorphenyl)-3-[(5-morpholin-4-yl-2*H-*tetrazol-2-yl)methyl]-*N*-piperidin-1-ylpyrazin-2-carbonsäureamid;
5,6-Bis(4-chlorphenyl)-3-[(5-morpholin-4-yl-1*H-*tetrazol-1-yl)methyl]-*N*-piperidin-1-ylpyrazin-2-carbonsäureamid;
5,6-Bis(4-chlorphenyl)-*N*-piperidin-1-yl-3-[(5-pyrrolidin-1-yl-2*H*-tetrazol-2-yl)methyl]pyrazin-2-carbonsäureamid;
5,6-Bis(4-chlorphenyl)-*N*-piperidin-1-yl-3-[(5-pyrrolidin-1-yl-1*H*-tetrazol-1-yl)methyl]pyrazin-2-carbonsäureamid;
5,6-Bis(4-chlorphenyl)-*N*-(4,4-difluorcyclohexyl)-3-{[(4-fluorbenzyl)oxy]methyl}pyrazin-2-carbonsäureamid;
5,6-Bis(4-chlorphenyl)-3-[(4,4-difluorpiperidin-1-yl)methyl]-*N*-piperidin-1-ylpyrazin-2-carbonsäureamid; oder
5,6-Bis-4-chlorphenyl)-*N*-(4,4-difluorcyclohexyl)-3-[(4,4-difluorpiperidin-1-yl)methyl]pyrazin-2-carbonsäureamid;
und deren pharmazeutisch annehmbaren Salzen.

5. Verbindungen nach Anspruch 1, ausgewählt aus einer oder mehreren der folgenden:
5,6-Bis(4-chlorphenyl)-3-[(piperidin-1-ylamino)carbonyl]pyrazin-2-carbonsäure-tert.-butylester;
5,6-Bis(4-chlorphenyl)-3-[(piperidin-1-ylamino)carbonyl]pyrazin-2-carbonsäurebutylester;
5,6-Bis(4-chlorphenyl)-3-[(piperidin-1-ylamino)carbonyl]pyrazin-2-carbonsäurecyclohexylester; und
5,6-Bis(4-chlorphenyl)-3-[(piperidin-1-ylamino)carbonyl]pyrazin-2-carbonsäurebenzylester und deren pharmazeutisch annehmbaren Salzen.

6. Verbindungen der Formel I nach einem der vorhergehenden Ansprüche zur Verwendung als Medikament.

7. Pharmazeutische Formulierung, enthaltend eine Verbindung der Formel I nach einem der Ansprüche 1 bis 5 und ein pharmazeutisch annehmbares Adjuvans, ein pharmazeutisch annehmbares Verdünnungsmittel oder einen pharmazeutisch annehmbaren Träger.

8. Verwendung einer Verbindung nach einem der Ansprüche 1 bis 5 bei der Herstellung eines Medikaments zur Behandlung oder Prophylaxe von Obesitas, psychiatrischen Erkrankungen, wie psychotischen Erkrankungen, Schizophrenie und manisch-depressiver Psychose, Ängstlichkeit, anxiodepressiven Erkrankungen, Depression, kognitiven Störungen, Gedächtnisstörungen, Zwangsneurosen, Anorexie, Bulimie, Aufmerksamkeitsstörungen, Epilepsie und verwandten Leiden, und neurologischen Erkrankungen wie Demenz, Parkinson-Krankheit, Chorea Huntington und Alzheimer-Krankheit, Erkrankungen des Immunsystems, des Herzkreislaufsystems, der Fortpflanzungsorgane und des Endokrinsystems, septischem Schock, mit den Atemwegen und dem Magen-Darm-Trakt in Zusammenhang stehenden Erkrankungen und Indikationen von anhaltendem Substanzmißbrauch, Sucht und/oder Rückfall.

9. Verwendung nach Anspruch 8 bei der Herstellung eines Medikaments zur Behandlung oder Prophylaxe von Obesitas.

## Revendications

1. Composé de formule (I) 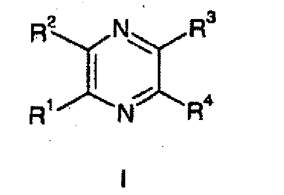 et les sels pharmaceutiquement acceptables de celui-ci, dans laquelle
R¹ et R² représentent indépendamment phényle, thiényle ou pyridyle dont chacun est indépendamment éventuellement substitué par un ou plusieurs groupements représentés par Z ;
Z représente un groupement C₁₋₈alkyle, un groupement C₁₋₆alcoxy, hydroxy, halogéno, trifluorométhyle, trifluorométhylthio, trifluorométhoxy, trifluorométhylsulfonyle, nitro, mono ou di C₁₋₃alkylamido, C₁₋₃alkylsulfonyle, C₁₋₃alkylsulfonyloxy, C₁₋₃alcoxycarbonyle, carboxy, cyano, carbamoyle, mono ou di C₁₋₃alkylcarbamoyle, sulfamoyle, acétyle, un groupement hétérocyclique aromatique qui est éventuellement substitué par un ou plusieurs halogéno, C₁₋₄alkyle, trifluorométhyle ou trifluorométhoxy et un groupement hétérocyclique saturé ou partiellement insaturé à 5 à 8 chaînons contenant un ou plusieurs hétéroatomes choisis parmi azote, oxygène ou soufre où le groupement hétérocyclique est éventuellement substitué par un ou plusieurs groupements C₁₋₃alkyle, hydroxy, fluoro, benzyle ou un groupement amino -NR^{x}R^{y} dans lequel R^{x} et R^{y} représentent indépendamment H ou C₁₋₄alkyle ;
R³ représente un groupement de formule X-Y-NR⁵R⁶ ;
dans laquelle X est CO ou SO₂
et Y est absent ou représente NH éventuellement substitué par un groupement C₁₋₃alkyle et R⁵ et R⁶ représentent indépendamment :
un groupement C₁₋₆alkyle ;
un groupement (amino) C₁₋₄alkyl- dans lequel l'amino est éventuellement substitué par un ou plusieurs groupements C₁₋₃alkyle ;
un groupement (C₃₋₁₂cycloalkyl) (CH₂)_{g}- dans lequel g est 0, 1, 2 ou 3 où le cycloalkyle est éventuellement substitué par un ou plusieurs fluoro, hydroxy, C₁₋₃alkyle C₁₋₃alcoxy, trifluorométhyle ou trifluorométhoxy ;
un groupement -(CH₂)ᵣ(phényle)ₛ dans lequel r est 0, 1, 2, 3 ou 4, s est 1 lorsque r est 0 sinon s est 1 ou 2 et les groupements phényle sont éventuellement indépendamment substitués par un ou plusieurs groupements représentés par Z ;
naphtyle ;
anthracényle ;
un groupement hétérocyclique saturé ou partiellement insaturé à 5 à 8 chaînons contenant un ou plusieurs hétéroatomes choisis parmi azote, oxygène ou soufre où le groupement hétérocyclique est éventuellement substitué par un ou plusieurs groupements C₁₋₃alkyle, hydroxy, fluoro, trifluorométhyle, benzyle ou un groupement amino -NR^{x}R^{y} dans lequel R^{x} et R^{y} représentent indépendamment H ou C₁₋₄alkyle ;
1-adamantylméthyle ;
un groupement -(CH₂)ₜHet dans lequel t est 0, 1, 2, 3 ou 4, et la chaîne alkylène est éventuellement substituée par un ou plusieurs groupements C₁₋₃alkyle et Het représente un groupement hétérocyclique aromatique éventuellement substitué par 1, 2 ou 3 groupements choisis parmi un groupement C₁₋₅alkyle, un groupement C₁₋₅alcoxy ou halogéno ;
ou R⁵ représente H et R⁶ est tel que défini ci-dessus ;
ou R⁵ et R⁶ ensemble avec l'atome d'azote auquel ils sont liés représentent un groupement hétérocyclique saturé ou partiellement insaturé à 5 à 8 chaînons contenant un azote et éventuellement l'un de ce qui suit : oxygène, soufre ou un azote supplémentaire, où le groupement hétérocyclique est éventuellement substitué par un ou plusieurs groupements C₁₋₃alkyle, hydroxy, fluoro, trifluorométhyle, trifluorométhoxy, benzyle, C₁₋₆alcanoyle ou un groupement amino -NR^{x}R^{y} dans lequel R^{x} et R^{y} représentent indépendamment H ou C₁₋₄alkyle ;
R⁴ représente un groupement de formule (CH2)ₙCOOR⁷ dans laquelle n est 0, 1, 2, 3 ou 4 ; et R⁷ représente un groupement C₄₋₁₂alkyle, un groupement C₃₋₁₂cycloalkyle, ou un groupement (C₃₋₁₂cycloalkyl)C₁₋₃alkyl- dont chacun est éventuellement substitué par un ou plusieurs de ce qui suit : un groupement C₁₋₆alkyle, fluoro, amino ou hydroxy, ou
R⁷ représente un groupement - (CH₂)ₐphényle dans lequel a est 0, 1, 2, 3 ou 4 et le groupement phényle est éventuellement substitué par un ou plusieurs groupements représentés par Z qui peut être identique ou différent ou
R⁷ représente un groupement hétérocyclique saturé ou partiellement insaturé à 5 à 8 chaînons contenant un ou plusieurs de ce qui suit : oxygène, soufre ou azote ; où le groupement hétérocyclique est éventuellement substitué par un ou plusieurs groupements C₁₋₃alkyle, un ou plusieurs groupements C₁₋₃acyle, hydroxy, amino ou benzyle ; ou
R⁴ représente un groupement de formule -(CH₂)ₒ-O-(CH₂)ₚ-R⁸ dans laquelle o et p représentent indépendamment un entier 0, 1, 2, 3 ou 4 et R⁸ représente un groupement C₁₋₁₂alkyle à condition que R⁴ ne soit pas un groupement C₁₋₃alcoxyméthyle, ou R⁸ représente phényle éventuellement indépendamment substitué par un ou plusieurs groupements Z ou R⁸ représente un groupement hétérocyclique aromatique ou un groupement hétérocyclique saturé ou partiellement insaturé à 5 à 8 chaînons contenant un ou plusieurs de ce qui suit : oxygène, soufre ou azote où chacun de ces cycles est éventuellement substitué par un ou plusieurs groupements représentés par Z qui peut être identique ou différent ;
R⁴ représente un groupement C₄₋₁₂alkyle éventuellement substitué par un ou plusieurs fluoro, hydroxy ou amino ; ou
R⁴ représente un groupement de formule -(CH₂)_{q}R⁹ dans lequel q est 0, 1, 2, 3 ou 4 et R⁹ représente un groupement C₃₋₁₂cycloalkyle, phényle, un groupement hétérocyclique aromatique ou un groupement hétérocyclique saturé ou partiellement insaturé à 5 à 8 chaînons contenant un ou plusieurs de ce qui suit : oxygène, soufre ou azote où chacun de ces cycles est éventuellement substitué par un ou plusieurs groupements représentés par Z qui peut être identique ou différent ; ou
R⁴ représente un groupement de formule -(CH₂)ₘ-O-(CO)-R¹⁰ dans laquelle m représente un entier 0, 1, 2, 3 ou 4, dans laquelle R¹⁰ représente un groupement C₁₋₁₂alkyle éventuellement substitué par un ou plusieurs fluoro, hydroxy ou amino, ou R¹⁰ représente un groupement de formule -(CH₂)_{q}R⁹ dans laquelle q et R⁹ sont tels que décrits précédemment,
où le terme groupement hétérocyclique aromatique signifie un cycle monocyclique aromatique à 5, 6 ou 7 chaînons ou un cycle bicyclique aromatique à 9 ou 10 chaînons, ayant jusqu'à cinq hétéroatomes du cycle choisis parmi oxygène, azote et soufre.

2. Formule I selon la revendication 1, telle que représentée par la formule IIa 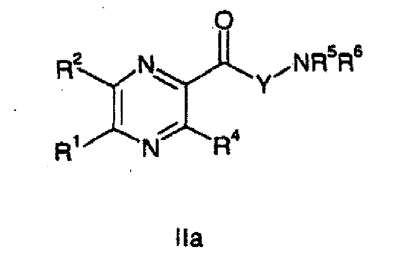 dans laquelle R¹ et R² représentent indépendamment phényle éventuellement indépendamment substitué par halogéno ou pyridyle,
R⁴ représente un groupement C₄₋₈alkyle, un groupement CH₂OR⁸ dans lequel R⁸ est un groupement C₄₋₈alkyle, un groupement CO₂R⁷ dans lequel R⁷ représente un groupement C₄₋₈alkyle,
et Y représente NH et
R⁵ représente H et
R⁶ représente perfluorophényle ou phényle éventuellement substitué par trifluorométhyle ou R⁵ et R⁶ ensemble avec l'azote auquel ils sont liés représentent pipéridino, morpholino ou pipérazino dont chacun est éventuellement substitué par un ou plusieurs groupements C₁₋₃alkyle, hydroxy, fluoro, trifluorométhyle, trifluorométhoxy, benzyle, C₁₋₆alcanoyle ou un groupement amino -NR^{x}R^{y} dans lequel R^{x} et R^{y} représentent indépendamment H ou C₁₋₄alkyle ;
ou Y est absent et
R⁵ représente H ou un groupement C₁₋₆alkyle éventuellement substitué par amino ;
R⁶ représente tétrahydropyranyle ou 4-pipéridinyle éventuellement substitué par un ou plusieurs groupements C₁₋₃alkyle, hydroxy, fluoro, trifluorométhyle, trifluorométhoxy, benzyle, C₁₋₆alcanoyle ou un groupement amino -NR^{x}R^{y} dans lequel R^{x} et R^{y} représentent indépendamment H ou C₁₋₄alkyle ou un groupement C₁₋₆alkyle éventuellement substitué par amino ;
ou
R⁵ et R⁶ ensemble avec l'azote auquel ils sont liés représentent pipéridino, morpholino ou pipérazino dont chacun est éventuellement substitué par C₁₋₃alkyle ou fluoro.

3. Composé de formule I selon la revendication 1 telle que représentée par la formule IIb 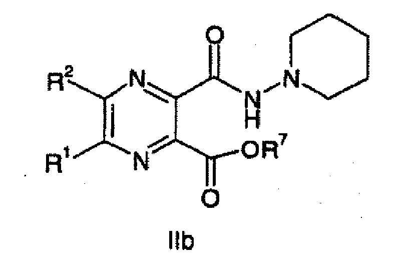 dans laquelle R¹ et R² représentent phényle indépendamment éventuellement substitué par un ou plusieurs chloro ; et
R⁷ représente butyle, *tert*-butyle, cyclohexyle ou benzyle.

4. Composé selon la revendication 1, choisi parmi un ou plusieurs de ce qui suit :
le 5,6-bis(4-chlorophényl)-3-[(pipéridin-1-ylamino)carbonyl]pyrazine-2-carboxylate de tert-butyle ;
le 5,6-bis(4-chlorophényl)-3-[(pipéridin-1-ylamino)carbonyl]pyrazine-2-carboxylate de butyle ;
le 5,6-bis(4-chlorophényl)-3-[(pipéridin-1-ylamino)carbonyl]pyrazine-2-carboxylate de cyclohexyle ;
le 5,6-bis(4-chlorophényl)-3-[(pipéridin-1-ylamino)carbonyl]pyrazine-2-carboxylate de benzyle ;
le 5,6-bis(4-chlorophényl)-3-({[cis-2-hydroxycyclohexyl]amino}carbonyl)pyrazine-2-carboxylate de tert-butyle ;
le 5,6-bis(4-chlorophényl)-3-({[trans-2-hydroxycyclohexyl]amino}carbonyl)pyrazine-2-carboxylate de tert-butyle ;
le 5,6-bis-(4-chlorophényl)-3-({2-[4-(trifluorométhyl)phényl]hydrazino}carbonyl)pyrazin e-2-carboxyate de tert-butyle ;
le 5,6-bis(4-chlorophényl)-3-[(morpholin-4-ylamino)carbonyl]pyrazine-2-carboxylate de tert-butyle ;
le 5,6-bis(4-chlorophényl)-3-({2-[4-(tert-butylhydrazino}carbonyl)pyrazine-2-carboxylate de tert-butyle ;
le 5,6-bis(4-chlorophényl)-3-{[(4,4-difluorocyclohexyl)amino]carbonyl}pyrazine-2-carboxylate de tert-butyle ;
le 5,6-bis(4-chlorophényl)-3-[(pentylamino)carbonyl]pyrazine-2-carboxylate de tert-butyle ;
le 5,6-bis(4-chlorophényl)-3-{[(1-éthylpropyl)amino]carbonyl}pyrazine-2-carboxylate de tert-butyle ;
le 5,6-bis(4-chlorophényl)-3-{[(4,4-difluoropipéridin-1-yl)amino]carbonyl}pyrazine-2-carboxylate de tert-butyle ;
le 5,6-bis(4-chlorophényl)-N-pipéridin-1-yl-3-[(4-propyl-1H-1,2,3-triazol-1-yl)méthyl]pyrazine-2-carboxamide ;
le 5,6-bis(4-chlorophényl)-3-(phénoxyméthyl)-*N-*pipéridin-1-ylpyrazine-2-carboxamide ;
le 5,6-bis(4-chlorophényl)-3-(morpholin-4-ylméthyl)-N-pipéridin-1-ylpyrazine-2-carboxamide ;
le 5,6-bis(4-chlorophényl)-3-(pipéridin-1-ylméthyl)-N-pipéridin-1-ylpyrazine-2-carboxamide ;
le 5,6-bis(4-méthylphényl)-3-[(pipéridin-1-ylamino)carbonyl]pyrazine-2-carboxylate de tert-butyle ;
le 5,6-bis(4-méthylphényl)-*N*-pipéridin-1-yl-3-(1*H-*tétrazol-1-ylméthyl)pyrazine-2-carboxamide ;
le 5,6-bis(4-méthylphényl)-*N*-pipéridin-1-yl-3-(2*H-*tétrazol-2-ylméthyl)pyrazine-2-carboxamide ;
le 5,6-bis(4-chlorophényl)-*N*-pipéridin-1-yl-3-(2*H-*tétrazol-2-ylméthyl)pyrazine-2-carboxamide ;
le 5,6-bis(4-chlorophényl)-*N*-pipéridin-1-yl-3-(1*H-*tétrazol-1-ylméthyl)pyrazine-2-carboxamide ;
le 5,6-bis(4-chlorophényl)-*N*-(4,4-difluorocyclohexyl)-3-(2H-tétrazol-2-ylméthyl)pyrazine-2-carboxamide ;
le 5,6-bis(4-chlorophényl)-*N*-(4,4-difluoropipéridin-1-yl)-3-(2*H*-tétrazol-2-ylméthyl)pyrazine-2-carboxamide ;
le 5,6-bis(4-chlorophényl)-3-[(5-méthyl-2*H-*tétrazol-2-yl)méthyl]-*N*-pipéridin-1-ylpyrazine-2-carboxamide ;
le 5,6-bis(4-chlorophényl)-3-[(5-méthyl-1*H-*tétrazol-1-yl)méthyl]-*N*-pipéridin-1-ylpyrazine-2-carboxamide ;
le 6-(4-chlorophényl)-5-(4-méthylphényl)-3-[(pipéridin-1-ylamino)carbonyl]pyrazine-2-carboxylate de tert-butyle;
le 5-(4-chlorophényl)-6-(4-méthylphényl)-3[(pipéridin-1-ylamino)carbonyl]pyrazine-2-carboxylate de tert-butyle ;
le 6-(4-chlorophényl)-5-(4-méthylphényl)-*N-*pipéridin-1-yl-3-(2*H*-tétrazol-2-ylméthyl)pyrazine-2-carboxamide ;
le 5-(4-chlorophényl)-6-(4-méthylphényl)-*N-*pipéridin-1-yl-3-(2*H*-tétrazol-2-ylméthyl)pyrazine-2-carboxamide ;
le pipéridin-1-ylamide d'acide 5,6-bis(4-chlorophényl)-3-propoxy-pyrazine-2-carboxylique ;
le 5,6-bis(4-chlorophényl)-*N*-pipéridin-1-yl-3-(2*H-*tétrazol-5-ylméthyl)pyrazine-2-carboxamide ;
le 5,6-bis(4-chlorophényl)-*N*-pipéridin-1-yl-3-(1*H-*tétrazol-5-yl)pyrazine-2-carboxamide ;
le 5,6-bis(4-chlorophényl)-3-[(5-morpholin-4-yl-2*H*-tétrazol-2-yl)méthyl]-*N*-pipéridin-1-ylpyrazine-2-carboxamide ;
le 5,6-bis-(4-chlorophényl)-3-[(5-morpholin-4-yl-1*H*-tétrazol-1-yl)méthyl]-*N*-pipéridin-1-ylpyrazine-2-carboxamide ;
le 5,6-bis(4-chlorophényl)-*N*-pipéridin-1-yl-3-[(5-pyrrolidin-1-yl-2*H*-tétrazol-2-yl)méthyl]pyrazine-2-carboxamide ;
le 5,6-bis(4-chlorophényl)-*N*-pipéridin-1-yl-3-[(5-pyrrolidin-1-yl-1*H*-tétrazol-1-yl)méthyl]pyrazine-2-carboxamide ;
le 5,6-bis(4-chlorophényl)-*N*-(4,4-difluorocyclohexyl)-3-{[(4-fluorobenzyl)oxy]méthyl}pyrazine-2-carboxamide ;
le 5,6-bis(4-chlorophényl)-3-[(4,4-difluoropipéridin-1-yl)méthyl]-N-pipéridin-1-ylpyrazine-2-carboxamide ; ou
le 5,6-bis-(4-chlorophényl)-*N*-(4,4-difluorocyclohexyl)-3-[(4,4-difluoropipéridin-1-yl)méthyl]pyrazine-2-carboxamide ;
et les sels pharmaceutiquement acceptables de ceux-ci.

5. Composé selon la revendication 1, choisi parmi l'un de ce qui suit :
le 5,6-bis(4-chlorophényl)-3-[(pipéridin-1-ylamino)carbonyl]pyrazine-2-carboxylate de tert-butyle ;
le 5,6-bis(4-chlorophényl)-3-[(pipéridin-1-ylamino)carbonyl]pyrazine-2-carboxylate de butyle ;
le 5,6-bis(4-chlorophényl)-3-[(pipéridin-1-ylamino)carbonyl]pyrazine-2-carboxylate de cyclohexyle ; et
le 5,6-bis(4-chlorophényl)-3-[(pipéridin-1-ylamino)carbonyl]pyrazine-2-carboxylate de benzyle et les sels pharmaceutiquement acceptables de ceux-ci.

6. Composé de formule I selon l'une quelconque des revendications précédentes, destiné à être utilisé comme médicament.

7. Formulation pharmaceutique comprenant un composé de formule I, tel que défini dans l'une quelconque des revendications 1 à 5, et un adjuvant, un diluant ou un support pharmaceutiquement acceptable.

8. Utilisation d'un composé selon l'une quelconque des revendications 1 à 5, dans la préparation d'un médicament destiné au traitement ou à la prophylaxie de l'obésité, des troubles psychiatriques tels que les troubles psychotiques, la schizophrénie ou les troubles bipolaires, de l'anxiété, des troubles anxio-dépressifs, de la dépression, des troubles cognitifs, des troubles de la mémoire, des troubles obsessifs compulsifs, de l'anorexie, de la boulimie, des troubles de l'attention, de l'épilepsie, et des affections apparentées et des troubles neurologiques tels que la démence, des troubles neurologiques, de la maladie de Parkinson, de la chorée de Huntington et de la maladie d'Alzheimer, des troubles immunitaires, cardiovasculaires, de la reproduction et endocriniens, du choc septique, des maladies liées aux systèmes respiratoires et gastro-intestinaux, et des indications d'abus prolongé, de dépendance et/ou de rechute.

9. Utilisation selon la revendication 8, dans la préparation d'un médicament destiné au traitement ou à la prophylaxie de l'obésité.
